# EUROPEAN PATENT APPLICATION

(11) **EP 4 357 345 A1**
(43) Date of publication of application: **24.04.2024**
(21) Application number: 22824326.7
(22) Date of filing: 17.06.2022
(51) Int. Cl.: C07D 471/04, C07D 401/14, C07D 401/12, A61K 31/4545, A61K 31/444, A61K 31/4439, A61P 17/02, A61P 17/10, A61P 17/06, A61P 17/14, A61P 17/00

(54) **CLY SERIES COMPOUND, PREPARATION METHOD THEREFOR AND USE THEREOF IN PREPARATION OF DRUGS**

(30) Priority: 17.06.2021 CN 202110668896
(71) Applicant: Nanjing Wellbest Pharmaceutical Co., Ltd, Nanjing, Jiangsu 210023 (CN)
(72) Inventor: CHEN, Min, Nanjing, Jiangsu 210023 (CN); WU, Yongping, Nanjing, Jiangsu 210023 (CN)
(74) Representative: Cabinet Laurent & Charras
(86) International application number: PCT/CN2022/099496
(87) International publication number: WO 2022/262854

(57) **Abstract**

Disclosed in the present invention are a CLY series compound, a preparation method therefor and the use thereof in the preparation of drugs. The CLY series compound is a compound with a structure of formula I, a tautomer thereof, a solvate thereof, or a pharmaceutically acceptable salt thereof. Animal model experiments show that the CLY series compound, in a chloasma mouse model, can obviously reduce the level oftyrosinase in the skin and blood, reduce the expression of the stem cell factor (SCF) and C-Kit protein in the skin, and inhibit the chromogenesis of chloasma, can obviously promote the healing of a wound on skin and reduce the formation of scars, in a hair loss mouse model, can obviously promote the hair growth and reduce damage to hair follicles, and in a rabbit ear acne model, can obviously reduce the symptoms and reduce pore blockage and acne formation. The CLY series compound can significantly prolong the survival time of acute GVHD mice, and can reduce clinical symptoms, exhibiting a therapeutic effect on acute GVHD; in a pulmonary fibrosis mouse model, same can obviously reduce the levels ofMMP-2 and MMP-9 in the lung tissue, increase the levels of TIMP-1 and VEGF, and simultaneously increase the levels of SOD and CAT enzymes in peripheral blood; and same can also improve the arthritis symptoms ofrheumatoid arthritis mice by means of reducing the level of IL-17 in peripheral blood and increasing inflammatory indexes such as IL-10. The compound can be used alone or in ombination with other drugs, and provides a new drug choice for the treatment of the above diseases.

## Description

### FIELD

The invention belongs to the field of pharmaceutical chemistry, particularly relates to a CLY series compound, a preparation method thereof, and the application thereof in the field of medicine.

### BACKGROUND

Chloasma is a kind of acquired pigmentation disorder, which mainly occurs in middle-aged and young women. Its pathogenesis is extremely complex, and there are many influencing factors. Tyrosine is oxidized to dopa in the melanosome under the action of tyrosinase. Dopa is further oxidized to dopa quinone by dopa oxidase, and finally, dopa quinone is oxidized to form melanin under the action of tyrosinase. A series of oxidation and antioxidation reaction disorders in this process may cause and promote the occurrence and development of chloasma. When the balance of a series of oxidation reactions is disordered, the body produces excessive oxygen free radicals, and the activities of antioxidant enzymes such as superoxide dismutase (SOD) are reduced, forming lipid peroxide. Lipid peroxide is unstable and will decompose rapidly to produce aldehydes. Its final product, malondialdehyde (MDA), will increase correspondingly, and attack phospholipids and proteins, resulting in oxidative damage of pigment cells, promoting the oxidation reaction of tyrosinase, increasing melanin and depositing it in the basal layer of skin. Therefore, it is of great significance for the prevention and treatment of chloasma to reduce the amount of MDA in skin tissue and increase the activity of antioxidant enzyme SOD. Chloasma is easy to recur and difficult to treat, few products on the market can effectively treat chloasma. The freckle is also easy to recur. Hydroquinone is the earliest and most widely used whitening agent, but it has been restricted in the treatment of whitening and chloasma due to its uneven distribution of skin pigment, strong irritation to the skin, and even skin cancer possibilities. Arbutin is one of the most widely used whitening agents in clinics, but its effect is limited.

Scar is a symptom that physical, biological, chemical, and other factors damage the skin and soft tissue of the human body, resulting in serious damage to the skin were soft tissue cannot completely repair itself normally, and the repairments are replaced by fiber tissue, which affects both appearance and function. Scars bring huge physical and mental pain to patients, especially the scars left after burns, scalds, and severe injuries. It is difficult to treat scars. At present, we can only make the red and hard scars soft and shallow, the wide scars narrow, and the thick scars thin, which cannot completely eliminate scars. Therefore, it is very important to start the intervention at the early stage of wound healing, which can effectively reduce the formation of scar, improve the appearance, correct the deformity, and restore function. At present, the commonly used methods to treat scars include surgical treatment, laser treatment, cryotherapy, and drug treatment. Commonly used drugs include glucocorticoids and retinoic acid. Glucocorticoid has an obvious anti-fibrosis effect, but has many toxic side effects. Retinoic acid is an intermediate product of vitamin A metabolism in the body, which can reduce local inflammation, promote the growth of epithelial cells, reduce collagen synthesis, and reduce the DNA synthesis of fibroblasts, thus inhibiting cell growth. The higher the concentration of retinoic acid drugs, the more significant the inhibitory effect on growth. However, the therapeutic effect of retinoic acid is limited, and there are many toxic side effects caused by the systematic application of retinoic acid. External use has obvious skin irritation, which increases asthe concentration increases.

Alopecia areata (AA) is a kind of Non-cicatricial alopecia. Alopecia areata is usually a sudden alopecia spot, which can affect the whole scalp in severe cases, and is called Alopecia Totalis (AT). When alopecia affects all the hair of the whole body, including axillary hair and pubic hair, it is called Alopecia Universalis (AU). At present, the etiology is not fully understood. Abnormal or unstable autoimmune function and neuropsychiatric factors are considered important related factors. Some patients with alopecia areata can recover naturally, but some patients with alopecia areata can last for several years. Minoxidil is a common topical drug for the treatment of alopecia areata, which can promote skin vascular dilation, improve local blood circulation and promote hair growth. Severe alopecia areata is often treated with glucocorticoids, which mainly include prednisolone, compound betamethasone, etc., which can be taken orally, externally, or intradermally. For patients who are not suitable for glucocorticoids, immunosuppressants can be used. Common drugs include cyclosporine, methotrexate, and other immunosuppressants, but immunosuppressants have many side effects.

Androgen alopecia (AGA), also known as seborrheic alopecia (SA), is an androgen-dependent hereditary hair loss, a common and frequently-occurring disease. AGA mostly occurs in men aged 20 to 30 years old, mainly at the top of the head. The hair loss area starts from the hairline on both sides of the forehead and gradually extends upwards. At the same time, the hair gradually becomes sparse and thin. At last, most or all of the hair on the top of the head falls off, showing a horseshoe appearance. The skin at the hair loss area of AGA is bright, and the pores are reduced or there is a little soft vellus hair left. The speed, range, and severity of AGA hair loss are affected by genetics and individuals. Generally, hair loss develops the fastest at the age of 30, and severe complete baldness is rare. Female AGA is mostly diffuse alopecia at the top of the head, and the hair on the top of the head becomes sparse. The epidemiological survey in China shows that the prevalence rate of AGA is about 21.3% in men and about 6.0% in women. The etiology and pathogenesis of androgenic alopecia are still unclear. It is generally believed that androgen and its receptor play a key role in the pathogenesis of this disease, and type II 5a reductase is an important factor in its pathogenesis. Under normal physiological conditions, androgen can stimulate the growth and development of hair in vivo but can induce hair loss in some specific parts. Testosterone is the main androgen in the body. It is converted into dihydrotestosterone by 5a reductase, and dihydrotestosterone can cause the transformation from terminal hair to vellus hair, which finally leads to hair loss. At present, there is no ideal treatment for AGA, a very difficult type of alopecia. Minoxidil is a non-specific drug for the treatment of alopecia and is a first-line topical drug approved by the FDA for the treatment of alopecia. However, it may cause hirsutism on the face and limbs in the course of using, and the effect will gradually disappear after stopping the treatment of Minoxidil. Because androgen plays a very important role in pathogenesis, the new treatment methods in recent years attempt to stop the hair follicle from becoming smaller through the anti-androgen effect. Finasteride is a type II 5a reductase selective inhibitor. In recent years, finasteride has been found to be effective in the treatment of AGA and can continuously improve hair growth. However, finasteride has adverse effects such as abnormal sexual function, transient reduction of sperm, and abnormal development of the male breast. In animal experiments, finasteride was found to have a teratogenic effect, so it should not be used in children and women of childbearing age. Cimetidine needs to be taken continuously for 5 months or more, and the side effects are male breast development, impotence, decreased libido, etc. Oral contraceptives: mainly include soronorgestrel, levonorgestrel, norethisterone, norgestrel (norgestrel oxime), norgestrel diester, and norgestrel acetate. They are commonly used to treat female AGA. After 6 to 12 months of treatment, hair will improve, but it cannot be used for long-term.

Acne vulgaris (acne) is a chronic inflammatory disease that usually occurs in the sebaceous glands of hair follicles, with an incidence of about 9.4%. The occurrence of acne is closely related to the physiological pathological changes in adolescent skin. The clinical manifestations of acne mainly include comedo, papules, pustules, nodules, cysts, and scars, which seriously impact the appearance and psychology of patients. Acne is related to multiple pathogeneses. The formation of comedo due to abnormal keratinization of hair follicles is an important basis for the pathogenesis of acne. Inflammation and infection are the pathogenic factors of acne. The sebaceous glands of acne patients are large, the secretion of sebaceous glands is increased, and the level of linoleic acid in sebum is relatively reduced, which affects the synthesis of fat, leading to the lack of fatty acid in the follicular epithelium, thus inducing excessive keratinization of follicles, making the epithelial cells unable to fall off normally, and the sebaceous glands of hair follicles become excessively small, and the sebum cannot be discharged smoothly to form acne. The mouth of the sebaceous gland of the hair follicle is blocked to form an anoxic environment, resulting in a large number of anaerobic propionibacterium acne, which decomposes sebum, produces chemical chemokines and white blood cells to form papules. A large number of neutrophils in the sebaceous glands of hair follicles gather and swallow Propionibacterium acnes, after which the inflammatory reaction occurs, causing a large number of pus cells to accumulate, forming pustules and cysts. After healing, it is easy toform concave scars. The increase in androgen level is the key link to accelerating the generation of acne, which causes abnormal skin keratinization to block the sebaceous gland ducts of hair follicles, leading to bacterial retention, reproduction, and inflammation.

Diseases with abnormal keratinization mechanisms similar to acne include ichthyosis, perifollicular keratosis (also known as lichen fur), follicular keratosis, porokeratosis, etc. Perifollicular keratosis is characterized by enlarged hair follicle mouth with an angle plug inside. Ichthyosis is characterized by decreased sweat and sebaceous glands, and keratin embolism in hair follicles. The above diseases are easy to recur and difficult to treat. To improve abnormal keratosis and eliminate angle suppositories and acne, retinoic acid drugs are mainly used. Retinoids can inhibit keratinization, reduce sebum secretion, promote normal keratinization of keratinocytes, and have the effects of regulating immunity and antiinflammatory, thus reducing the formation of comedo, papules, and pustules. Clinically, they are widely used in treatments of acne, ichthyosis, peridermal keratosis, follicular keratosis, porokeratosis, and other abnormal keratosis diseases. However, retinoic acid drugs are irritating to the skin for external use, which can easily cause skin redness, swelling, and tingling, and aggravate the original skin lesions. Long-term external use of retinoids can lead to skin thinning, photosensitivity, and skin barrier damage, etc. Oral retinoic acid drugs have adverse reactions such as liver damage and elevated blood lipids. Therefore, more drugs are needed to treat the above diseases.

Psoriasis is a common chronic recurrent inflammatory skin disease. The prevalence rate of psoriasis in the world is about 0.1%~3%. The risk of metabolic syndrome and cardiovascular damage is increased in patients with moderate and severe psoriasis. Therefore, early diagnosis and treatment are of great significance for controlling and improving the symptoms of psoriasis and preventing concomitant diseases. External treatment is the first choice for mild to moderate psoriasis. Glucocorticoid has a good external effect, but it is not suitable for long-term, continuous and large-scale use; External use of retinoic acid has a good effect on plaque psoriasis, and skin irritation should be paid attention to; Vitamin D3 derivatives, such as calcipotriol, also have good efficacy but are not suitable for facial and skin wrinkles; Calmodulin inhibitors (such as tacrolimus, pimecrolimus, etc.) can be used in the scalp, skin wrinkles, genitals, and other parts, but long-term and large-scale use can increase the risk of lymphatic and skin cancer; Various cutin promoters (such as tar preparation, dithranol ointment, 10%-15% camptothecin ointment and salicylic acid ointment) can also be used externally, but the effect is limited. Immunosuppressants can be used to treat moderate and severe patients, but there are many adverse reactions after long-term use, such as bone marrow suppression, liver, and kidney function damage, and increased risk of infection.

Eczema (also known as atopic dermatitis) is a kind of skin inflammatory reaction with severe itching caused by a variety of internal and external factors, which is easy to recur. The etiology of eczema is complex. Mild and moderate eczema is mainly treated externally. Appropriate drugs should be selected according to the skin lesions of eczema. For subacute and chronic eczema, appropriate glucocorticoids, tar preparations, or immunomodulators, such as tacrolimus ointment, pimecrolimus ointment, etc. shall be used. Severe patients can use glucocorticoids and immunosuppressants systematically, but there are many adverse reactions, so it is not suitable for long-term use

Graft versus host disease (GVHD) is caused by the T lymphocytes in the allograft of the allograft donor after transplantation. Stimulated by a series of "cytokine storms" launched by the recipient, they significantly enhance their immune response to the recipient antigen. T lymphocytes in donor grafts launch cytotoxic attacks against recipient target cells, and skin, liver, and intestine are the main targets. The incidence of acute graft-versus-host disease is 30% - 45%, and the incidence of chronic disease is lower than that of acute disease. Allogeneic hematopoietic stem cell transplantation is an effective treatment for curable hematological tumors and hematopoiesis disorders. Acute graft-versus-host disease is GVHD's main serious complication, with high incidence rate, high mortality rate, and high disability rate, which is an important cause of nonrecurrent death of malignant hematological disease. Steroid hormone is the first-line treatment for acute GVHD, but about 50% of patients have hormone resistance, and GVHD reaction cannot be controlled. Other second-line treatment drugs for GVHD reactions, such as monoclonal antibodies (CD3, CD25 monoclonal antibodies, etc.) targeting T cell surface antigens, and chemotherapy drugs (mycophenolate mofetil, tacrolimus, etc.), have reliable therapeutic effects on GVHD, but the consequent low immunity and possible infection have seriously weakened the advantages of these drugs, and ultimately do not extend the survival time of patients. Therefore, it is urgent to find a new treatment that can effectively control hormone-resistant acute GVHD.

Pulmonary fibrosis is a diffuse lung disease with unknown etiology and complicated pathogenesis. It is now recognized that it is the result of excessive deposition of extracellular matrix caused by excessive repair after lung injury. The pathogenesis of pulmonary fibrosis is unknown. It is now agreed that repeated injury and excessive repair of alveolar epithelium are the keys to pathogenesis. The pathological feature is that long-term chronic lung inflammation and persistent alveolar injury lead to the accumulation of extracellular matrix metalloproteinase (MMP), especially the abnormal increase of MMP-2 and MMP-9, and the decrease of tissue inhibitor of metalloproteinase-1 (TIMP-1), which destroys its balance, leading to the aggregation of a large amount of extracellular matrix, tissue cell remodeling and excessive deposition of collagen in the lung. At the same time, it may inhibit the expression of vascular endothelial growth factor (VEGF) in tissues, reduce the permeability of pulmonary microveinvessels, inhibit the proliferation of vascular endothelial cells and vascular regeneration, aggravate lung tissue injury, and ultimately lead to diffuse pulmonary interstitial disease - pulmonary fibrosis. At present, there are no effective anti-fibrosis drugs. Glucocorticoids are commonly used to reduce the progress of anti-fibrosis, but the curative effect is limited and there are many adverse reactions. The current treatment methods are far from meeting the clinical needs. We need to find more new drugs with good efficacy and fewer side effects to control the disease progress, reduce recurrence and complications, and reduce mortality.

For the above diseases, the current treatment methods are far from meeting the clinical needs. We need to find more new drugs with good curative effects, fewer side effects, and low prices to control the disease progression and reduce the recurrence and complications.

### SUMMARY

The purpose of the invention is to provide CLY-series compounds with medicinal value or pharmaceutically acceptable salts thereof.

Another object of the invention is to provide preparation methods for the above compounds.

The further object of the present invention is to provide the applications of the above compounds.

The purpose of the invention can be achieved by the following measures:

The present invention provides a compound represented by the formula I, a tautomer thereof, a solvate thereof, or a pharmaceutically acceptable salt thereof,

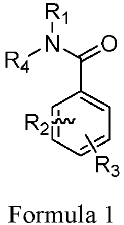

wherein R1 is a substituted or unsubstituted 5-6-membered heterocycle containing at least one of N, O, and S(such as isoquinoline group) or a combination ring of benzene ring and the abovementioned 5- to 6-membered heterocycle, or a combination ring of at least two of these heterocycles. The combination ring here refers to a ring formed by two ring structures through two adjacent atoms, such as pyrrolidine; The substituent is H, halogen, or (C1-C4) alkyl; R2 is H, halogen, hydroxyl, methoxy, ethoxy, amino, methyl, or ethyl; R3 is H, halogen, hydroxyl, methoxy, ethoxy, amino, (C1-C3) alkyl or the following groups: 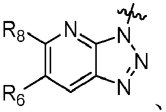 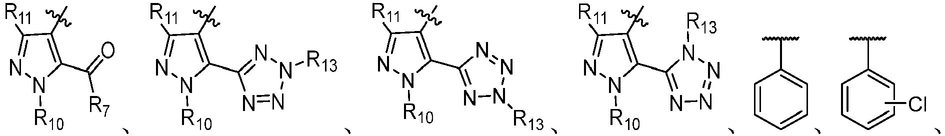 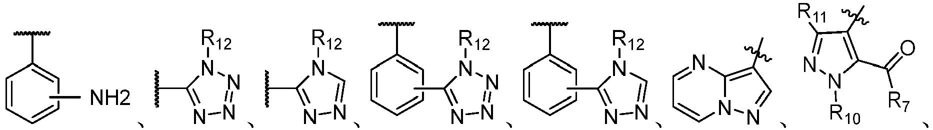 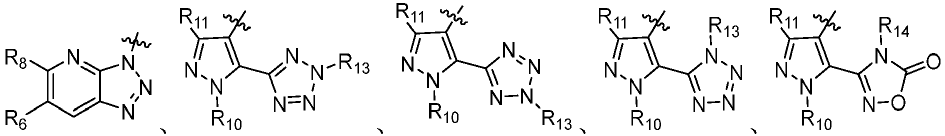 or 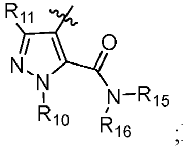 R4 is a substituted or unsubstituted 5-6-membered cycloalkyl or a 4-7-membered heterocycle with 1-3 heteroatoms selected from N or O, and the substituent group is selected from H, - NH2, - OH, (C1-C4) alkyl, (C1-C4) alkoxy, amino, and (C1-C4) alkylamino;

Preferably, R4 is

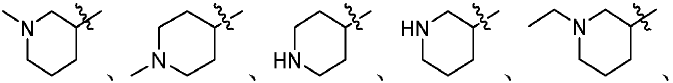

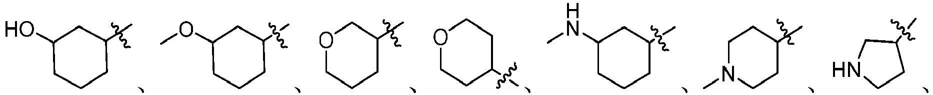

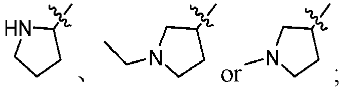

Preferably, if R3 is H, halogen, hydroxyl, methoxy, amino, methyl, or the following groups: 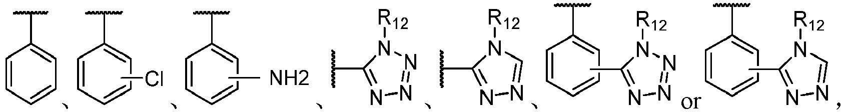 R₄ is

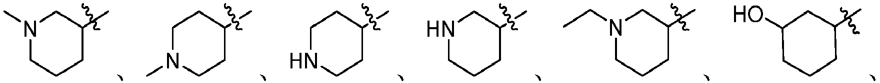

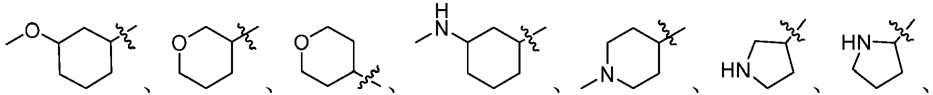

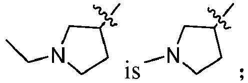

If R₃is 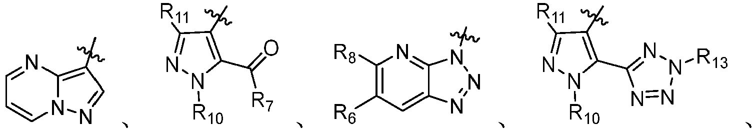 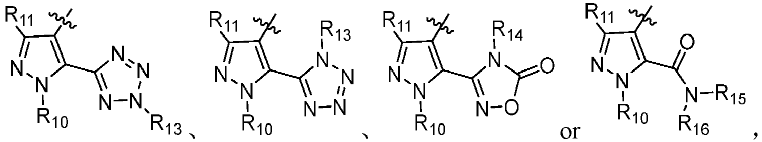 R₄is 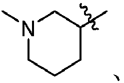 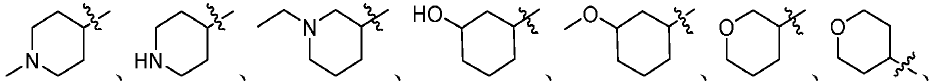 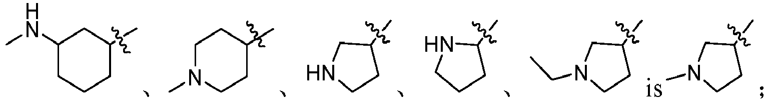
wherein R₆ and R₈ are independently H, methyl, halogen or (C1-C4) alkyl, and R₆ and R₈ are not both halogen; Preferably, R6 is H or methyl, and R8 is H;
R₇ is hydroxy, (C1-C4) alkoxy, (C1-C4) alkoxycarbonyloxy(C1-C4) alkyl or (C1-C4) alkyl carbonyloxy (C1-C4) alkyl;
R₁₀ and R₁₁ are independently H, (C1-C4) alkyl or (C3-C6) cycloalkyl;
R₁₂ is selected from H, halogen, - OH, - NH₂ or (C1-C3) alkyl;
R₁₃ is H, (C1-C4) alkyl, (C1-C4) alkylcarbonyloxy (C1-C4) alkyl or (C1-C4) alkoxycarbonyloxy (Cl-C4) alkyl;
R₁₄ is H, (C1-C4) alkyl, (C1-C4) alkylcarbonyloxy (C1-C4) alkyl or (C1-C4) alkoxycarbonyloxy (Cl-C4) alkyl;
R₁₅ is hydroxy, tetrazolyl, (C1-C2) alkylsulfonyl or trifluoromethylsulfonyl, and R₁₆ is H, (C1-C4) alkyl, (C1-C4) alkylcarbonyloxy (C1-C4) alkyl or (C1-C4) alkoxycarbonyloxy (Cl-C4) alkyl.

In some embodiments, R₁ is isoquinoline-1-yl, and R₁ is optionally substituted by chlorine or methyl.

In some embodiments, R₂ is H, hydroxy or methyl.

In some embodiments, R₁ is a substituted or unsubstituted pyridyl or pyrrolopyridyl; The substituent is H, chlorine or methyl.

In some embodiments, R₁₂ is selected from H, halogen, - OH, - NH₂ or methyl; In some embodiments, R₁₂ is selected from H.

The present invention provides compounds with formula I-a structure, their tautomers, their solvates or pharmaceutically acceptable salts, 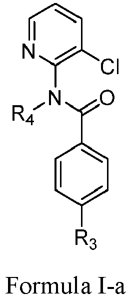
wherein R₃ is selected from any one of H, halogen, hydroxyl, methoxy, amino, methyl or the following substituted or unsubstituted groups: 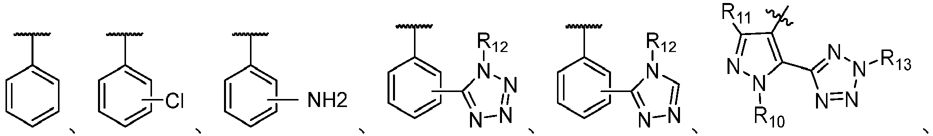 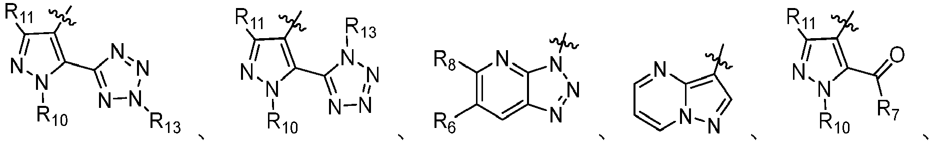 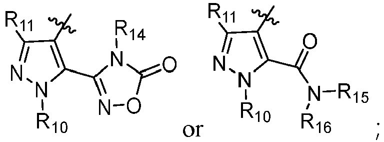 R4is 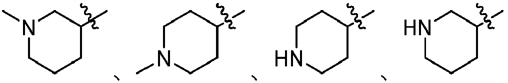 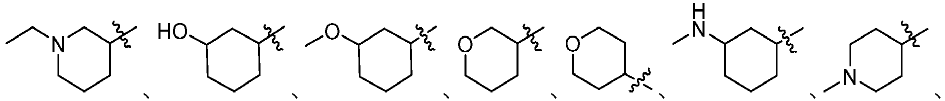 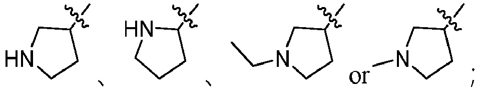
wherein R₆, R₇, R₈, R₁₀, R₁₁, R₁₂, R₁₃, R₁₄, R₁₅, R₁₆ are defined to be consistent with the above content.

Preferably, if R₃ is selected from H, halogen, hydroxyl or methoxy, amino, methyl or the following substituted or unsubstituted groups: 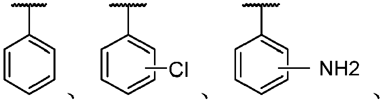 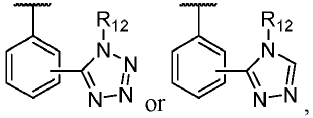 then R₄ is

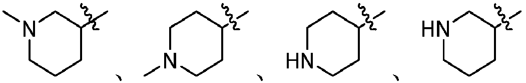

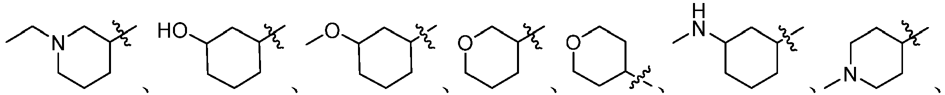

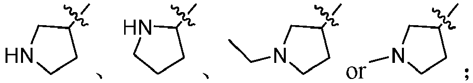

If R₃ is 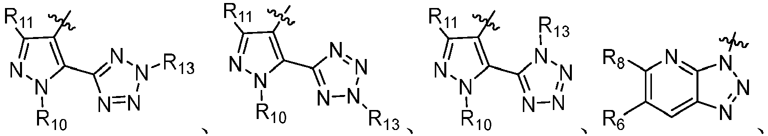 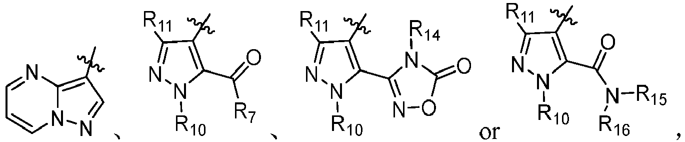 then R₄ is 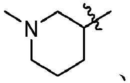 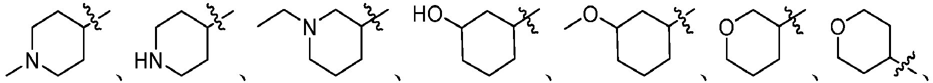 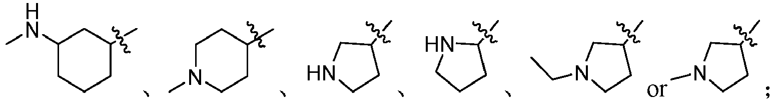
wherein R₆ and R₈ are independently H, methyl, halogen or (C1-C4) alkyl, and R₆ and R₈ are not both halogen; Preferably, R6 is H or methyl, and R8 is H;
R₇ is hydroxy, (C1-C4) alkoxy, (C1-C4) alkoxycarbonyloxy (C1-C4) alkoxy or (C1-C4) alkylcarbonyloxy (C1-C4) alkoxy;
R₁₀ and R₁₁ are independently H, (C1-C4) alkyl or (C1-C4) cycloalkyl;
R₁₂ is selected from H, halogen, -OH, -NH₂ or (C1-C3) alkyl;
R₁₃ is H, (C1-C4) alkyl, (C1-C4) alkylcarbonyloxy (C1-C4) alkyl or (C1-C4) alkoxycarbonyloxy (Cl-C4) alkyl;
R₁₄ is H, (C1-C4) alkyl, (C1-C4) alkylcarbonyloxy (C1-C4) alkyl or (C1-C4) alkoxycarbonyloxy (Cl-C4) alkyl;
R15 is hydroxy, tetrazolyl, (C1-C2) alkylsulfonyl or trifluoromethylsulfonyl, and R16 is H, (C1-C4) alkyl, (C1-C4) alkylcarbonyloxy (C1-C4) alkyl or (C1-C4) alkoxycarbonyloxy (Cl-C4) alkyl.

In some embodiments, compounds with formula I-a structure, their tautomers, their solvates or pharmaceutically acceptable salts, R₆ is H, and R₈ is H;

In some embodiments, compounds with formula I-a structure, their tautomers, their solvates or pharmaceutically acceptable salts, R₁₂ are selected from H, halogen, - OH, - NH₂ or methyl; In some embodiments, R₁₂ is selected from H.

In some embodiments, compounds with formula I-a structure, their tautomers, their solvates or pharmaceutically acceptable salts, R₁₁ is H, R₁₀ is H or methyl, and R₁₃ is

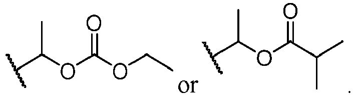

The present invention provides a compound represented by the formula I-b structure, a tautomer thereof, a solvate thereof or a pharmaceutically acceptable salt thereof, 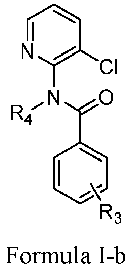
wherein R₃ is H, halogen, hydroxyl, methoxy, amino, methyl or the following substituted or unsubstituted groups: 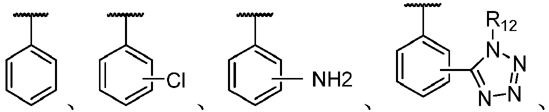 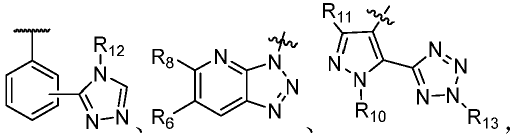 R₄ is 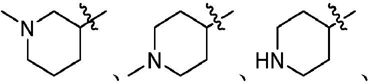 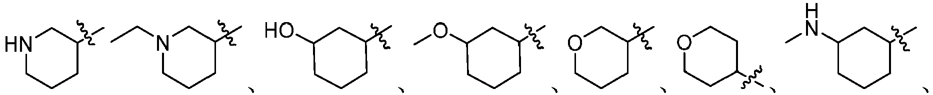 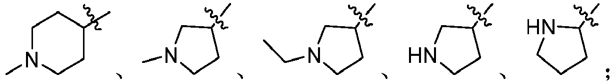
wherein R₆, R₈, R₁₀, R11, R₁₂ and R₁₃ are defined to be consistent with the above content.

Preferably, if R₃ is H, halogen, hydroxyl, methoxy, amino, methyl or the following substituted or unsubstituted groups: 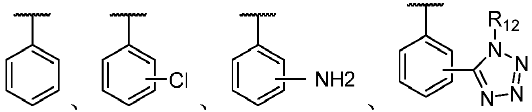 or 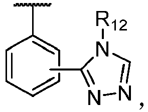 then R₄ is

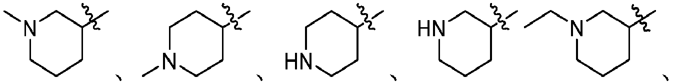

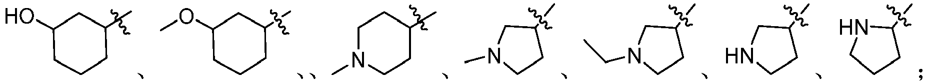

If R₃ is 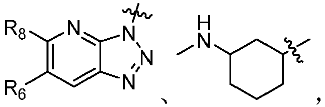 R4is 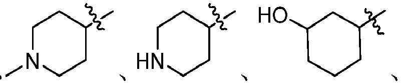 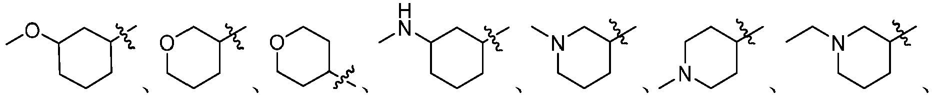 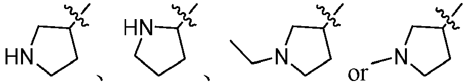 wherein R₆, R₈, R₁₀, R₁₁, R₁₂ and R₁₃ are defined to be consistent with the above content.

In some embodiments, the invention provides a compounds represented by the formula 1-b, a tautomer, a solvate thereof or a pharmaceutically acceptable salt thereof, wherein R₁₂ are selected from H, halogen, -OH, -NH₂ or methyl; In some embodiments, R₁₂ is selected from H.

In some specific embodiments, the invention provides the following compounds, the tautomers thereof, the solvates thereof or the pharmaceutically acceptable salts thereof, but not limited to the following compounds:

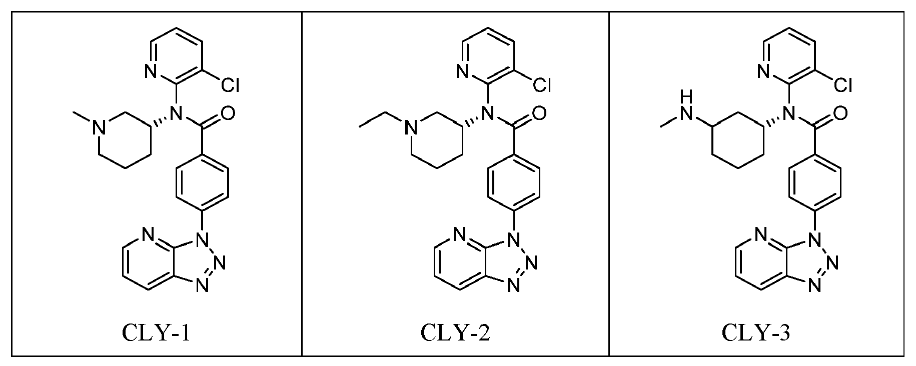

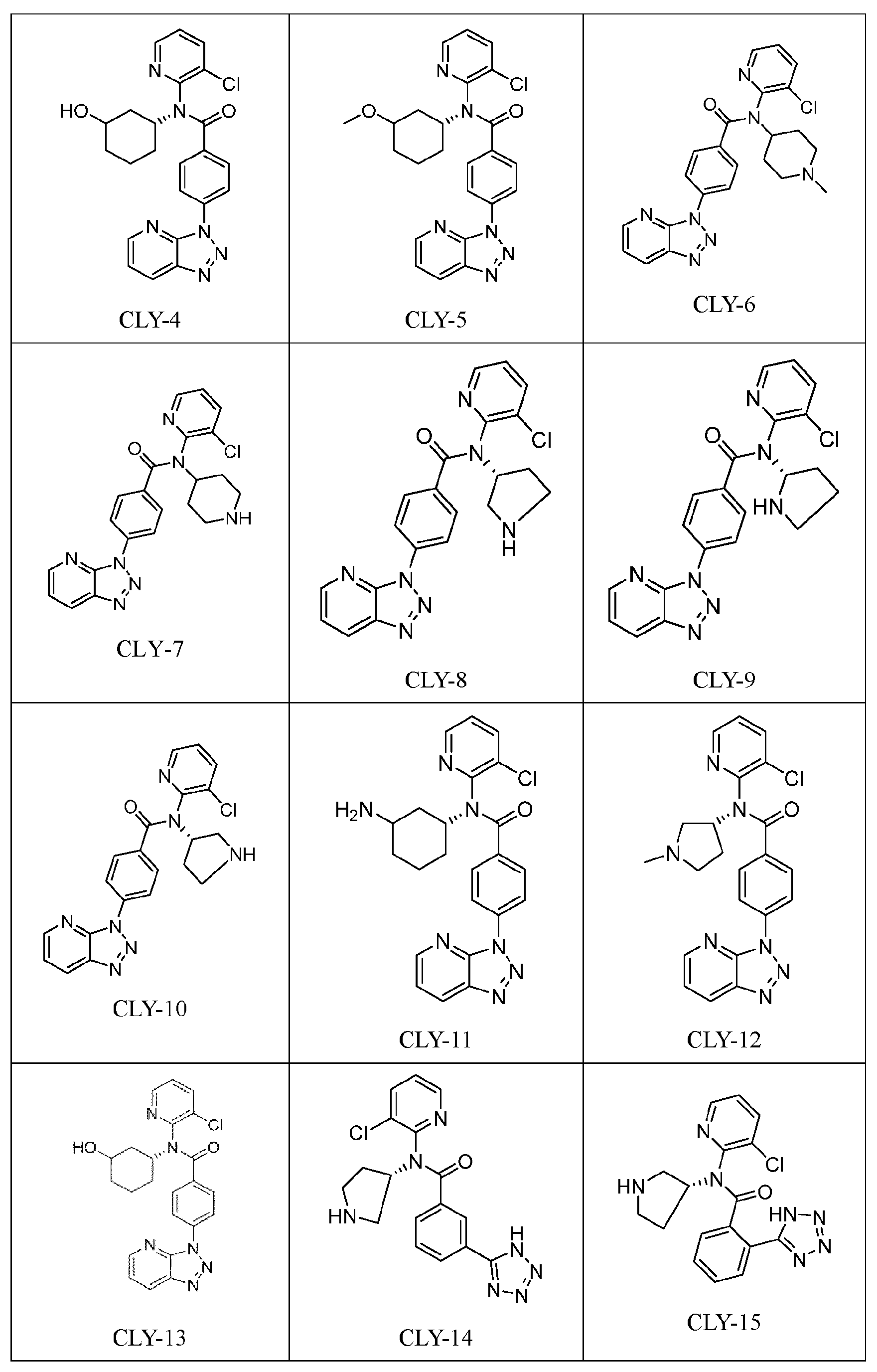

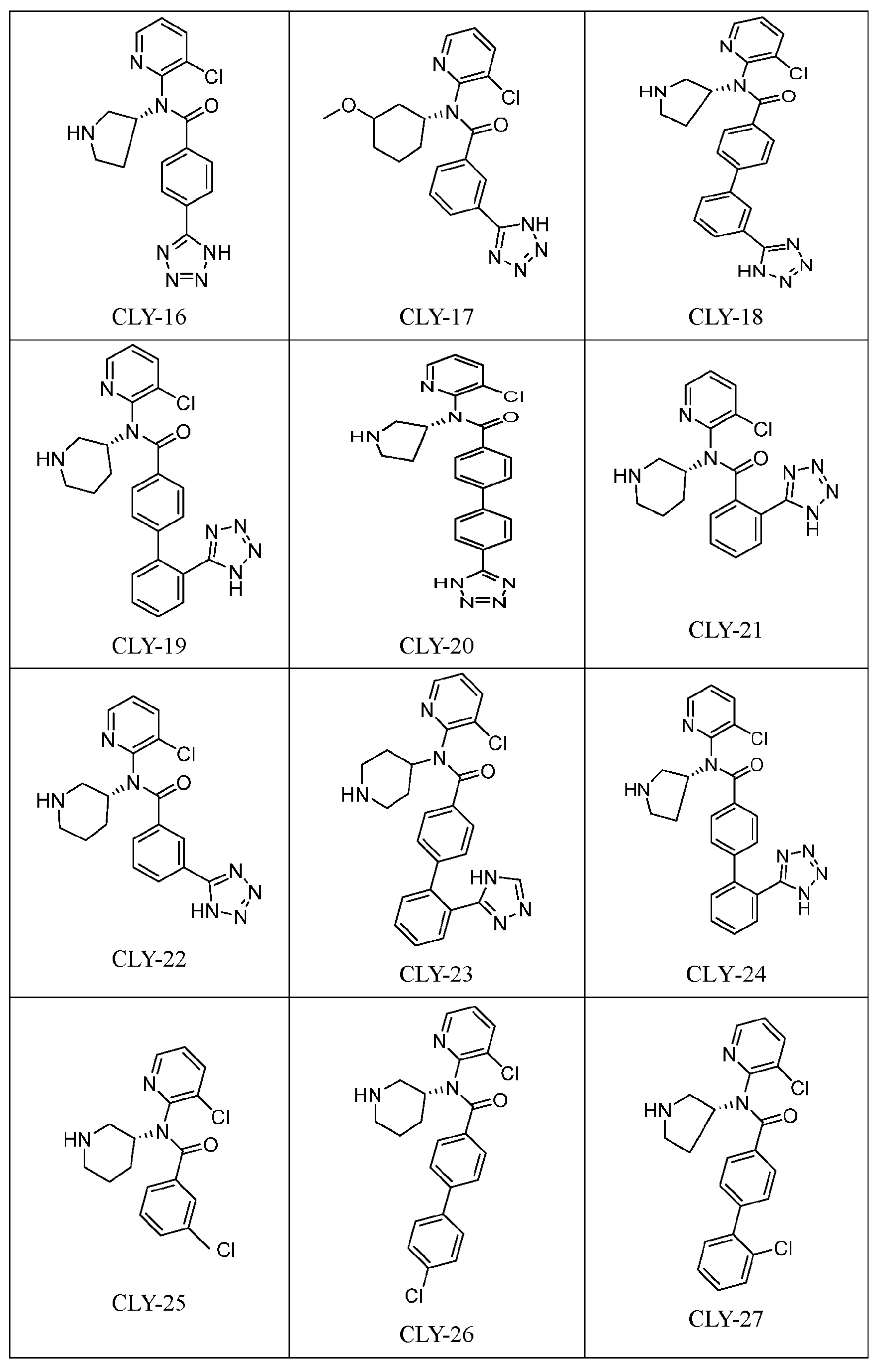

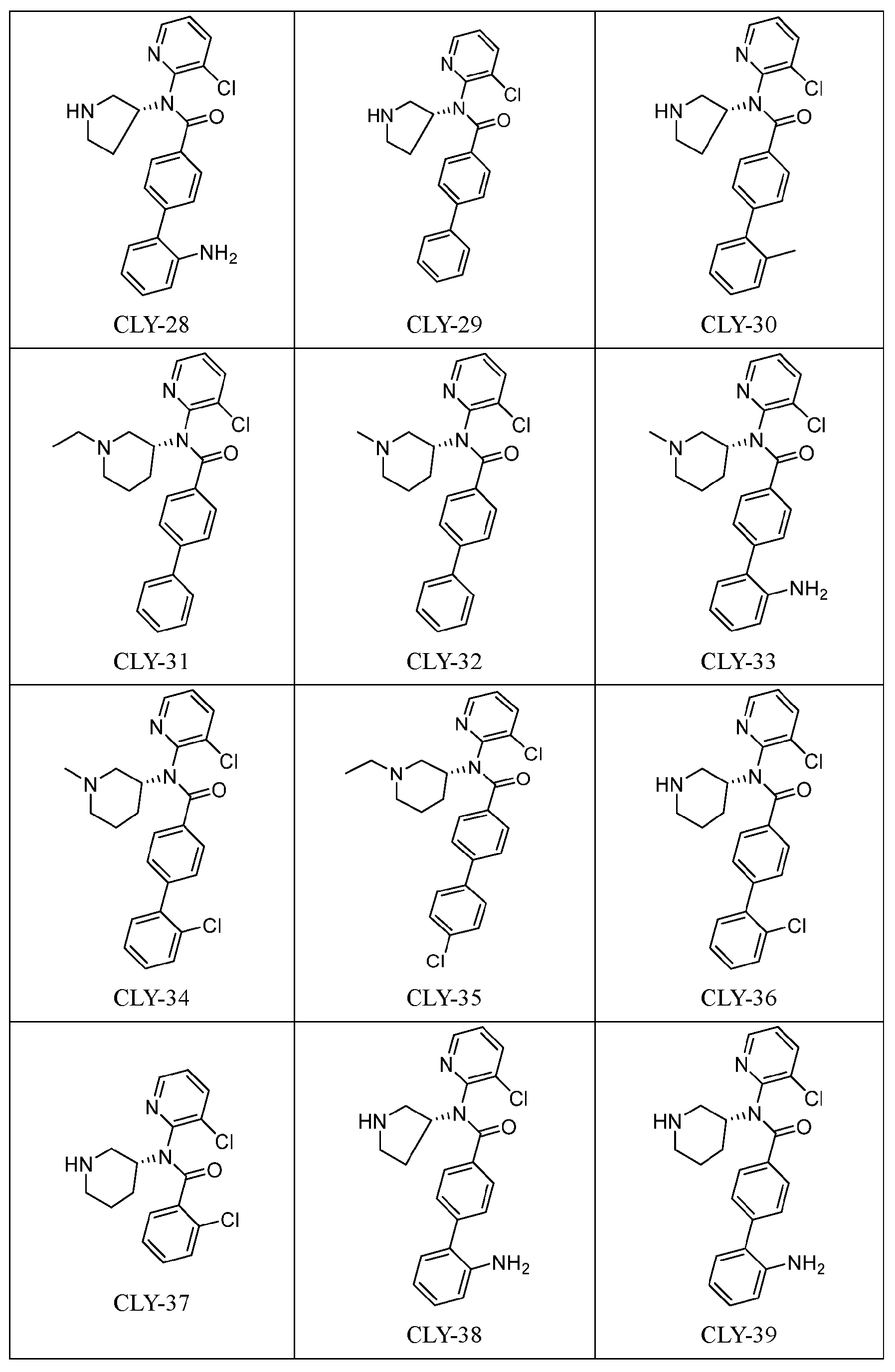

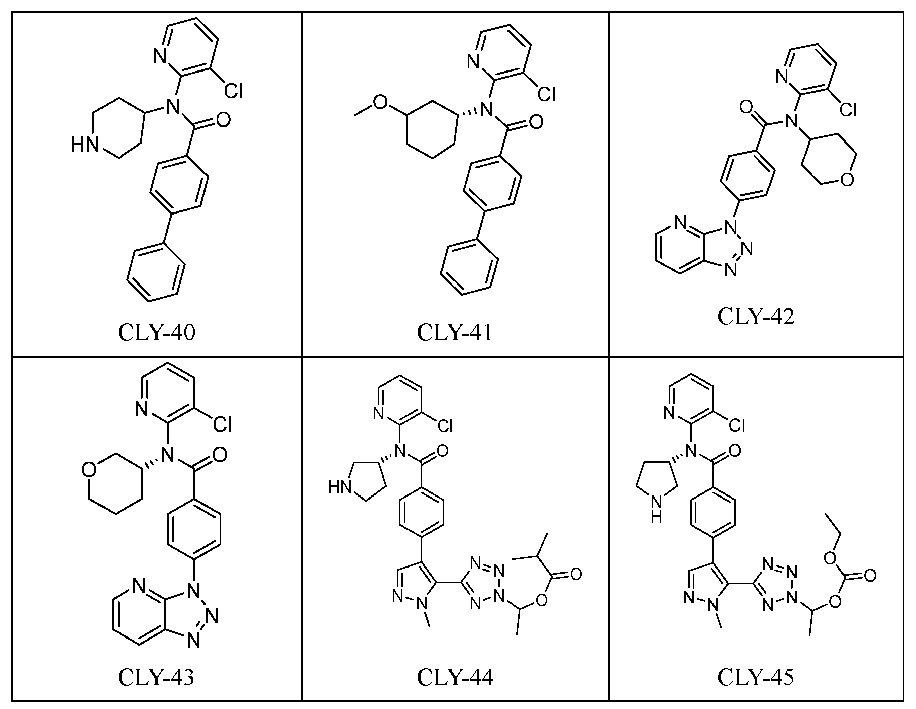

The invention also provides a preparation method of the compound represented by the formula (I):

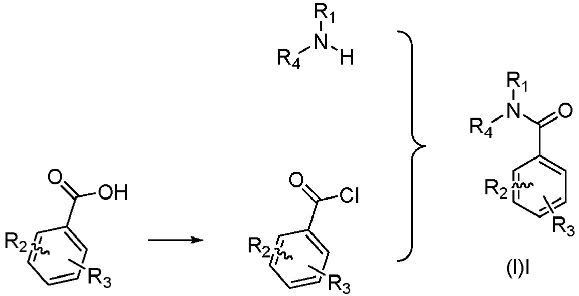

The raw material of 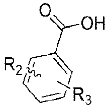 is used to produce 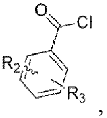 and then 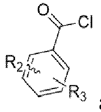 and 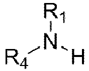 are used to produce the final product I;
Among the above materials, R₁, R₂, R₃, and R₄ are defined as above.

The invention also provides a pharmaceutical composition, which is composed of the compounds or its pharmaceutically acceptable salt as the active ingredient or main active ingredient, supplemented by pharmaceutically acceptable carrier.

The invention also provides the application of the above compounds in the preparation of drugs for treating and/or preventing diseases.

In some embodiments, the present invention provides the application of the compounds described in the present invention in the preparation of drugs for the treatment and/or prevention of chloasma, scar, androgenic alopecia, seborrheic alopecia, alopecia areata, acne, pulmonary fibrosis, psoriasis, eczema or graft versus host disease, etc.

The compounds or composition described in the present invention can be prepared into any pharmaceutically permissible forms of prepared drugs, such as a preparation suitable for oral, parenteral, intraperitoneal, intravenous, intra-arterial, transdermal, sublingual, intramuscular, rectal, transbuccal, intranasal, inhalation, vaginal, intraocular, local, external skin, subcutaneous, intrafat, intra-articular, intraperitoneal or any intrathecal administration.

In a preferred embodiment, the dosage forms of the invention are gel, emulsion, paste, liniment, lotion, spray, solution, tablet, granule, oral liquid, capsule, drop pill, enema, film, or injection.

### Definitions in the specification:

"C5-C6 monohydric alcohol" refers to a saturated aliphatic hydrocarbon group containing 5 or 6 carbon atoms replaced by a hydroxyl group, including linear and branched groups.

"Heterocycle" refers to a saturated ring group with 4 to 7 ring atoms, wherein one or two, or three ring atoms are heteroatoms selected from N, O, or S (O) m (where m is an integer from 0 to 2), and the remaining ring atoms are C, wherein one or two C atoms can be optionally replaced by carbonyl. The heterocyclic ring can optionally be independently replaced by one, two, or three substituents.

"Alkyl" refers to the saturated aliphatic hydrocarbon group with 1-20 carbon atoms, including linear and branched groups (the number range mentioned in this application, such as "1-4", refers to this group, which is alkyl at this time, and can contain 1 carbon atom, 2 carbon atoms, 3 carbon atoms or 4 carbon atoms. Alkyl can be substituted or unsubstituted.

"Cycloalkyl group" refers to a single ring or fused ring that is all carbon ("fused" ring means that each ring in the system shares an adjacent pair of carbon atoms with other rings in the system) group, one or more of which does not have a fully connected π electronic system. Examples of cycloalkyl include but are not limited to cyclopropane, cyclobutane, cyclopentane, cyclopentene, cyclohexane, adamantane, cyclohexadiene, cycloheptane and cycloheptatriene.

"Alkoxy" means - O - (unsubstituted alkyl) or - O - (unsubstituted cycloalkyl). Representative examples include, but are not limited to, methoxy, ethoxy, propioxy, butoxy, cyclopropyloxy, cyclobutoxy, cyclopentoxy, cyclohexoxy, etc.

"Alkylamino" means - NH - (unsubstituted alkyl), - NH - (unsubstituted cycloalkyl), - N - (unsubstituted alkyl) 2 or - N - (unsubstituted cycloalkyl) 2. Representative examples include but are not limited to methylamine, ethylamine, propylamine, butylamine, cyclopropylamine, cyclobutylamine, cyclopentalamine, cyclohexylamine, etc.

"(C1-C4) alkoxycarbonyloxy (C1-C4) alkyl" means (C1-C4) alkyl - O-C (O) - O - (C1-C4) alkyl -.

"(C1-C4) alkyl carbonyloxy (Cl-C4) alkyl" - represents (C1-C4) alkyl C (O) - O - (C1-C4) alkyl -. The compound CLY series of the invention or its pharmaceutically acceptable salt can be used in the pharmaceutical field. Animal model experiments show that the compound CLY series can significantly reduce the tyrosinase level in the skin and blood of the chloasma model, reduce the expression of hepatocyte factor (SCF), C-kit protein in the skin, and inhibit the formation of pigment in chloasma; It can obviously promote skin wound healing and reduce scar formation; It can significantly promote the hair growth of androgenic alopecia mouse model and reduce the damage of androgen to hair follicles; CLY series can significantly inhibit the inflammatory reaction of psoriasis and eczema mice models; CLY series of compounds can significantly improve the survival time of acute GVHD mice, alleviate clinical symptoms, and show therapeutic effect on acute GVHD; CLY series compounds can significantly reduce the levels of MMP-2 and MMP-9 in the lung tissue of pulmonary fibrosis mice model, increase the levels of TIMP-1 and VEGF, and increase the levels of SOD and CAT enzymes in the peripheral blood, which shows that they have an inhibitory effect on pulmonary fibrosis; CLY series of compounds can improve the arthritis symptoms of rheumatoid arthritis mice by reducing the level of IL-17 in peripheral blood and increasing inflammatory indicators such as IL-10. Because many diseases have a common pathogenesis pathway, the efficacy of this compound includes but is not limited to the above diseases. The compound can be used alone or in combination with other drugs, providing new drug choices for the treatment of the above diseases.

### BRIEF DESCRIPTION OF DRAWINGS

Fig. 1 CLY series compounds can significantly promote hair growth of hair loss model mice.
Fig. 2 CLY series compounds can significantly reduce the psoriatic inflammatory reaction in mice.
Fig. 3 CLY-2 nuclear magnetic testing results.
Fig. 4 CLY-8 nuclear magnetic testing results.

### DETAILED DESCRIPTION

The invention will be further described in combination with the embodiments below. It should be understood that the specific embodiments described here are only used to explain the invention and are not used to limit the invention. Any simple improvement of the preparation method of the invention under the premise of the concept of the invention belongs to the protection scope of the invention. In the following embodiments where they do not indicate the specific conditions of the experimental method, they are usually interpreted according to the well-known means in the field. The test materials used in the following embodiments, unless otherwise specified, are purchased from conventional biochemical reagent stores.

Example 1: Methods for preparing (R) - 4 - (3H - [1,2,3] triazolo [4,5-b] pyridin-3-yl) - N - (3-chloropyridin-2-yl) - N - (1-methylpiperidin-3-yl) benzamide (CLY-1) and (R) - 4 - (3H - [1,2,3] triazolo [4,5-b] pyridin-3-yl) - N - (3-chloropyridin-2-yl) - N - (1-ethylpiperidin-3-yl) benzamide (CLY-2):

### 1. Synthetic circuit

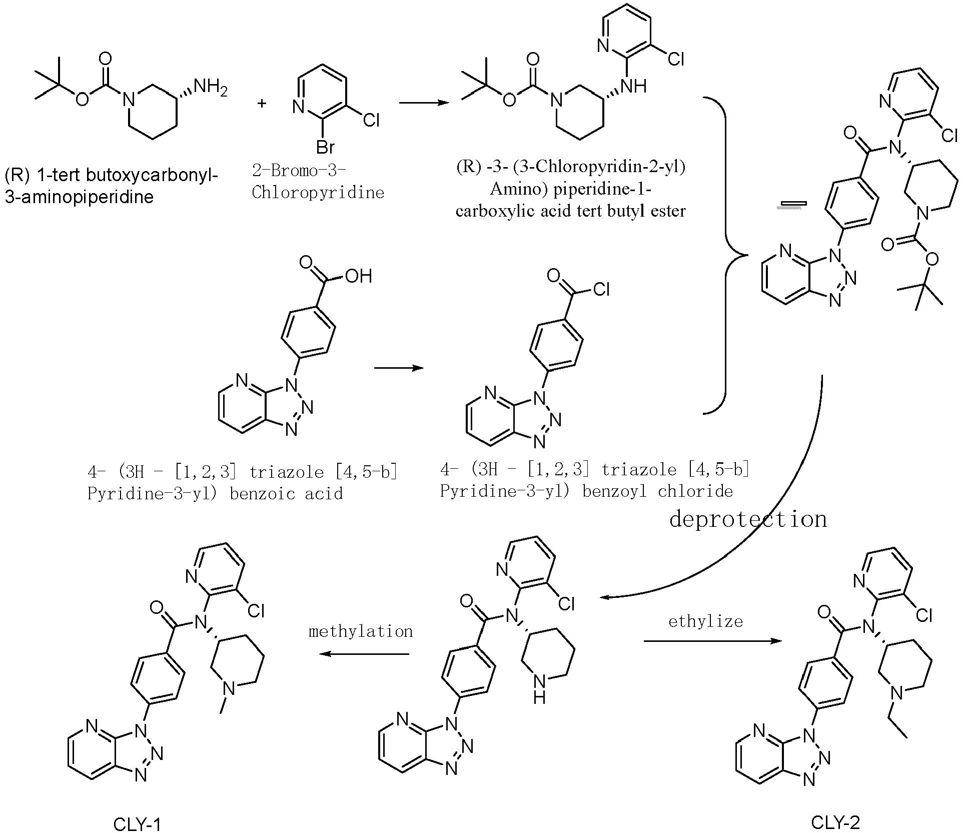

### 2. Specific implementation:

### (1) Synthesis of (R) - 3 - ((3-chloropyridin-2-yl) amino) piperidine-1-carboxylic acid tert-butyl ester.

(R)-1-Boc-3-aminopiperidine, 2-bromo-3-chloropyridine, sodium tert-butanol, dioxane were added into a 250ml container, protected by nitrogen, stirred, added RuPhosPd G3, ligand RuPhos, heated to 100 °C, reacted for 7 hours, stopped the reaction, poured into the water, extracted with ethyl acetate in batches, combined with the ethyl acetate layer, washed with water for 3 times, recovered the ethyl acetate layer to obtain the residual paste, and mixed with silica gel, (R) - 3 - ((3-chloropyridin-2-yl) amino) piperidine-1-carboxylic acid tert-butyl ester was obtained by silica gel column chromatography.

### (2) Synthesis of piperidine amide

4 - (3H - [1,2,3] triazole [4,5-b] pyridine-3-yl) benzoic acid was added to the container bottle, then pre-dried toluene, N, N-dimethylformamide and sulfoxide chloride were added, heated and stirred at 30 °C until clear, and the solvent was recovered under reduced pressure to obtain residual solid. Added 50ml of pre-dried tetrahydrofuran, (R) - 3 - ((3-chloropyridin-2-yl) amino) piperidine-1-carboxylic acid tert-butyl ester, stirred until it was clear, soakedtest tube into the ice water, added lithium di-trimethylsilyl-amine, stirred for 1 hour, removedit from the ice water bath, stirred for 2 hours, poured it into the water, extracted with ethyl acetate, washed the ethyl acetate layer, and recovered the solvent under reduced pressure to obtain the paste.

### (3) Synthesis of piperidine

Piperidine amide, dichloromethane, and trifluoroacetic acid were stirred overnight at room temperature. Poured it into the water, added sodium bicarbonate to adjust pH=10-11, extracted it with dichloromethane, washed it and recovered the solvent under reduced pressure to obtain paste residue, and obtained piperidine through silica gel column chromatography.

### (4) Synthesis of CLY-1

Dissolved piperidine in dichloromethane, addediodoethane, stirred silver carbonate in dark at room temperature for 48 hours, and directly put the reaction solution on a silica gel column to obtain CLY-1.

### Chemical formula of CLY-1: C23H22ClN7O

Hydrogen spectrum d4-MeOH: 8.83 (1H), 8.60 (1H), 8.54 (1H), 8.29 (2H), 7.82 (1H), 7.61 (3H), 7.42 (1H), 5.06 (1H), 3.82 (1H), 3.63 (1H), 3.39 (1H), 2.95 (1H), 2.40 - 1.87 (6H), 1.55 - 1.41 (1H).

### (5) Synthesis of CLY-2

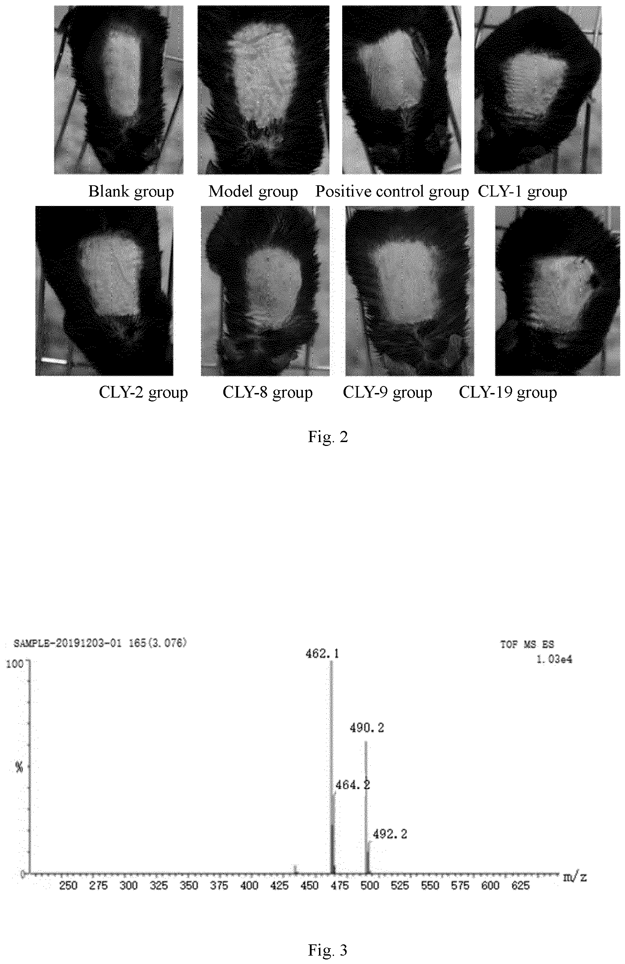

Dissolved piperidine in dichloromethane, added iodoethane, stirred silver carbonate in dark at room temperature for 48 hours, and directly put the reaction solution on silica gel column to obtain CLY-2. The CLY-2 mass spectrum results are shown in Figure 3.

### Example 2 Method for preparing (R) - 4 - (3H - [1,2,3] triazolo [4,5-b] pyridin-3-yl) - N - (3-chloropyridin-2-yl) - N - (pyrrolidin-3-yl) benzamide (CLY-8):

### 1. Synthetic route

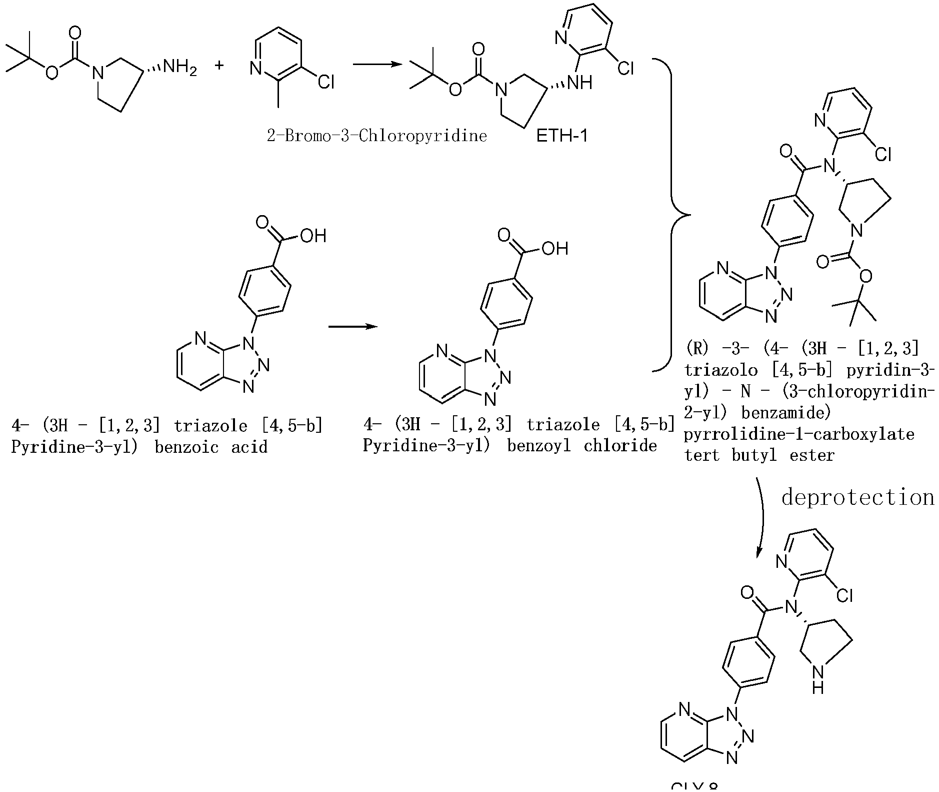

### 3. Specific synthesis steps:

### (1) Synthesis of ETH-1

(R) - 1-Boc-3-aminopyrrolidine, 2-bromo-3-chloropyridine, sodium tert-butanol and toluene were added to a three-necked bottle, and the stirring was started with nitrogen protection at the same time. Palladium acetate, 1,1 '- biphenyl-2-naphthol, and tris (dimethylamino) phosphine were added, heated to 100 °C, and reacted for 7 hours. After the reaction stopped, they were poured into the water and extracted with ethyl acetate in batches. After merging the ethyl acetate layer, washed it with water, recovered the ethyl acetate layer, and obtained the residual paste and ETH-1 through silica gel column chromatography.

### (2) Synthesis of pyrrolidine amide

4 - (3H - [1,2,3] triazole [4,5-b] pyridine-3-yl) benzoic acid was added to the solvent, oxalyl chloride and catalyst N, N-dimethylformamide were added to react at 25~55 °C until it was clear, and the solvent was recovered under reduced pressure, the residue was added to the solvent, and (R) - 3 - ((3-chloropyridin-2-yl) amino) piperidine-1-carboxylic acid tert-butyl esterwas added, cooled to below 5 °C in the ice water bath, and bis (trimethylsilyl) amine lithium was added to obtain pyrrolidine amide, That is, (R) - 3 - (4 - (3H - [1,2,3] triazolo [4,5-b] pyridin-3-yl) - N - (3-chloropyridin-2-yl) benzamide) pyrrolidine-1-carboxylic acid tert-butyl ester.

### (3) Synthesis of compound CLY-8

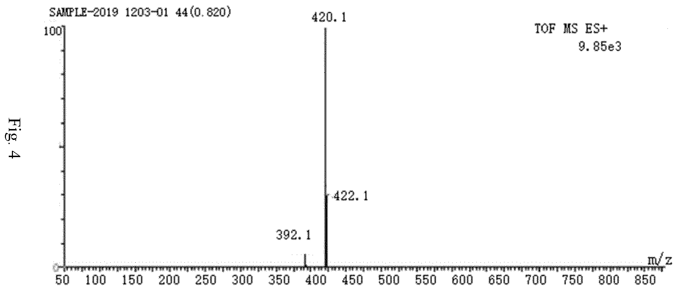

Pyrrolidine amide, dichloromethane, 4 mol/L hydrochloric acid methanol solution, were put together and stirred overnight at room temperature. Poured it into the water, added sodium bicarbonate to adjust pH=10-11, extracted it with dichloromethane, washed it, recovered the solvent under reduced pressure to obtain the paste residue, and through silica gel column chromatography, obtained the compound (R) - 4 - (3H - [1,2,3] triazolo [4,5-b] pyridin-3-yl) - N - (3-chloropyridin-2-yl) - N - (pyrrolidin-3-yl) benzamide (CLY-8 for short), M+H=420.1 (see Figure 4 for the mass spectrometric detection results).

The following compounds in Table 1 can also be synthesized by referring to the same method above:

### Graph 1

| | |
|---|---|
| 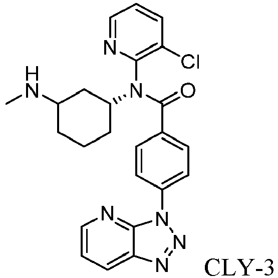 | Chemical Formula: C24H24ClN7O |
| | Molecular Weight: 461.95 |
| | Data in hydrogen spectrum CDCl3: 0.73 (1) , 1.35 (2) , 1.64 (2) , 1.81 (1) , 2.33 (2) , 2.41 (3) , 2.73 (2) , 4.58 (1) , 6.92 (1) , 7.24 (1) , 7.55 (1) , 7.86 (1) , 8.05 (2) , 8.3 (2) , 8.43 (1) , 8.65 (1) |
| | MS (ES+) :462 (M+H) |
| 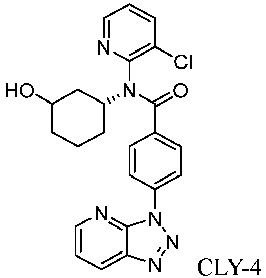 | Chemical Formula: C23H21ClN6O2 |
| | Molecular Weight: 448.91 |
| | Data in hydrogen spectrum CDCl3: 0.71 (1) , 1.31 (2) , 1.59 (2) , 1.71 (1) , 2.23 (2) , 2.63 (2) , 3.98 (1) , 6.92 (1) , 7.24 (1) , 7.55 (1) , 7.86 (1) , 7.95 (2) , 8.31 (2) , 8.43 (1) , 8.65 (1) |
| | MS (ES+) :449(M+H) |
| 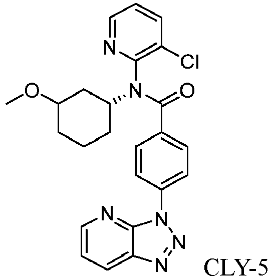 | Chemical Formula: C24H23ClN6O2 |
| | Molecular Weight: 462.94 |
| | Data in hydrogen spectrum CDCl3: 0.69 (1) , 1.25 (2) , 1.64 (2) , 2.33 (2) , 2.61 (3) , 2.73 (2) , 4.28 (1) , 6.92 (1) , 7.24 (1) , 7.55 (1) , 7.86 (1) , 8.01 (2) , 8.32 (2) , 8.43 (1) , 8.52 (1) |
| | MS (ES+) :463(M+H) |
| 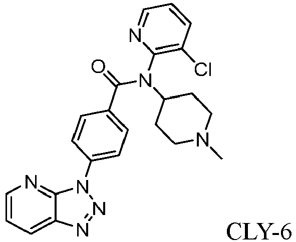 | Chemical Formula: C23H22ClN7O |
| | Molecular Weight: 447.93 |
| | Data in hydrogen spectrum CDCl3: 1.97 (4), 2.33 (3), 2.36 (2), 2.72 (2), 4.7 (1), 6.92 (1), 7.24 (1), 7.55 (1), 7.8 (1), 8.11 (2), 8.32 (2), 8.43 (1), 8.53 (1), |
| | MS (ES+) :448(M+H) |
| 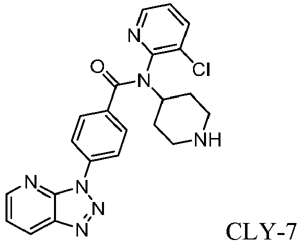 | Chemical Formula: C22H20ClN7O |
| | Molecular Weight: 433.90 |
| | Data in hydrogen spectrum CDCl3: 1.87 (4), 2.26 (3), 2.62 (2), 4.42 (1), 6.91 (1), 7.24 (1), 7.55 (1), 7.83 (1), 8.11 (2), 8.32 (2), 8.43 (2) |
| | MS (ES+) :434(M+H) |
| 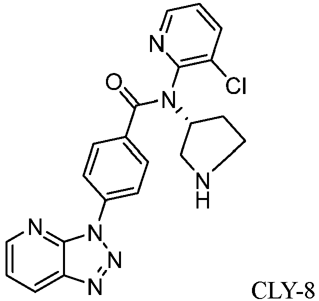 | Chemical Formula: C21H18ClN7O |
| | Molecular Weight: 419.87 |
| | Data in hydrogen spectrum CDCl3: δ2.29 (2H), δ2.57 (1H), δ2.74 (2H), δ3.31 (2H), δ4.61 (1H), δ6.86 ~ 8.87 (10H) |
| | MS (ES+) :420(M+H) |
| 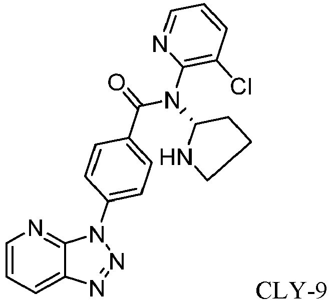 | Chemical Formula: C21H18ClN7O |
| | Molecular Weight: 419.87 |
| | Data in hydrogen spectrum CDCl3: δ1.59 (2H), δ2.10 (2H), δ2.42 (2H), δ2.93 (1H), δ5.95 (1H), δ6.90 ~ 8.97 (10H) |
| | MS (ES+) :420(M+H) |
| 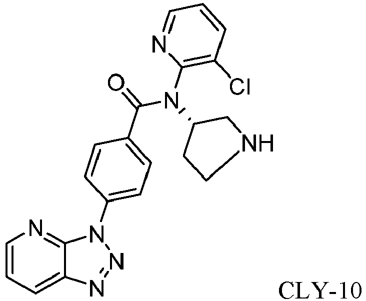 | Chemical Formula: C21H18ClN7O |
| | Molecular Weight: 419.87 |
| | Data in hydrogen spectrum CDCl3: δ2.29 (2H), δ2.57 (1H), δ2.74 (2H), δ3.31 (2H), δ4.61 (1H), δ6.86 ~ 8.87 (10H) |
| | MS (ES+) :420.1(M+H) |
| 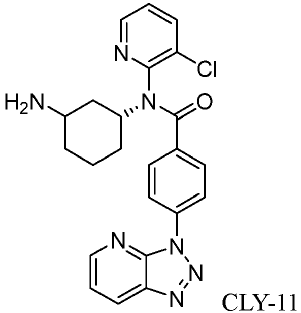 | Chemical Formula: C23H22ClN7O |
| | Molecular Weight: 447.93 |
| | Data in hydrogen spectrum CDCl3: δ1.51 (2H), δ2.04 ∼ 2.72 (8H), δ3.51 (1H), δ4.52 (1H), δ6.75 ∼ 8.97 (10H) |
| | MS (ES+) :448(M+H) |
| 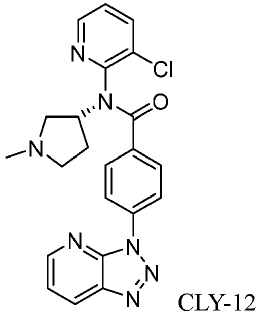 | Chemical Formula: C22H20ClN7O |
| | Molecular Weight: 433.90 |
| | Data in hydrogen spectrum CDCl3: δ1.73 (2H), δ2.18 (3H), δ2.27 ∼ 2.72 (4H), δ4.41 (1H), δ6.89 ∼ 8.76 (10H) |
| | MS (ES+) :434(M+H) |
| 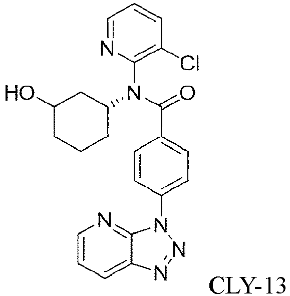 | Chemical Formula: C24H23ClN6O2 |
| | Molecular Weight: 462.94 |
| | Data in hydrogen spectrum CDCl3: δ1.33 ∼ 1.96 (8H), δ3.24 (1H), δ3.34 (3H), δ3.57 (1H), δ6.82 ∼ 8.92 (10H) |
| | MS (ES+) :463(M+H) |

### Example 3: Synthesis of (R) - N - (3-chloropyridin-2-yl) - N - (pyrrolidin-3-yl) - 3 - (1H-tetrazol-5-yl) benzidine (CLY-14)

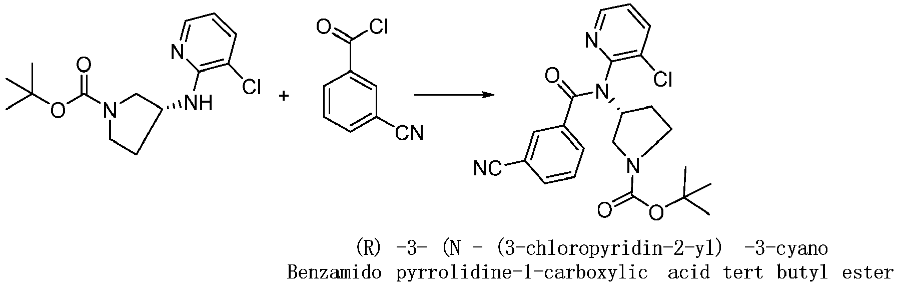

Step 1, as in Example 3, (R) - 3 - (N - (3-chloropyridin-2-yl) - 3-cyanobenzamide) pyrrolidine-1-carboxylic acid tert-butyl ester can be obtained.

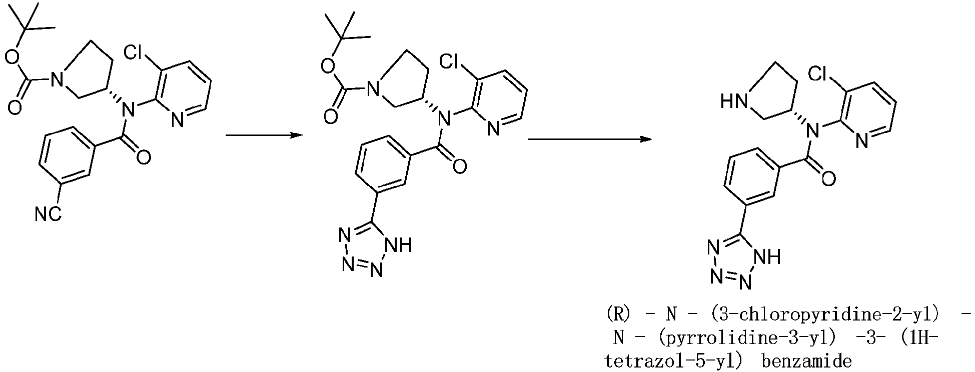

Step 2, (R) - 3 - (N - (3-chloropyridin-2-yl) - 3-cyanobenzamide) pyrrolidine-1-carboxylic acid tert-butyl ester, sodium **azide**, and triethylamine hydrochloride were added to N, N-dimethylformamide, stirred at 100 °C for 20 hours, cooled, poured into the water, added concentrated hydrochloric acid dropwise to pH2-3, filtered to obtain solid, washed with water and dried. Used dichloromethane, dissolved in methanol, added 4mol/L of hydrogen chloride dioxane solution at room temperature, stirred overnight, and concentrated under reduced pressure to obtain solid. After silica gel chromatography, (R) - N - (3-chloropyridin-2-yl) - N - (pyrrolidin-3-yl) - 3 - (1H-tetrazol-5-yl) benzamide was obtained, MS (ES+): 370 (M+H).

### Chemical Formula: C17H16ClN7O

### Molecular Weight: 369.81

Data in hydrogen spectrum CDCl3: 0.77 (1H), 2.29 (2H), 2.59 (2H), 3.29 (2H), 4.53 (1H), 6.92 (1H), 7.24 (1H), 7.43 (1H), 7.63 (1H), 7.83 (1H), 8.21 (1H), 8.64 (1H), 8.72 (1H) .

The following compounds in Table 2 can also be synthesized by referring to the same method:

### Graph 2

| | |
|---|---|
| 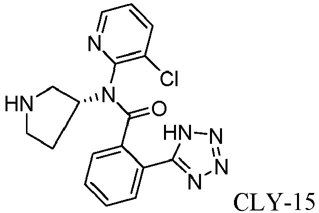 | Chemical Formula: C17H16ClN7O |
| | Molecular Weight: 369.81 |
| | Data in hydrogen spectrum CDCl3: 0.80 (1H), 2.24 (2H), 2.61 (2H), 3.28 (2H), 4.55 (1H), 6.90 (1H), 7.21 (1H), 7.40 (1H), 7.57 (1H), 7.93 (1H), 8.31 (1H), 8.74 (1H), 8.79 (1H)_{∘} |
| | MS (ES+) :370 (M+H) |
| 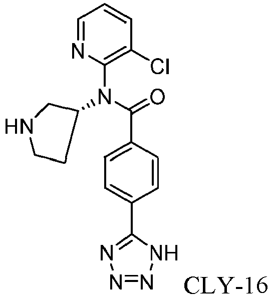 | Chemical Formula: C17H16ClN7O |
| | Molecular Weight: 369.81 |
| | Data in hydrogen spectrum CDCl3: 0.72 (1H), 2.34 (2H), 2.60 (2H), 3.31 (2H), 4.62 (1H), 6.89 (2H), 7.37 (1H), 7.62 (2H), 7.87 (1H), 8.57 (1H), 8.78 (1H)_{∘} |
| | MS (ES+) :370 (M+H) |
| 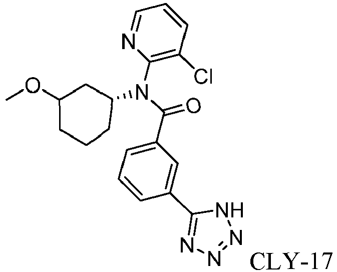 | Chemical Formula: C20H21ClN6O2 |
| | Molecular Weight: 412.88 |
| | Data in hydrogen spectrum CDCl3: 0.57 (1H), 1.1 (1H), 1.65 (2H), 1.73 (1H), 1.82 (2H), 1.94 (1H), 3.35 (3H), 3.42 (1H), 4.58 (1H), 6.92 (1H), 7.75 (1H), 7.86 (1H), 7.94 (1H), 7.97 (1H), 8.02 (1H), 8.55 (1H), 9.13 (1H), |
| | MS (ES+) :413 (M+H) |
| 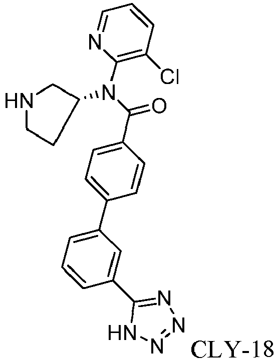 | Chemical Formula: C23H20ClN7O |
| | Molecular Weight: 445.91 |
| | Data in hydrogen spectrum CDCl3: 0.82 (1H), 2.32 (2H), 2.65 (2H), 3.30 (2H), 4.64 (1H), 6.85 (1H), 7.04 (2H), 7.36 (1H), 7.45 (1H), 7.54 (1H), 7.62 (2H), 7.77 (1H), 7.87 (1H), 8.27 (1H), 8.49 (1H)_{∘} |
| | MS (ES+) :446 (M+H) |
| 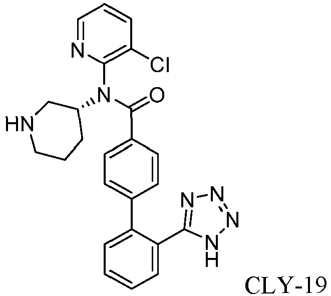 | Chemical Formula: C24H22ClN7O |
| | Molecular Weight: 459.94 |
| | Data in hydrogen spectrum CDCl3: 1.68 (1H), 1.73 (1H), 1.8 (1H), 2.56 (1H), 2.69 (2H), 3.06 (1H), 3.34 (1H), 4.28 (1H), 6.86 (1H), 7.05 (2H), 7.46 (2H), 7.53 (1H), 7.6 (1H), 7.72 (2H), 7.77 (1H), 8.26 (1H), 9.15 (2H), |
| | MS (ES+) :460 (M+H) |
| 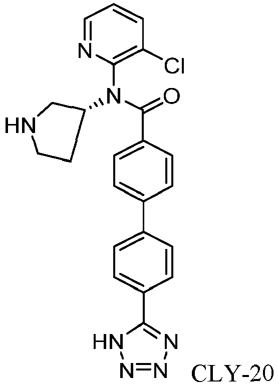 | Chemical Formula: C23H20ClN7O |
| | Molecular Weight: 445.91 |
| | Data in hydrogen spectrum CDCl3: 0.78 (1H), 2.21 (2H), 2.65 (2H), 3.30 (2H), 4.70 (1H), 6.82 (1H), 7.01 (2H), 7.38 (2H), 7.59 (1H), 7.65 (2H), 7.87 (2H), 8.57 (1H), 8.79 (1H)_{∘} |
| | MS (ES+) :446 (M+H) |
| 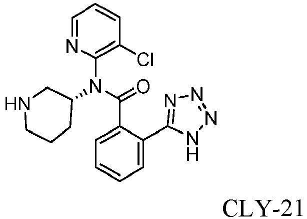 | Chemical Formula: C18H18ClN7O |
| | Molecular Weight: 383.84 |
| | Data in hydrogen spectrum CDCl3: 1.58 (1H), 1.73 (1H), 1.80 (1H), 2.56 (1H), 2.71 (2H), 3.06 (1H), 3.34 (1H), 4.28 (1H), 6.86 (1H), 7.68 (1H), 7.77 (1H), 7.85 (1H), 7.89 (1H), 8.32 (1H), 8.68 (1H), 9.45 (2H), |
| | MS (ES+) :384 (M+H) |
| 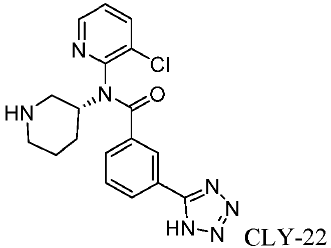 | Chemical Formula: C18H18ClN7O |
| | Molecular Weight: 383.84 |
| | Data in hydrogen spectrum CDCl3: 1.68 (1H), 1.73 (1H), 1.8 (1H), 2.56 (1H), 2.71 (2H), 3.06 (1H), 3.34 (1H), 4.28 (1H), 6.86 (1H), 7.68 (1H), 7.77 (1H), 7.85 (1H), 7.89 (1H), 8.02 (1H), 8.35 (1H), 10.45 (2H), |
| | MS (ES+) :384 (M+H) |
| 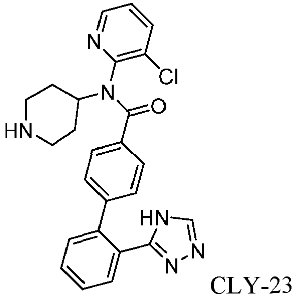 | Chemical Formula: C25H23ClN6O |
| | Molecular Weight: 458.95 |
| | Data in hydrogen spectrum CDCl3: 1.82 (2H), 1.9 (2H), 2.72 (2H), 3.14 (2H), 4.72 (1H), 5.81 (2H), 6.92 (1H), 7.02 (2H), 7.33 (1H), 7.42 (2H), 7.75 (2H), 7.8 (1H), 8.31 (1H), 8.49 (1H), 8.89 (1H), |
| | MS (ES+) :459 (M+H) |
| 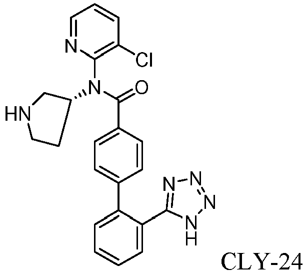 | Chemical Formula: C23H20ClN7O |
| | Molecular Weight: 445.91 |
| | Data in hydrogen spectrum CDCl3: 0.84 (1H), 2.29 (2H), 2.62 (2H), 3.32 (2H), 4.65 (1H), 6.89 (1H), 6.97 (2H), 7.26 (1H), 7.42 (1H) 7.59 (1H), 7.67 (2H), 7.97 (1H), 8.27 (1H), 8.67 (1H), 8.98 (1H)_{∘} |
| | MS (ES+) :446 (M+H) |
| 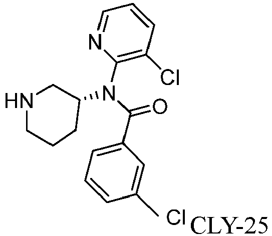 | Chemical Formula: C17H17Cl2N3O |
| | Molecular Weight: 350.24 |
| | Data in hydrogen spectrum CDCl3: δ1.5 ~ 2.07 (4H), δ2.01 (1H), 62.42 ~ 2.85 (4H), δ3.41 (1H), δ7.74 ~ 8.43 (7H) |
| | MS (ES+) :351(M+H) |
| 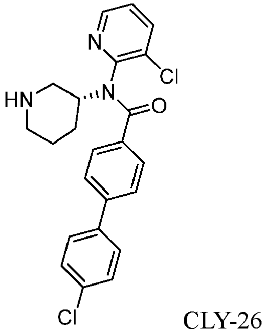 | Chemical Formula: C23H21Cl2N3O |
| | Molecular Weight: 426.34 |
| | Data in hydrogen spectrum CDCl3: δ1.51 ~ 2.02 (4H), δ2.05 (1H), δ2.41 ~ 2.82 (4H), δ3.371 (1H), δ7.64 ~ 8.33 (11H) |
| | MS (ES+) :427(M+H) |
| 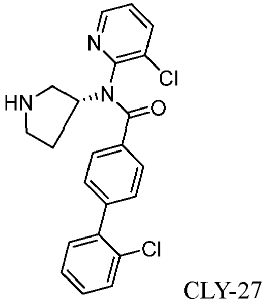 | Chemical Formula: C22H19Cl2N3O |
| | Molecular Weight: 412.31 |
| | Data in hydrogen spectrum CDCl3: 0.77 (1H), 2.22-2.30 (2H), 2.70 (2H), 3.32 (2H), 4.63 (1H), 6.86 (1H), 7.01 (1H), 7.36 (1H), 7.37 (2H), 7.68 (2H), 7.77 (1H), 7.75 (2H), 8.45 (1H) _{∘} |
| | MS (ES+) :413 (M+H) |
| 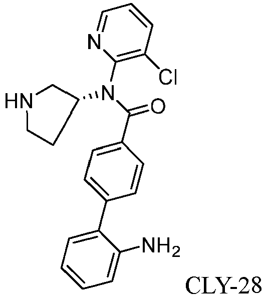 | Chemical Formula: C22H21ClN4O |
| | Molecular Weight: 392.89 |
| | Data in hydrogen spectrum CDCl3: 0.73 (1H), 2.25 (2H), 2.78 (2H), 3.01 (2H), 3.32 (2H), 4.61 (1H), 6.96 (1H), 7.07 (2H), 7.24 (1H), 7.36 (1H), 7.44 (1H), 7.56 (1H), 7.67 (2H), 7.77 (1H), 8.61 (1H)_{∘} |
| | MS (ES+) :393 (M+H) |
| 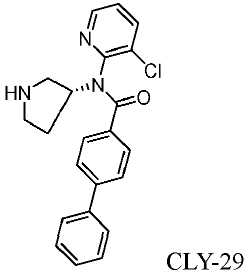 | Chemical Formula: C22H20ClN3O |
| | Molecular Weight: 377.87 |
| | Data in hydrogen spectrum CDCl3: 0.76 (1H), 2.29-2.32 (2H), 2.74 (2H), 3.31 (2H), 4.61 (1H), 6.86 (1H), 7.04 (2H), 7.36 (1H), 7.40 (2H), 7.67 (2H), 7.77 (1H), 7.87 (2H), 8.86 (1H)_{∘} |
| | MS (ES+) :378 (M+H) |
| 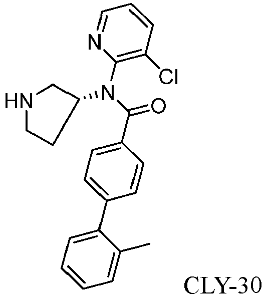 | Chemical Formula: C23H22ClN3O |
| | Molecular Weight: 391.90 |
| | Data in hydrogen spectrum CDCl3: 0.76 (1H), 2.29-2.32 (2H), 2.74 (2H), 2.87 (3), 3.21 (2H), 4.57 (1H), 6.89 (1H), 7.01 (2H), 7.36 (3H), 7.57 (2H), 7.77 (1H), 7.87 (1H), 8.36 (1H)_{∘} |
| | MS (ES+) :392(M+H) |
| 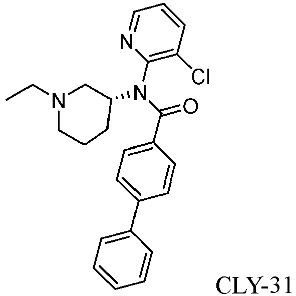 | Chemical Formula: C25H26ClN3O |
| | Molecular Weight: 419.95 |
| | Data in hydrogen spectrum CDCl3 : 0.75 (3H), 0.92 (2H), 2.12 (2H) 2.29 (2H), 2.74 (2H), 3.31 (2H), 4.61 (1H), 6.86 (1H), 7.04 (2H), 7.36 (1H), 7.40 (2H), 7.67 (2H), 7.77 (1H), 7.87 (2H), 8.60 (1H)_{∘} |
| | MS (ES+) :420 (M+H) |
| | |
| 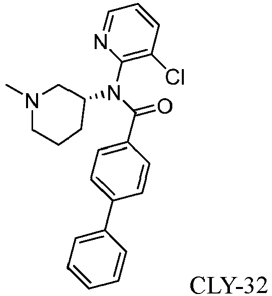 | Chemical Formula: C24H24ClN3O |
| | Molecular Weight: 405.93 |
| | Data in hydrogen spectrum CDCl3: 0.91 (2H) , 1.78 (1H) , 2.12 (1H) , 2.28 (3H) , 2.69 (1H) , 2.89 (1H) , 3.28 (1H) , 3.36 (1H) , 4.35 (1H) , 6.86 (1H) , 7.04 (2H) , 7.36 (1H) , 7.4 (1H) , 7.4 (1H) , 7.67 (2H) , 7.77 (1H) , 7.87 (1H) , 7.87 (1H) , 8.56 (1H) |
| 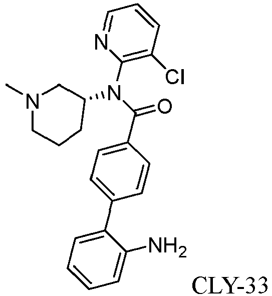 | Chemical Formula: C24H25ClN4O |
| | Molecular Weight: 420.94 |
| | Data in hydrogen spectrum CDCl3: 0.92 (2H), 1.93 (2H), 2.12 (3H) 2.29 (2H), 2.74 (2H), 3.31 (2H), 4.61 (1H), 6.86 (1H), 7.04 (2H), 7.36 (1H), 7.40 (2H), 7.52 (1H), 7.67 (1H), 7.73 (1H), 7.84 (1H), 8.26 (1H) |
| | MS (ES+) :421 (M+H) |
| 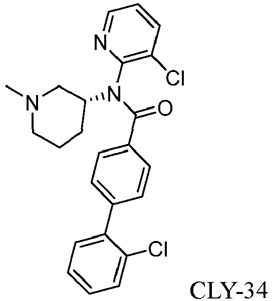 | Chemical Formula: C24H23Cl2N3O |
| | Molecular Weight: 440.37 |
| | Data in hydrogen spectrum CDCl3: 0.92 (2H), 2.12 (3H) 2.29 (2H), 2.74 (2H), 3.31 (2H), 4.61 (1H), 6.86 (1H), 7.04 (2H), 7.36 (1H), 7.40 (2H), 7.52 (1H), 7.67 (1H), 7.77 (1H), 7.87 (1H), 8.26 (1H) MS (ES+) :441 (M+H) |
| 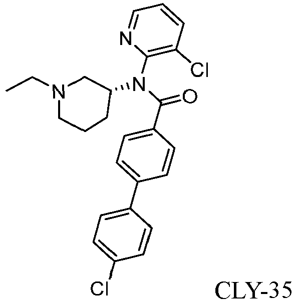 | Chemical Formula: C25H25Cl2N3O |
| | Molecular Weight: 454.40 |
| | Data in hydrogen spectrum CDCl3: 0.81 (3H), 0.92 (2H), 2.12 (2H) 2.29 (2H), 2.74 (2H), 3.31 (2H), 4.61 (1H), 6.86 (1H), 7.04 (2H), 7.36 (1H), 7.40 (2H), 7.67 (2H), 7.87 (2H), 8.36 (1H) |
| | MS (ES+) :455(M+H) |
| 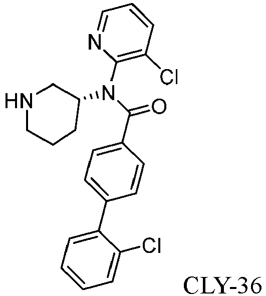 | Chemical Formula: C23H21Cl2N3O |
| | Molecular Weight: 426.34 |
| | Data in hydrogen spectrum CDCl3: 1.68 (2), 1.8 (1), 2.56 (1), 2.69 (2), 3.06 (1), 3.34 (1), 4.28 (1), 4.9 (1), 6.8 (2), 6.86 (1), 7.15 (3), 7.38 (1), 7.76 (3), 8.42 (1), |
| | MS (ES+) :427(M+H) |
| 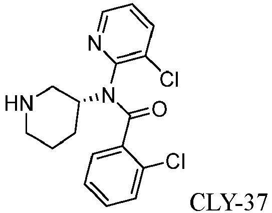 | Chemical Formula: C17H17Cl2N3O |
| | Molecular Weight: 350.24 |
| | Data in hydrogen spectrum CDCl3: δ1.5 ~ 2.07 (4H), δ2.01 (1H), 62.42 ~ 2.85 (4H), δ3.41 (1H), δ7.74 ~ 8.43 (7H) |
| | MS (ES+) :351(M+H) |
| 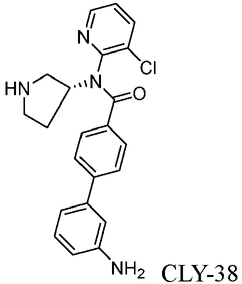 | Chemical Formula: C22H21ClN4O |
| | Molecular Weight: 392.89 |
| | Data in hydrogen spectrum CDCl3: 0.69 (1H), 2.22 (2H), 2.73 (2H), 2.98 (2H), 3.38 (2H), 4.61 (1H), 6.92 (1H), 7.01 (2H), 7.19 (1H), 7.32 (1H), 7.41 (1H), 7.46 (1H), 7.62 (2H), 7.87 (1H), 8.26 (1H) |
| | MS (ES+) :393 (M+H) |
| 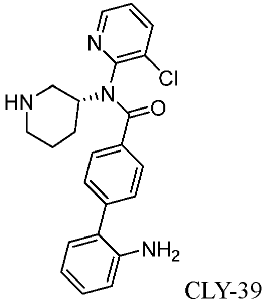 | Chemical Formula: C23H23ClN4O |
| | Molecular Weight: 406.91 |
| | Data in hydrogen spectrum CDCl3: 1.73 (2), 1.8 (1), 2.56 (1), 2.71 (2), 3.06 (1), 3.34 (1), 4.28 (1), 4.42 (3), 6.53 (1), 6.72 (2), 6.86 (2), 6.98 (1), 7.26 (1), 7.56 (2), 7.77 (1), 8.43 (1), |
| | MS (ES+) :407 (M+H) |
| 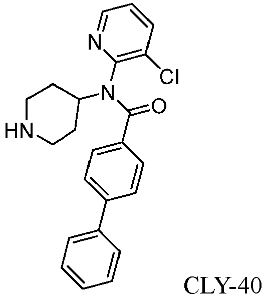 | Chemical Formula: C23H22ClN3O |
| | Molecular Weight: 391.90 |
| | Data in hydrogen spectrum CDCl3: 1.63-1.73 (2), 1.8 (1), 2.56 (1), 2.69 (2), 3.06 (1), 3.34 (1), 4.28 (1), 4.9 (1), 6.86 (1), 6.92 (2), 7.36 (3), 7.67 (2), 7.77 (1), 7.87 (2), 8.31 (1), |
| | MS (ES+) :392(M+H) |
| 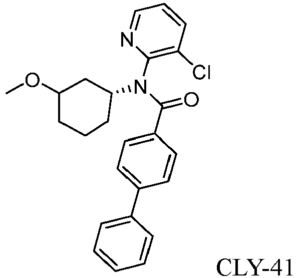 | Chemical Formula: C25H25ClN2O2 |
| | Molecular Weight: 420.94 |
| | Data in hydrogen spectrum CDCl3: 0.57 (1H), 1.1 (1H), 1.65 (2H), 1.73 (1H), 1.82 (2H), 1.94 (1H), 3.35 (3H), 3.42 (1H), 4.58 (1H), 6.92 (1H), 7.1 (2H), 7.36 (1H), 7.4 (2H), 7.76 (2H), 7.86 (1H), 7.87 (2H), 8.95 (1H), |
| | MS (ES+) :421 (M+H) |

### Example 4: Synthesis of (R) - 4 - (3H - [1,2,3] triazolo [4,5-b] pyridin-3-yl) - N - (3-chloropyridin-2-yl) - N - (tetrahydro-2H-pyran-3-yl) benzamide (CLY-42).

### Step 1: Synthesis of (R) - 3-chloro-N - (tetrahydro-2H-pyran-3-yl) pyridin-2-amine.

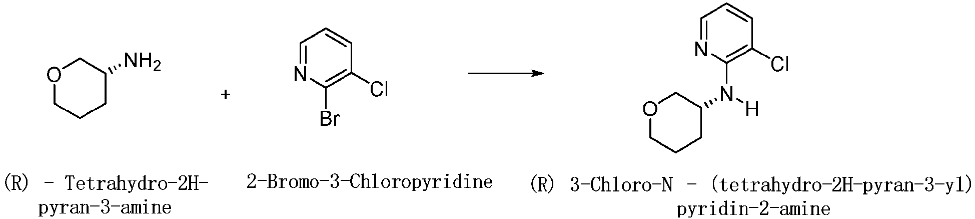

(R) - tetrahydro-2H-pyran-3-amine, 2-bromo-3-chloropyridine, sodium tert-**butanol** and tetrahydrofuran were added into a 250ml three-necked bottle, protected by nitrogen, stirred, added palladium acetate, 1,1 '- biphenyl-2-naphthol, tris (dimethylamino) phosphine, heated to 65 °C, reacted for 12 hours, stopped the reaction, poured into the water, extracted with ethyl acetate in batches, combined with the ethyl acetate layer, washed with the water, and recovered the ethyl acetate layer to obtain the residual paste, (R) - 3-chloro-N - (tetrahydro-2H-pyran-3-yl) pyridin-2-amine was obtained by silica gel column chromatography.

### Step 2:

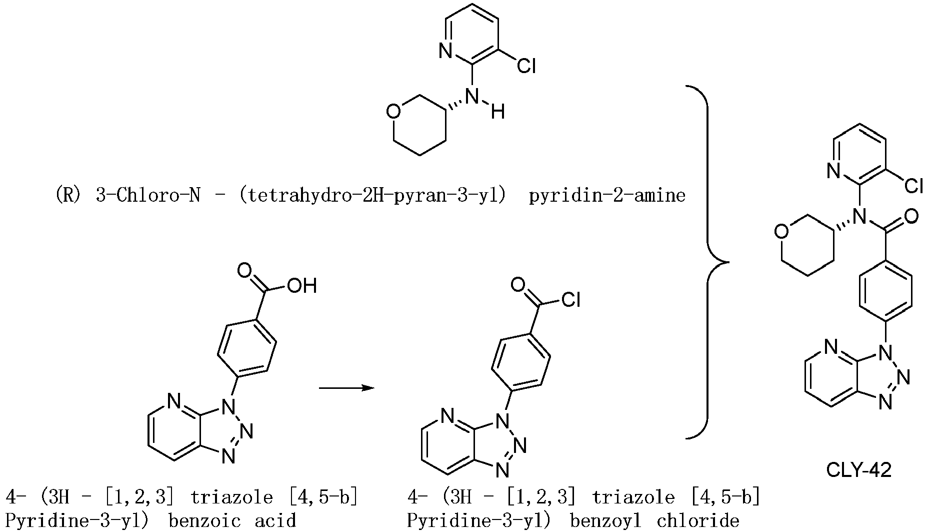

4 - (3H - [1,2,3] triazole [4,5-b] pyridin-3-yl) benzoic acid was added to dichloromethane, oxalyl chloride and catalyst N, N-dimethylformamide were added to react at room temperature until it was clear, and the solvent was recovered under reduced pressure. The residue was added to tetrahydrofuran solvent, and the tetrahydrofuran solution of (R) - 3-chloro-N - (tetrahydro-2H-pyran-3-yl) pyridin-2-amine, the product of the previous step, was added. The temperature was cooled to below 5 °C in the ice water bath, and the bis (trimethylsilyl) amine lithium solution was added, Stirred the reaction at room temperature overnight, poured it into the water, extracted it with ethyl acetate, washed it with water, recovered the ethyl acetate under reduced pressure, and obtained the residue through column chromatography to obtain (R) - 4 - (3H - [1,2,3] triazolo [4,5-b] pyridin-3-yl) - N - (3-chloropyridin-2-yl) - N - (tetrahydro-2H-pyran-3-yl) benzamide, MS (ES+): 435 (M+H).

Referring to the same methods, we can also synthesize 43) . 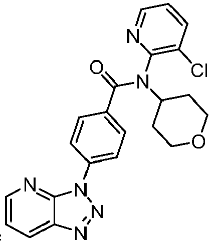 < CLY-

### Example 5: Synthesis of ethyl 1 - (5 - (4 - (4 - ((3-chloropyridin-2-yl) (((R) - pyrrolidin-3-yl) carbamoyl) phenyl) - 1-methyl-1H-pyrazol-5-yl) - 2H-tetrazol-2-yl) methyl isobutyrate (CLY-44).

### Step 1:

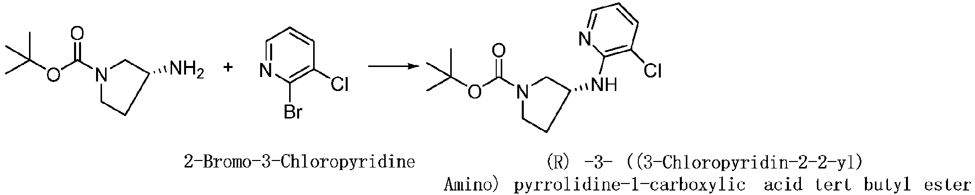

### (1) Synthesis of (R) - 3 - ((3-chloropyridin-2-2-yl) amino) pyrrolidine-1-carboxylic acid tert-butyl ester.

(R)-1-tert-butyloxycarbonyl-3-aminopyrrolidine, 2-bromo-3-chloropyridine, sodium tert-butanol, and toluene were added into a three-necked bottle, protected by nitrogen, and then stirred. Palladium acetate, 1,1 '- biphenyl-2-naphthol, **and** tris (dimethylamino) phosphine were added, heated to 100 °C, and reacted for 7 hours. After stopping the reaction, the mixture was poured into the water, extracted with ethyl acetate in batches, combined with ethyl acetate layer, and washed with water, and the ethyl acetate layer was recovered to obtain residual paste, through silica gel column chromatography, (R) - 3 - ((3-chloropyridin-2-yl) amino) pyrrolidine-1-carboxylic acidtert-butyl ester was obtained.

### Step 2:

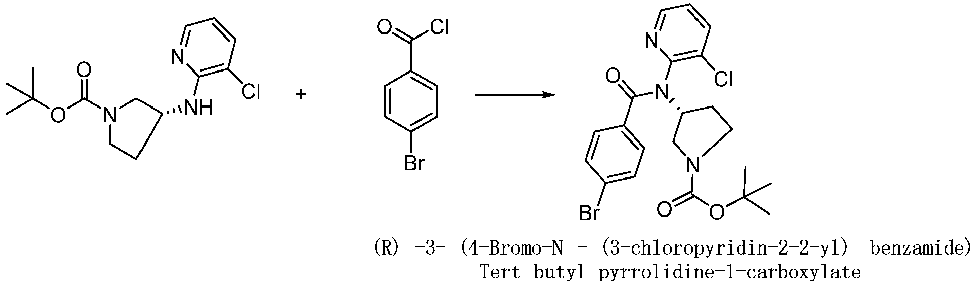

At 0 °C, 1M bis (trimethylsilyl) amine lithium solution was added to (R) - 3 - ((3-chloropyridin-2-yl) amino) pyrrolidine-1-carboxylic **acid** tert-butyl ester and 4-bromobenzoyl chloride in tetrahydrofuran solution, stirred overnight at room temperature, diluted with ethyl acetate and washed with water, decompressed and concentrated the ethyl acetate layer to obtain the residue column chromatography, and obtained (R) - 3 - (4-bromo-N - (3-chloropyridin-2-yl) benzamide) pyrrolidine-1-carboxylic acid tert-butyl ester.

### Step 3: Preparation of (R) - 3 - (N - (3-chloropyridin-2-yl) - 4 - (4,4,5,5-tetramethyl-1,3,2-dioxabiran-2-yl) benzamide) pyrrolidine-1-carboxylic acid tert-butyl ester.

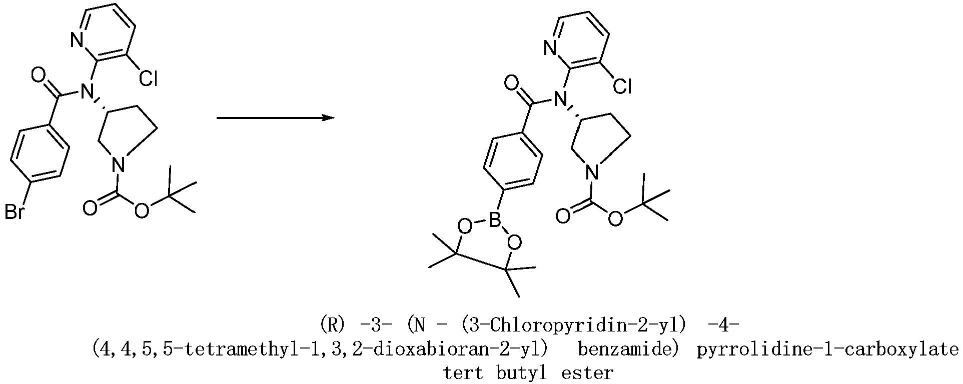

Added (R) - 3 - (4-bromo-N - (3-**chloropyridin**-2-2-yl) benzamide) pyrrolidine-1-carboxylic acid tert-butyl ester, bis (pinacol) diboron, potassium acetate into 1,4-dioxane and stirred, added palladium chloride after nitrogen protection, heated to 100 °C and stirred for 5 hours, and concentrated under reduced pressure, The residue was separated by silica gel column chromatography to obtain (R) - 3 - (N - (3-chloropyridin-2-yl) - 4 - (4,4,5,5-tetramethyl-1,3,2-dioxaborolan-2-yl) benzamide) pyrrolidine-1-carboxylic acid tert-butyl ester.

### Step 4: Synthesis of 1 - (5 - (4 - (4 - ((3-chloropyridin-2-yl) (((R) - pyrrolidin-3-yl) carbamoyl) phenyl) - 1-methyl-1H-pyrazol-5-yl) - 2H-tetrazol-2-yl) ethyl isobutyrate.

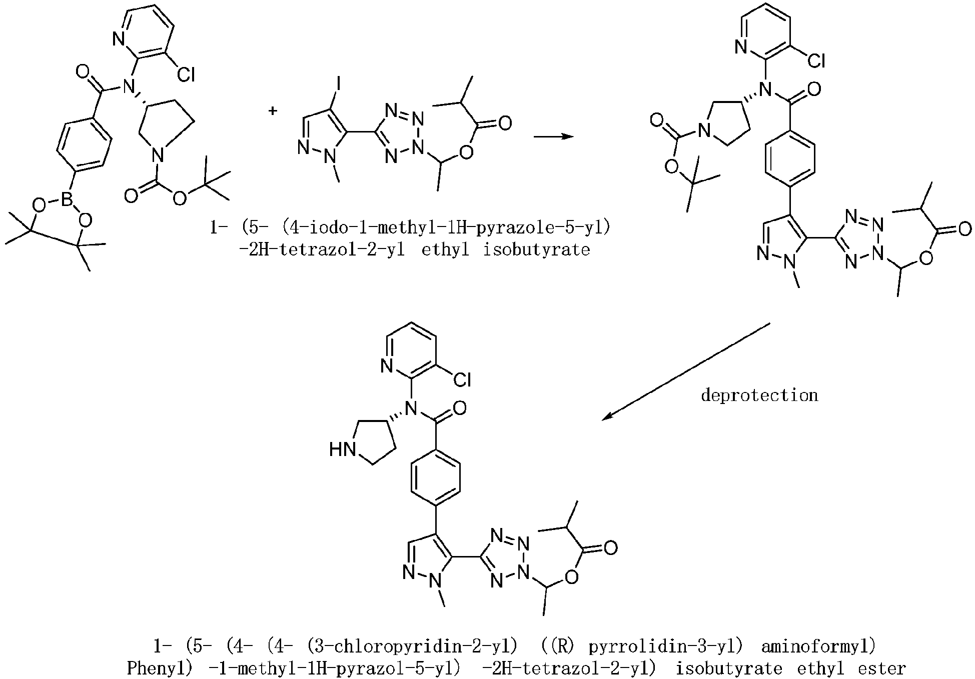

Added (R) - 3 - (N - (3-chloropyridin-2-yl) - 4 - (4,4,5,5-tetramethyl-1,3,2-dioxabioran-2-yl) benzamide) pyrrolidine-1-carboxylic acid tert-butyl ester, 1 - (5 - (4-iodo-1-methyl-1H-pyrazole-5-yl) - **2H**-tetrazol-2-yl) isobutyrate ethyl ester, tripotassium phosphate, methanesulfonic acid (2-dicyclohexylphosphine-2 ', 6' - dimethoxy-1,1 '- biphenyl) (2' - amino-1,1 '- biphenyl-2-yl) palladium (II) to 50% 1, 4 - In dioxane aqueous solution, left reacted at 100 °C for 5 hours under nitrogen protection. After the reaction solution was reduced to room temperature, diluted it with 200ml ethyl acetate, separated the ethyl acetate layer, washed it with water for three times, dried it with anhydrous sodium sulfate, and recovered the ethyl acetate under reduced pressure to obtain the residue. Dissolved the residue in dichloromethane, added 1, 4-dioxane hydrogen chloride solution and stirred for 2 hours, recovered the solvent to dryness under reduced pressure, and separated with silica gel chromatography to obtain 1 - (5 - (4 - (4 - ((3-chloropyridin-2-yl) (((R) - pyrrolidin-3-yl) carbamoyl) phenyl) - 1-methyl-1H-pyrazol-5-yl) - 2H-tetrazol-2-yl) ethyl isobutyrate. MS (ES+) :565 (M+H).

Referring to the same methods, we can also synthesize (CLY-45) .

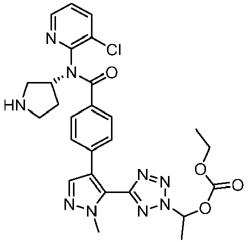

### Example 6 Pharmacokinetic properties of rats

### 1. Experimental method:

Male SD rats were randomly assigned to each group, 12 rats in each compound group, and then randomly divided into two groups. Six rats were administered intravenously (1mg/kg), and blood was collected and plasma was separated respectively at 0.0833, 0.25, 0.5, 1, 2, 4, 6, 8, and 24 hours after administration. The other six rats were administered intragastrically (5mg/kg), and blood was collected and plasma was separated respectively at 0.25, 0.5, 1, 2, 4, 6, 8, and 24 hours after administration. The concentration of each compound in plasma was determined by LC/MS/**MS** method, and the relevant pharmacokinetic parameters were calculated by using the software Phoenix WinNonlin 6.2.1, the bioavailability in rats was calculated, and the pharmacokinetic properties of each compound were evaluated.

### 2. Experimental results

Among all the above compounds, compounds CLY-1, CLY-2, CLY-3, CLY-4, CLY-5, CLY-8, CLY-11, CLY-13, CLY-17, CLY-18, CLY-19, CLY-21, CLY-26, CLY-28, CLY-30, CLY-32, CLY-35, CLY-36, CLY-37, CLY-39, and CLY-44 **have** good bioavailability in rats, 69.8%, 72.6%, 69.5%, 59.5%, 53.8%, 78.2%, 63.5%, 52.3%, 52.5%, 46.9%, 66.3%, 49.6%, 48.5%, 42.6%, 46.2%, 58.6%, respectively. 62.2% 68.6%, 56.5%, 65.6%, 50.6%, indicating that the above compounds have good pharmacokinetic properties.

### Example 7 Effect of compounds CLY on chloasma model of guinea pig

### 1. Experimental method

### 1.1 Experimental materials

1.1.1 Reagents: progesterone (20 mg/ml) was purchased from Shanghai CP General Pharmaceutical Co., Ltd., arbutin ointment was purchased from Shanghai Xinxianfeng Pharmaceutical Co., Ltd., and tyrosine, malondialdehyde (**MDA**), superoxide dismutase (SOD) kits were purchased from Nanjing Jiancheng Bioengineering Institute.
1.1.2 Instrument: DY89 - II electric glass homogenizer was purchased from Ningbo Xinlan Biotechnology Co., Ltd., and the system biological **microscope** (Image-Pro Plus 6.0) was purchased from Media Cybernetics company of the United States.
1.1.3 Experimental animals: SPF grade healthy pure female **guinea** pigs, weighing (230 ± 30) g, from Shanghai Slac Experimental Animal Technology Co., Ltd.
1.1.4 Preparation method of treatment cream: The **excipient** matrix components include methyl silicone oil (15%), stearic acid (6%), white vaseline (5%), liquid paraffin (5%), octadecanol (5%), glycerin (20%), alkyl aryl polyglycol ether (1%), fatty alcohol polyoxyethylene ether (1%), tween-807 (1%), ethyl paraben (0.1%), distilled water (about 31-55%), and are mixed with appropriate amount of CLY series compounds to form 0.25% mixed emulsion. The cream matrix used in this embodiment refers to the matrix components after the active ingredients are removed from the cream.

### 1.2 Animal grouping and modeling

According to the body weight, they were randomly **divided** into the model control group (applied cream matrix), blank control group (applied cream matrix), CLY-1 treatment group (applied 0.25% CLY-1 cream on the skin), CLY-2 treatment group (0.25% CLY-2 cream on the skin), CLY-3 treatment group (0.25% CLY-3 cream on the skin), CLY-4 treatment group (0.25% CLY-4 cream on the skin), CLY-5 treatment group (0.25% CLY-5 cream on the skin) CLY-8 treatment group (0.25% CLY-8 cream on the skin), CLY-11 treatment group (0.25% CLY-11 cream on the skin), CLY-19 treatment group (0.25% CLY-19 cream on the skin), CLY-36 treatment group (0.25% CLY-36 cream on the skin), CLY-39 treatment group (0.25% CLY-39 cream on the skin), positive treatment group (0.25% arbutin cream on the skin), 6 in each group. In addition to the blank control group, all guinea pigs in the other groups were injected with 20 mg/ml progesterone injection (7.5 mg/kg) into the muscle at the root of the hind leg, once a day, for 30 consecutive days to establish the chloasma model. The model of guinea pig was successfully replicated when the skin of the back model area showed uniform and stable dark brown spots with clear boundaries. After modeling, the guinea pigs in the model control group, blank control group, each CLY series treatment group, and positive treatment group were treated with corresponding cream on the back for intervention, once a day, for 30 consecutive days.

### 1.3 Observation indicators

### (1) Determination of tyrosine, MDA content, and SOD activity

Took one piece of skin tissue from each guinea pig, separately. Washed it with precooled normal saline, removed subcutaneous fat and other connective tissue, and then wiped it dry. Each piece of skin tissue was cut to 0.5g. Then put it into **five** small tubes containing 2.0 ml of precooled normal saline. The high-speed dispersion machine homogenizes at a speed of 10 r/min, lasted for 10 seconds, repeated once, centrifuged at a speed of 3500 r/m for 15 minutes, and took the supernatant. Tyrosine was determined by HPLC, MDA was determined by the thiobarbituric acid method, SOD was determined by the xanthine oxidase method, and tyrosine, MDA content, and SOD activity were determined according to the instructions of the kit.

### (2) Pathomorphological observation of skin melanocytes

Took 1 piece of spare skin tissue from each of all guinea pigs, about 2 cm × 1 cm, 10% paraformaldehyde fixation, pathological tissue detection, immunohistochemical staining, observation of melanocyte staining and number, and judgment of **positive** cells according to the literature: none, 0 point< 15%, 0.5 points< 30%, 1 point;> 30%, 2 points. Observed 5 visual fields in each slice, found out the positive target with brown reaction in the cytoplasm of skin epidermis and accessory epithelial cells, and used BX50F4 Beihang pathological image analysis system for quantitative analysis to obtain the average area, the ratio of target area to statistical field area (areal density), the ratio of target number to statistical field area (number density), the average gray level, the average optical density and the integrated optical density of each guinea pig's 5 visual fields of melanin positive target.

### 1.4 Statistical methods

SPSS16.0 software was used for statistics. The measurement data were expressed by mean ± standard deviation (x ± s). The comparison between multiple groups was performed by one-way ANOVA, and the comparison between groups was performed by t-test. P<0.05 was statistically significant.

### 2. Experimental results

### (1) Tyrosine, MDA content, and SOD activity of guinea pigs in each group

The detection results of tyrosine, MDA content, and SOD **activity** of guinea pigs in each group are shown in Table 3. The content of tyrosine and MDA in the skin of guinea pigs in the model group was higher than that in the blank group, and the activity of SOD was lower than that in the blank group, indicating that the skin chloasma model was successfully established; Compared with the model group, the content of tyrosine and MDA in the skin of the CLY series treatment group and the positive treatment group were lower, while the activity of SOD was higher, the difference was statistically significant (P<0.05).

**Table 3 Comparison of tyrosine, MDA content and SOD activity of guinea pigs in each group**

| Group | n | tyrosine (ug) | MDA (nmol/ml) | SOD (NU/ml) |
|---|---|---|---|---|
| Model group | 6 | 0.68±0.16 | 13.73±1.62 | 86.63±12.72 |
| Blank group | 6 | 0.42±0.06* | 10.16±0.95* | 107.12±7.35* |
| CLY-1group | 6 | 0.45±0.07* | 11.32±1.15* | 103.65±9.63* |
| CLY-2group | 6 | 0.43±0.06* | 10.86±1.19* | 105.23±9.82* |
| CLY-3group | 6 | 0.51±0.07* | 11.57±1.18* | 97.45±9.23* |
| CLY-4group | 6 | 0.53±0.06* | 11.62±1.15* | 95.12±8.65* |
| CLY-5group | 6 | 0.43±0.05* | 10.65±1.14* | 105.89±9.86* |
| CLY-8group | 6 | 0.43±0.03* | 10.59±1.12* | 105.68±9.75* |
| CLY-11group | 6 | 0.54±0.06* | 11.62±1.21* | 95.13±9.16* |
| CLY-19group | 6 | 0.56±0.07* | 11.75±1.26* | 96.53±9.21* |
| CLY-36group | 6 | 0.58±0.08* | 11.83±1.27* | 93.42±9.03* |
| CLY-39group | 6 | 0.46±0.05* | 10.81±1.23* | 103.16±9.65* |
| Positive group | 6 | 0.49±0.09* | 11.82±1.43* | 101.26±10.36* |

| | | | | |
|---|---|---|---|---|
| *Compared with the model group, P<0.05 | | | | |

### (2) The area, quantity, and depth of melanocytes in guinea pigs in each group

The area, quantity, and depth of melanocytes in guinea pigs in each group are shown in Table 4 and Table 5. Compared with the blank group, the area of melanin deposition in the skin of guinea pigs in the model group increased, the number of **melanocytes** increased, the optical density increased, and the color deepened; Compared with the model group, the area of melanosis, the number of melanocytes, the optical density and the color of the skin of guinea pigs in each CLY series treatment group and positive treatment group decreased.

**Table 4 Comparison of area and quantity of guinea pig melanocytes in each group**

| Group | n | Area (um2) | Area density | Number | Number density |
|---|---|---|---|---|---|
| Model group | 6 | 2335.12±436.45 | 0.013±0.005 | 3.24±1.23 | 5.53±1.69 |
| Blank group | 6 | 316.97±55.12* | 0.003±0.001* | 1.14±0.38* | 1.67±0.81* |
| CLY-1group | 6 | 372.31±58.32* | 0.005±0.002* | 1.93±0.55* | 1.91±0.96* |
| CLY-2group | 6 | 366.26±62.15* | 0.004±0.002* | 1.75±0.53* | 1.88±1.34* |
| CLY-3group | 6 | 436.32±79.12* | 0.008±0.003* | 2.13±0.61* | 2.82±1.25* |
| CLY-4group | 6 | 496.56±86.23* | 0.009±0.003* | 2.45±0.67* | 2.95±1.41* |
| CLY-5group | 6 | 345.31±56.21* | 0.004±0.001* | 1.43±0.32* | 1.75±1.29* |
| CLY-8group | 6 | 341.26±54.82* | 0.003±0.001* | 1.36±0.29* | 1.71±1.26* |
| CLY-11group | 6 | 417.53±73.16* | 0.006±0.002* | 1.98±0.56* | 2.16±1.38* |
| CLY-19group | 6 | 405.62±73.45* | 0.006±0.003* | 1.95±0.52* | 2.18±1.35* |
| CLY-36group | 6 | 495.32±79.43* | 0.007±0.004* | 1.98±0.59* | 2.67±1.38* |
| CLY-39group | 6 | 458.06±76.32* | 0.006±0.003* | 1.39±0.32* | 2.03±1.24* |
| Positive group | 6 | 489.13±95.67* | 0.006±0.002* | 2.39±0.72* | 2.29±1.65* |

| | | | | | |
|---|---|---|---|---|---|
| *Compared with model group, P<0.05 | | | | | |

**Table 5 Comparison of melanocyte depth of guinea pigs in each group**

| Group | n | Average gray level | Average optical density | Integrated optical density |
|---|---|---|---|---|
| Model group | 6 | 0.68±0.16 | 13.59±1.45 | 87.65±11.32 |
| Blank group | 6 | 0.39±0.06* | 10.16±0.95* | 108.63±8.12* |
| CLY-1group | 6 | 0.43±0.07* | 11.23±1.21* | 103.34±9.71* |
| CLY-2group | 6 | 0.41±0.06* | 10.68±1.18* | 106.96±9.84* |
| CLY-3 group | 6 | 0.52±0.08* | 11.42±1.32* | 95.36±9.86* |
| CLY-4group | 6 | 0.58±0.09* | 11.61±1.38* | 92.45±9.36* |
| CLY-5group | 6 | 0.40±0.05* | 10.59±1.13* | 107.06±9.57* |
| CLY-8group | 6 | 0.39±0.04* | 10.48±1.11* | 103.15±9.52* |
| CLY-11group | 6 | 0.46±0.06* | 11.26±1.22* | 101.38±9.92* |
| CLY-19group | 6 | 0.45±0.06* | 11.28±1.24* | 102.65±9.97* |
| CLY-36group | 6 | 0.47±0.07* | 11.53±1.22* | 97.62±9.91* |
| CLY-39group | 6 | 0.47±0.07* | 11.06±1.17* | 101.36±9.72* |
| Positive group | 6 | 0.42±0.05* | 11.91±1.36* | 97.96±10.37* |

| | | | | |
|---|---|---|---|---|
| *Compared with model group, P<0.05 | | | | |

### 3. Experimental conclusion

CLY-1, CLY-2, CLY-3, CLY-4, CLY-5, CLY-8, CLY-11, CLY-19, CLY-36, and CLY-39 can,by improving SOD enzyme activity in **skin** tissue, reduce the content of tyrosine and MDA, inhibit the tyrosinase activity of melanocytes and melanoma cells, enhance the redox reaction of skin cells, reduce the production of free radicals, and inhibit the formation of melanin, thereby treating chloasma.

### Example 8 Effect of compound CLY series on rat scar model

### 1. Experimental method

1.1 Preparation method of treatment cream: The excipient matrix components included methyl silicone oil (15%), stearic acid (6%), white vaseline (5%), liquid paraffin (5%), octadecanol (5%), glycerin (20%), alkylaryl polyglycol ether (1%), fatty alcohol polyoxyethylene ether (1%), tween-807 (1%), ethyl paraben (0.1%), distilled water (about 31-55%), and mixed with appropriate amount of CLY series compounds to form a mixed emulsion. The emulsion matrix used in this embodiment refers to the matrix component after the active ingredient was removed from the emulsion.

1.2 Grouping and modeling of experimental animals: SPF grade male rats, weighing (210 ± 28) g, from the Animal Center of Nanyi University. **The** rats were numbered according to their body weight and using the random array method, samples were divided into the model control group (applied cream matrix), CLY-1 treatment group (applied 0.5% CLY-1 cream on the skin), CLY-2 treatment group (applied 0.5% CLY-2 cream on the skin), CLY-3 treatment group (0.5% CLY-3 cream on the skin), CLY-4 treatment group (0.5% CLY-4 cream on the skin), CLY-8 treatment group (0.5% CLY-8 cream on the skin), CLY-11 treatment group (0.5% CLY-11 cream on the skin) CLY-19 treatment group (0.5% CLY-19 cream on the skin), CLY-36 treatment group (0.5% CLY-36 cream on the skin), and CLY-39 treatment group (0.5% CLY-39 cream on the skin), 6 in each group. Rats in each group were anesthetized by intraperitoneal injection of 2% pentobarbital sodium (120 mg/kg) and fixed on the operating table, and then chose a piece of 4 × 5 cm of intact skin on the back, 8% sodium sulfide hair removal, used tissue scissors to cut a wound with a diameter of 2.4 cm and a depth of muscle fascia at the hair removal site, and damage part of the muscle surface fascia. Animals were kept in separate cages to prevent rats from biting and licking. Applied 2% iodine tincture daily to the wound for routine disinfection, and observed the wound healing of rats.

### 2. Experimental results

### 2.1 Observation results of rat wound

The wounds were routinely disinfected every day, and the wounds of rats were observed on the 1st, 3rd, 5th, 7th, 12^{th}, and 20th days. Since the fifth day, the wound recovery rate of each CLY series treatment group was significantly faster than that of the model group, and the wound area gradually decreased. On the 12th day, the wounds in each treatment group had basically recovered, while the model group still had about 0.5cm2 of wounds. By the 20th day, the wounds in each group had recovered, and the model control group left obvious scars, while each treatment group only left different amounts of pigmentation.

### 3. Experimental conclusion

CLY-1, CLY-2, CLY-3, CLY-4, CLY-8, CLY-11, CLY-19, CLY-36, and CLY-39 can significantly promote skin wound healing and reduce scar formation.

### Example 9 Effect of compound CLY series on rat hair loss model

### 1. Experimental method

### 1.1 Materials

(1) Compound CLY-1, CLY-2, CLY-3, CLY-4, CLY-5, CLY-8, CLY-11, CLY-19, CLY-36, CLY-39 tincture preparation method: 75% ethanol was mixed with appropriate amount of above compounds to prepare a tincture of different concentrations.
(2) Positive treatment drug: 5% minoxidil tincture (trade name: Mandi, Zhejiang Wanma Pharmaceutical Co., Ltd.).
(3) Experimental animals: SPF Wistar rats, male, from **Shanghai** Slac Experimental Animal Co., Ltd.

### 1.2 Animal grouping and modeling

Wistar rats were,according to weights, randomly divided into the negative control group (75% ethanolfor external use), model group (75% **ethanolfor** external use), positive control group (external 5% minoxidil tincture), CLY-1 external treatment group (external 5% CLY-1 tincture), CLY-2 external treatment group (external 5% CLY-2 tincture), CLY-3 external treatment group (external 5% CLY-3 tincture), CLY-4 external treatment group (external 5% CLY-4 tincture), and CLY-5 external treatment group (external 5% CLY-5 tincture) according to their body weight CLY-8 external treatment group (external5% CLY-8 tincture), CLY-11 external treatment group (external 5% CLY-11 tincture), CLY-19 external treatment group (external 5% CLY-19 tincture), CLY-36 external treatment group (external 5% CLY-36 tincture), CLY-39 external treatment group (external 5% CLY-39 tincture), CLY-1 intravenous injection group (2 mg/kg. d), CLY-3 intravenous injection group (2 mg/kg. d), CLY-4 intravenous injection group (2 mg/kg. d) CLY-5 intravenous injection group (2mg/kg. d), CLY-8 intravenous injection group (2mg/kg. d), CLY-11 intravenous injection group (2mg/kg. d), CLY-19 intravenous injection group (2mg/kg. d), CLY-36 intravenous injection group (2mg/kg. d), and CLY-39 intravenous injection group (2mg/kg. d), with 10 rats in each group. Before the experiment, an area of 4 cm × 5 cm was selected on each rat on the back of the neck to remove hair as the observation area. In addition to the negative control group, rats were subcutaneously injected with testosterone propionate injection [5ml/(kg · d)] once a day for 60 days to establish the androgenic alopecia (AGA) model. After continuous subcutaneous injection of testosterone propionate for 4 weeks, the hair of rats gradually fell off, and the remaining hair became thin and brittle, which proved that the AGA hair loss model was successfully established. At the same time, the drug was applied to the observation area on the back of the rats in the corresponding drug group at the rate of 2 mL/(one time), twice a day, at an interval of 8 hours; Intravenous administration once a day. The negative control group and the model control group were smeared with 75% ethanol solution, 2 mL/(one rat per time), twice a day, for 60 days.

### 1.3 Observation indicators and test methods

Took 10 hairs from the observation area on the back of each rat every 15 days after administration, and measured the hair length with a **vernier** caliper. After 60 days of administration, the skin of the experimental observation area was taken for routine tissue dehydration, paraffin embedding, HE staining, and light microscopy to observe the histopathological changes of the rat skin hair follicles and sebaceous glands. Semi-quantitative analysis was performed for each group. The grading criteria are as follows: the normal structure of dermal tissue cells, subcutaneous hair follicles, and sebaceous glands is marked as "1": no hyperplasia of dermal tissue is found, the lesions of hair follicles and sebaceous glands are limited, and no inflammation is found under the skin is marked as "±": no obvious hyperplasia of dermal tissue is found, and the follicles are obviously cystic. No obvious hyperplasia of sebaceous glands is found, and no inflammation is found under the skin is marked as "+": the dermal tissue has segmental hyperplasia, which is not obvious, A small number of hair follicles have cystic changes, and the sebaceous glands have mild hyperplasia and hypertrophy. There is no obvious inflammation under the skin, which is marked as "++": the dermal tissue cells of the skin have different degrees of segmental hyperplasia, and some hair follicles have cystic changes, showing uneven size of hair follicles, and no cells around. The sebaceous glands have hyperplasia, and the nuclei in the proliferative glands are few. A few rats have mild inflammatory hyperplasia under the skin, which is marked as "+++".

### 2. Experimental results

### 2.1 Effect of CLY on hair growth of rats

The hair length of rats in each treatment group was longer than that in the model control group on the 15th, 30th, 45^{th}, and 60th day of **administration,** with statistical significance (P<0.01). Compared with the positive treatment group, the difference was statistically significant (P<0.05). See Table 6

**Table 6 Effect of each group on hair growth length of rats**

| Group | 15-day hair length (mm) | 30-day hair length (mm) | 45-day hair length (mm) | 60-day hair length (mm) |
|---|---|---|---|---|
| Negative control group | 9.4* | 10.5* | 13.4* | 15.5* |
| Model control group | 6.3 | 7.5 | 9.6 | 11.6 |
| Positive control group | 7.6* | 8.5* | 13.1 * | 16.2* |
| CLY-1external use group | 8.3 * | 9.3 * | 14.2* | 17.6* |
| CLY-2 external use group | 9.1* | 10.1* | 15.3* | 18.9* |
| CLY-3 external use group | 7.5* | 8.6* | 12.45* | 16.3 * |
| CLY-4 external use group | 7.2* | 8.3 * | 12.26* | 16.1* |
| CLY-5 external use group | 9.2* | 10.2* | 15.5* | 18.9* |
| CLY-8 external use group | 9.3 * | 10.3* | 15.7* | 19.2* |
| CLY-11 external use group | 8.2* | 9.1* | 13.89* | 17.4* |
| CLY-19 external use group | 8.5* | 9.4* | 14.02* | 17.6* |
| CLY-36 external use group | 8.1* | 8.7* | 13.81* | 17.1* |
| CLY-39 external use group | 9.1* | 9.3 * | 14.8* | 18.2* |
| CLY-1 venous group | 8.7* | 9.6* | 14.6* | 17.9* |
| CLY-2 venous group | 9.2* | 10.3* | 15.6* | 19.1* |
| CLY-3 venous group | 7.3 * | 8.9* | 13.9* | 16.8* |
| CLY-4 venous group | 7.1 * | 8.6* | 13.5* | 16.3 * |
| CLY-5 venous group | 7.8* | 9.3 * | 13.7* | 16.4* |
| CLY-8 venous group | 7.3 * | 9.5 * | 13.8* | 17.8* |
| CLY-11 venous group | 8.6* | 9.3 * | 14.7* | 17.6* |
| CLY-19 venous group | 8.8* | 9.7* | 14.9* | 17.8* |
| CLY-36 venous group | 8.6* | 9.2* | 14.2* | 17.3 * |
| CLY-39 venous group | 9.3 * | 9.9* | 14.9* | 18.1* |

| | | | | |
|---|---|---|---|---|
| *Compared with the model control group, the difference was statistically significant (P<0.01) | | | | |

### 2.2 Effect of CLY on the morphology of hair follicles in the superficial dermis of the skin tissue in the observation area of rats

In the model group, some dermal tissue cells of the rats had different degrees of segmental thickening, and there was mild lymphocyte **proliferation** under the skin of the rats; Some of the subcutaneous hair follicles of rats had obvious cystic changes. The size of the hair follicles was different. There was exfoliative keratin in the enlarged hair follicles. There was slight fibrosis around the hair follicles. The cells around the hair follicles disappeared or the cell layers were significantly reduced. There seemed to be calcification in the lumen dyed blue. The number of sebaceous glands increased. Some of the glands had hypertrophy. The nucleus of hypertrophic glands was significantly reduced, and the number of normal hair follicles decreased. Compared with the model group, the lesions of dermal tissue cells, subcutaneous hair follicles, and sebaceous glands of rats in each treatment group were alleviated to different degrees. Compared with the model control group, the number of injured hair follicles in the skin of rats in each treatment group was significantly reduced, with a statistically significant difference (P<0.01). Compared with the model control group, the lesions of dermal tissue cells, subcutaneous hair follicles, and sebaceous glands of rats in each treatment group were significantly reduced, with a statistically significant difference (P<0.01). Compared with the positive treatment group, the difference was statistically significant (P<0.05). See Table 7.

**Table 7 Effects of each group on skin hair follicles and sebaceous glands of rats**

| Groups | Number | - | ± | + | ++ | +++ | Pvalue* |
|---|---|---|---|---|---|---|---|
| Negative control group | 10 | 10 | 0 | 0 | 0 | 0 | <0.01 |
| Model control group | 10 | 0 | 0 | 1 | 4 | 5 | - |
| Positive control group | 10 | 0 | 2 | 4 | 3 | 1 | <0.01 |
| CLY-1 external use group | 10 | 0 | 5 | 4 | 1 | 0 | <0.01 |
| CLY-2 external use group | 10 | 0 | 6 | 3 | 1 | 0 | <0.01 |
| CLY-3 external use group | 10 | 0 | 4 | 5 | 1 | 0 | <0.01 |
| CLY-4 external use group | 10 | 0 | 3 | 4 | 3 | 0 | <0.01 |
| CLY-5 external use group | 10 | 0 | 6 | 4 | 0 | 0 | <0.01 |
| CLY-8 external use group | 10 | 0 | 7 | 3 | 0 | 0 | <0.01 |
| CLY-11 external use group | 10 | 0 | 5 | 4 | 1 | 0 | <0.01 |
| CLY-19 external use group | 10 | 0 | 5 | 3 | 2 | 0 | <0.01 |
| CLY-36 external use group | 10 | 0 | 5 | 2 | 3 | 0 | <0.01 |
| CLY-39 external use group | 10 | 0 | 6 | 3 | 1 | 0 | <0.01 |
| CLY-1 venous group | 10 | 0 | 6 | 4 | 0 | 0 | <0.01 |
| CLY-2 venous group | 10 | 0 | 7 | 3 | 0 | 0 | <0.01 |
| CLY-3 venous group | 10 | 0 | 5 | 4 | 1 | 0 | <0.01 |
| CLY-4 venous group | 10 | 0 | 4 | 5 | 1 | 0 | <0.01 |
| CLY-5 venous group | 10 | 0 | 8 | 2 | 0 | 0 | <0.01 |
| CLY-8 venous group | 10 | 0 | 8 | 2 | 0 | 0 | <0.01 |
| CLY-11 venous group | 10 | 0 | 6 | 3 | 1 | 0 | <0.01 |
| CLY-19 venous group | 10 | 0 | 6 | 2 | 2 | 0 | <0.01 |
| CLY-36 venous group | 10 | 0 | 6 | 1 | 3 | 0 | <0.01 |
| CLY-39 venous group | 10 | 0 | 7 | 2 | 1 | 0 | <0.01 |

| | | | | | | | |
|---|---|---|---|---|---|---|---|
| Note: * is statistically significant compared with the model control group (P<0.01) | | | | | | | |

### 3. Experimental conclusion

CLY-1, CLY-2, CLY-3, CLY-4, CLY-5, CLY-8, CLY-11, CLY-19, CLY-36, and CLY-39, for both external use and system application, can significantly promote the hair growth of rat hair loss model, and reduce the damage to subcutaneous hair follicles and sebaceous glands, with no obvious adverse reactions.

### Example 10 Effect of compound CLY series on mouse hair loss model1. Experimental method

### 1.1 Materials

(1) Preparation method of tincture of compounds CLY-1, CLY-2, CLY-3, CLY-4, CLY-8, CLY-11, CLY-19, CLY-36, and CLY-39: 60% ethanol was mixed with the above compounds to prepare a tincture of 2% concentration.
(2) Positive treatment drug: 2% minoxidil tincture (produced by the Institute of Dermatology, Chinese Academy of Medical Sciences)
(3) Experimental animals: SPF grade male healthy C57BL/6 mice, half male and half female, 6-8 weeks old, weighing 20-25g, provided by Chengdu Yaokang Biotechnology Co., Ltd.

### 1.2 Animal grouping and modeling

The experimental mice were divided into thenegative control group (external use of 60% ethanol), model group (external use of 60% ethanol), positive control group (external use of 2% minoxidil tincture), CLY-1 external treatment group (external use of 2% CLY-1 tincture), CLY-2 external treatment group (external use of 2% CLY-2 tincture), CLY-3 external treatment group (external use of 2% CLY-3 tincture), CLY-4 external treatment group (external use of 2% CLY-4 tincture), CLY-8 external treatment group (external use of 2% CLY-8 tincture) CLY-11 external treatment group (2% CLY-11 tincture), CLY-19 external treatment group (2% CLY-19 tincture), CLY-36 external treatment group (2% CLY-36 tincture), and CLY-39 external treatment group (2% CLY-39 tincture). There were 6 animals in each group, half male and half female. An area of 4cm×5 cm from the back of experimental mice with hair removed was selected, and the hair removal area was painted yellow with 3% picric acid solution to determine the observation area of the hair removal experiment. In addition to the negative control group, mice in other groups were injected subcutaneously with testosterone propionate injection [8ml/(kg · d)] at the back of the neck, once a day, for 60 consecutive days, to establish the model of androgenic alopecia (AGA). At the same time of modeling, the rats in the corresponding drug group were smeared with drugson the observation area of the back at the rate of 1 ml/(piece · time), twice a day, with an interval of 2 hours. The normal control group and the model control group were smeared with the excipient (60% ethanol solution), 1 ml/(one time), twice a day, for 60 consecutive days.

### 1.3 Observation indicators and test methods

10 hairs were extracted from the observation area on the back of each mouse every 15 days after administration, andthe hair length was measured with a vernier caliper. After 60 days of administration, the skin of the experimental observation area was taken for routine tissue dehydration, paraffin embedding, HE staining, and light microscopy to observe the histopathological changes of mouse skin hair follicles and sebaceous glands. Semi-quantitative analysis was performed for each group. The grading criteria are as follows: the normal structure of dermal tissue cells, subcutaneous hair follicles, and sebaceous glands is marked as "1": no hyperplasia of dermal tissue is found, the lesions of hair follicles and sebaceous glands are limited, and no inflammation is found under the skin is marked as "±": no obvious hyperplasia of dermal tissue is found, and the follicles are obviously cystic. No obvious hyperplasia of sebaceous glands is found, and no inflammation is found under the skin is marked as "+": the dermal tissue has segmental hyperplasia, which is not obvious, A small number of hair follicles have cystic changes, and the sebaceous glands have mild hyperplasia and hypertrophy. There is no obvious inflammation under the skin, which is marked as "++": the dermal tissue cells of the skin have different degrees of segmental hyperplasia, and some hair follicles have cystic changes, showing uneven size of hair follicles, and no cells around. The sebaceous glands have hyperplasia, and the nuclei in the proliferative glands are few. A few subcutaneous mild inflammatory hyperplasia is marked as "+++".

### 2. Experimental results

### 2.1 Effect of CLY series on hair growth in mice

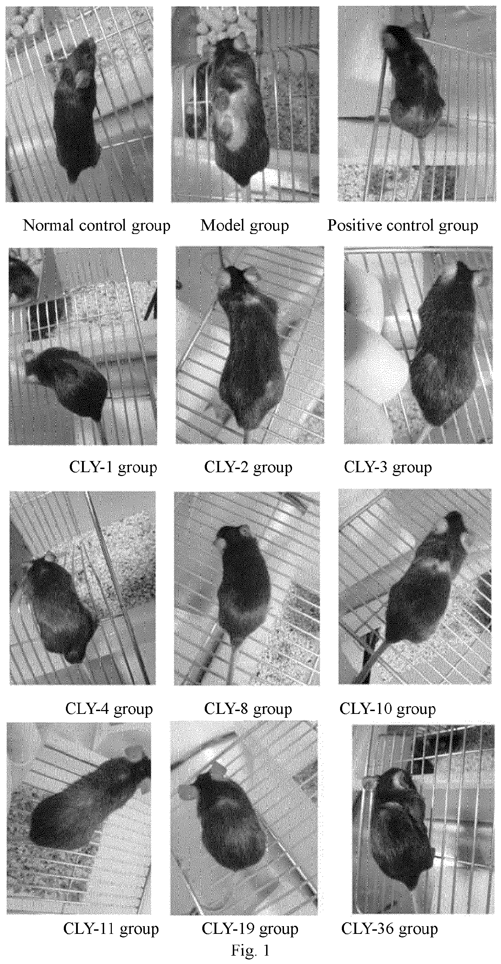

The hair length of mice in each group was longer than that in the model control group on the 15th, 30th, 45^{th}, and 60th day of administration, with statistical significance (P<0.01), and the difference was statistically significant compared with the positive treatment group (P<0.05). See Table 8. The hair growth of mice in each group on the 30th day of administration is shown in Figure 1.

**Table 8 Effect of each group on hair growth length of mice**

| Group | 15-day hair length (cm) | 30-day hair length (cm) | 45-day hair length (cm) | 60-day hair length (cm) |
|---|---|---|---|---|
| Negative control group | 0.565* | 0.802* | 0.920* | 0.993* |
| Model control group | 0.373 | 0.619 | 0.643 | 0.703 |
| Positive control group | 0.591* | 0.814* | 0.825* | 0.842* |
| CLY-1 group | 0.653* | 0.851 * | 0.858* | 0.951 * |
| CLY-2 group | 0.676* | 0.865* | 0.869* | 0.962* |
| CLY-3 group | 0.646* | 0.843* | 0.847* | 0.946* |
| CLY-4 group | 0.641* | 0.837* | 0.841 * | 0.939* |
| CLY-8 group | 0.682 * | 0.872* | 0.886* | 0.971* |
| CLY-11group | 0.637* | 0.831 * | 0.834* | 0.932* |
| CLY-19 group | 0.623* | 0.826* | 0.828* | 0.915* |
| CLY-3 6 group | 0.628* | 0.824* | 0.823 * | 0.919* |
| CLY-3 9 group | 0.665* | 0.845* | 0.867* | 0.956* |

| | | | | |
|---|---|---|---|---|
| *Compared with the model control group, the difference was statistically significant (P<0.01) | | | | |

### 2.2 Effect of CLY on the morphology of hair follicles in the superficial dermis of the skin tissue in the observation area of mice

In the model group, some dermal tissue cells of the mice had different degrees of segmental thickening, and there was mild lymphocytic **hyperplasia** under the skin of the mice; Some of the subcutaneous hair follicles of mice had obvious cystic changes. The size of the hair follicles was different. There was exfoliative keratin in the enlarged hair follicles. There was slight fibrosis around the hair follicles. The cells around the hair follicles disappear or the cell layers were significantly reduced. There seemed to be calcification in the cavity dyed blue. The number of sebaceous glands was increased. Some of the glands had hypertrophy. The nucleus of the hypertrophic glands was significantly reduced, and the number of normal hair follicles was reduced. Compared with the model group, the lesions of dermal tissue cells, subcutaneous hair follicles, and sebaceous glands of mice in each treatment group were alleviated to different degrees. Compared with the model control group, the number of damaged hair follicles in the skin of mice in each treatment group was significantly reduced, the difference was statistically significant (P<0.01). Compared with the model control group, the lesions of dermal tissue cells, subcutaneous hair follicles, and sebaceous glands of mice in each treatment group were significantly reduced, the difference was statistically significant (P<0.01). See Table 9.

**Table 9 Effects of each group on skin hair follicles and sebaceous glands of mice (mice)**

| Group | Number | - | ± | + | ++ | +++ | Pvalue* |
|---|---|---|---|---|---|---|---|
| Normal control group | 6 | 6 | 0 | 0 | 0 | 0 | <0.01 |
| Model control group | 6 | 0 | 0 | 0 | 1 | 5 | - |
| Positive control group | 6 | 0 | 2 | 1 | 3 | 0 | <0.01 |
| CLY-1 group | 6 | 0 | 3 | 2 | 1 | 0 | <0.01 |
| CLY-2 group | 6 | 0 | 4 | 2 | 0 | 0 | <0.01 |
| CLY-3 group | 6 | 0 | 3 | 1 | 2 | 0 | <0.01 |
| CLY-4 group | 6 | 0 | 2 | 2 | 2 | 0 | <0.01 |
| CLY-8 group | 6 | 0 | 4 | 2 | 0 | 0 | <0.01 |
| CLY-11 group | 6 | 0 | 3 | 1 | 2 | 0 | <0.01 |
| CLY-19 group | 6 | 0 | 3 | 2 | 1 | 0 | <0.01 |
| CLY-36 group | 6 | 0 | 3 | 1 | 2 | 0 | <0.01 |
| CLY-39 group | 6 | 0 | 3 | 2 | 1 | 0 | <0.01 |

| | | | | | | | |
|---|---|---|---|---|---|---|---|
| Note: * is statistically significant compared with the model control group (P<0.01) | | | | | | | |

### 3. Experimental conclusion

CLY-1, CLY-2, CLY-3, CLY-4, CLY-8, CLY-11, CLY-19, CLY-36, and CLY-39 can significantly promote the hair growth of mouse hair loss model and reduce the damage to subcutaneous hair follicles and sebaceous glands.

### Example 11 Effect of compound CLY series on rabbit ear acne model

### 1. Experimental method

### 1.1 Materials

(1) Preparation method of treatment cream: The excipient matrix components included methyl silicone oil (15%), stearic acid (6%), white vaseline (5%), liquid paraffin (5%), octadecanol (5%), glycerin (20%), alkyl aryl polyglycol ether (1%), fatty alcohol polyoxyethylene ether (1%), tween-807 (1%), ethyl paraben (0.1%), distilled water (about 31-55%), and mixed with appropriate amount of CLY series compounds to form a mixed emulsion. The emulsion matrix used in this embodiment refers to the matrix component after the active ingredient was removed from the emulsion.
(2) Positive therapeutic drug: 0.1% adapalene gel (Trade name: Differin, produced by Laboratoires Galderma, France).
(3) Experimental animals: ordinary New Zealand rabbits, 1.8~2.1 kg, male, from Shanghai Slac Laboratory Animal Co., Ltd.

### 1.2 Animal grouping and modeling

New Zealand rabbits were,according to weights, randomly divided into the model control group (applied cream matrix), positive treatment group (appliedDifferin on the skin), CLY-1 external treatment group (0.25% CLY-1 cream on the skin), CLY-2 external treatment group (0.25% CLY-2 cream on the skin), CLY-3 external treatment group (0.25% CLY-3 cream on the skin), CLY-4 external treatment group (0.25% CLY-4 cream on the skin) CLY-8 external treatment group (0.25% CLY-8 cream on the skin), CLY-9 external treatment group (0.25% CLY-9 cream on the skin), CLY-11 external treatment group (0.25% CLY-11 cream on the skin), CLY-19 external treatment group (0.25% CLY-19 cream on the skin), CLY-36 external treatment group (0.25% CLY-36 cream on the skin); CLY-1 intravenous injection group (1mg/kg. d), CLY-2 intravenous injection group (1mg/kg. d), CLY-3 intravenous injection group (1mg/kg. d), CLY-4 intravenous injection group (1mg/kg. d), CLY-8 intravenous injection group (1mg/kg. d), CLY-9 intravenous injection group (1mg/kg. d), CLY-11 intravenous injection group (1mg/kg. d), CLY-19 intravenous injection group (1mg/kg. d), and CLY-36 intravenous injection group (1mg/kg. d), with 10 in each group. Took the inner side of the rabbit's right ear for depilation treatment as the observation area, and the inner side of the left ear of all rabbits as their own negative control, and applied 95% alcohol. The inner side of the right ear of the model group and the treatment group were both coated with 2% coal tar (Alfa Aesar China, which used 95% alcohol to prepare 2% coal tar solution), and evenly applied the sterile cotton swab to the opening of the ear duct on the inner side of the rabbit's ear within the range of about 2 cm × 2cm, once a day, 0.5 mL each time, and wiped the previous application site with warm water for 14 consecutive days to establish the acne micro-pigmentation model. Visually observed the changes of local skin, including ear thickness, hardness, roughness, and whether there was a black angle plug at the hair follicle mouth. 18 hours after the last application, the sample was sacrificed and skin was taken. The skin tissue was taken from the application site by using a 5 mm punch, fixed with 10% formaldehyde, embedded in paraffin, and stained with HE. The histopathological observation and analysis were carried out.

### 1.3 Observation indicators

The histological grading criteria of the acne model: 3 grades according to histological grade. Level 0 "1" means that there are only loose keratinized cells in the funnel, without acne; Level 1: the skin on the surface of the rabbit ear is red, or a small amount of dense keratinized material is seen at the funnel of the hair follicle, and the funnel does not expand "+"; Grade 2: moderately dense keratinized material is seen at the funnel of hair follicle and extends to the sebaceous gland, accompanied by hyperplasia of sebaceous duct and "2+" expansion of funnel; Level 3 is that there are extensive keratinizing substances in the hair follicles. The tight keratin embolism in the hair follicles causes severe expansion of the hair follicles, obvious hyperplasia of the sebaceous duct epithelium, skin bulges and scars, and some sebaceous glands degenerate "3+".

The histopathological changes were observed under the microscope, and the thickness of five different epidermises on a slice was measured with the Biomias99 image analysis system, and the average value was calculated; Detected the area of the two hair follicles and the diameter of the four sebaceous glands with the same position and the most complete structure in the four sections, calculated their average values, and then subtracted the data of the left and right external auditory canal of the rabbits in each group to obtain the difference in the thickness of the left and right ear epidermis, the difference in the area of the hair follicles and the difference in the diameter of the sebaceous glands.

### 1.4 Statistical treatment

SPSS16 software was used for statistical analysis. The paired t-test was used for the left and right self-contrast, and the t-test was used for the comparison between the groups, P<0.05 was statistically significant.

### 2. Experimental results

Macroscopic observation: after 14 days of applying coal tar, the left ear skin of rabbits in all groups was soft, and the hair follicles in the external ear canal were arranged in order, without acne, papules, and pustules. In the model control group, the thickness of the right ear increased, the skin became hard, the surface became rough and dry, most of the hair follicle mouth saw blackhead acne, the hair follicle mouth bulged and showed papules, the contact was hard, and part of it fused into a piece. The right ear of each external treatment group showed mild dry and thick skin, with a small amount of blackhead acne. All intravenous injection groups showed that most of the follicular papules in the right ear of rabbits disappeared, the skin became thin and soft, acne was significantly reduced, the pores were significantly reduced, and there was no desquamation, which was basically close to the normal rabbit ear. Compared with the left ear, the skin of the right ear in the positive treatment group was slightly red, with a little desquamation and a small amount of acne.

Tissue section observation: the left ear of the model group showed a thin epidermis, visible hair follicles, and a clear junction between the dermis and epidermis. After the right ear of the model group was made, the epidermis was thickened, the keratinization was excessive, the granular layer and the spinous layer were thickened, there were angle plugs blocking the hair follicle mouth, the hair follicle expanded, and extended to the sebaceous gland, the hair follicle funnel was filled with keratinization material, making the hair follicle funnel expand to a pot shape; The superficial dermal capillaries expanded, and inflammatory cells infiltrated around the hair follicles, with a small number of neutrophils; The number and volume of sebaceous glands increased.

Histological grading of experimental acne under microscope in each group (see Table 10): the difference between the right ear (experimental control) and the left ear (blank control) in the model group was statistically significant (P<0.05), indicating that the rabbit ear acne model was successfully established; The difference between the right ear of rabbits in each treatment group and the right ear of rabbits in the model group was statistically significant (P<0.05), indicating that the positive control group and each treatment group could improve acne skin lesions.

**Table 10 Histological grading of acne in each group**

| Group/histological grading | n | - | 1+ | 2+ | 3+ |
|---|---|---|---|---|---|
| Left ear (negative) in model group | 10 | 10 | 0 | 0 | 0 |
| Model group right ear | 10 | 0 | 1 | 4 | 5 |
| CLY-1 external use group | 10 | 5 | 3 | 2 | 0 |
| CLY-2 external use group | 10 | 6 | 2 | 2 | 0 |
| CLY-3 external use group | 10 | 4 | 4 | 2 | 0 |
| CLY-4 external use group | 10 | 3 | 5 | 2 | 0 |
| CLY-8 external use group | 10 | 8 | 1 | 1 | 0 |
| CLY-9 external use group | 10 | 5 | 4 | 1 | 0 |
| CLY-11 external use group | 10 | 5 | 3 | 2 | 0 |
| CLY-19 external use group | 10 | 5 | 2 | 3 | 0 |
| CLY-36 external use group | 10 | 5 | 1 | 4 | 0 |
| CLY-39 external use group | 10 | 6 | 3 | 1 | 0 |
| CLY-1 venous group | 10 | 7 | 2 | 1 | 0 |
| CLY-2 venous group | 10 | 8 | 1 | 1 | 0 |
| CLY-3 venous group | 10 | 6 | 2 | 2 | 0 |
| CLY-4 venous group | 10 | 5 | 3 | 2 | 0 |
| CLY-8 venous group | 10 | 9 | 1 | 0 | 0 |
| CLY-9 venous group | 10 | 7 | 3 | 0 | 0 |
| CLY-11 venous group | 10 | 7 | 2 | 1 | 0 |
| CLY-19 venous group | 10 | 8 | 1 | 1 | 0 |
| CLY-36 venous group | 10 | 7 | 1 | 2 | 0 |
| CLY-39 venous group | 10 | 7 | 2 | 1 | 0 |
| Positive treatment group | 10 | 5 | 2 | 3 | 0 |

As shown in Table 11, the skin thickness, hair follicle plot, and sebaceous gland diameter of the right ear (experimental control) of the rabbits in the model group, compared with the left ear (blank control), had statistically significant differences (P<0.05), which proved that the rabbit ear acne model was successfully replicated; The skin thickness, hair follicle accumulation and sebaceous gland diameter of the right ear of rabbits in each treatment group were significantly decreased compared with those in the model group, the differences were statistically significant (P<0.05), suggesting that the positive control group and each treatment group could improve the skin pathological damage of acne.

**Table 11 Ear epidermal thickness, hair follicle area and sebaceous gland diameter in each group**

| Group | n | epidermal thickness (mm) | hair follicle area (mm²) | sebaceous gland diameter (mm) |
|---|---|---|---|---|
| Model group left ear (negative) | 10 | 0.1326±0.0078* | 0.1083±0.0938* | 0.0452±0.0656* |
| Model group right ear | 10 | 0.2981±0.0249 | 0.4768±0.1531 | 0.4512±0.1526 |
| CLY-1 external use group | 10 | 0.2376±0.0182* | 0.2736±0.0967* | 0.0825±0.0389* |
| CLY-2 external use group | 10 | 0.2348±0.0169* | 0.2639±0.0934* | 0.0687±0.0375* |
| CLY-3 external use group | 10 | 0.2385±0.0198* | 0.2923±0.0987* | 0.0837±0.0412* |
| CLY-4 external use group | 10 | 0.2436±0.0215* | 0.3254±0.1126* | 0.0958±0.0456* |
| CLY-8 external use group | 10 | 0.2237±0.0168* | 0.2115±0.0832* | 0.0663±0.0307* |
| CLY-9 external use group | 10 | 0.2389±0.0189* | 0.2834±0.0981* | 0.0715±0.0412* |
| CLY-11 external use group | 10 | 0.2372±0.0176* | 0.2745±0.0946* | 0.0786±0.0392* |
| CLY-19 external use group | 10 | 0.2365±0.0181* | 0.2766±0.0953* | 0.0795±0.0398* |
| CLY-36 external use group | 10 | 0.2387±0.0186* | 0.2773±0.0945* | 0.0805±0.0406* |
| CLY-39 external use group | 10 | 0.2312±0.0152* | 0.2315±0.0662* | 0.0716±0.0323* |
| CLY-1 venous group | 10 | 0.2287±0.0171* | 0.2256±0.0863* | 0.0536±0.0312* |
| CLY-2 venous group | 10 | 0.2265±0.0165* | 0.2232±0.0859* | 0.0496±0.0298* |
| CLY-3 venous group | 10 | 0.2316±0.0196* | 0.2287±0.0883* | 0.0575±0.0354* |
| CLY-4 venous group | 10 | 0.2363±0.0235* | 0.2465±0.0923* | 0.0625±0.0386* |
| CLY-8 venous group | 10 | 0.1843±0.0156* | 0.1845±0.0653* | 0.0572±0.0289* |
| CLY-9 venous group | 10 | 0.2126±0.0167* | 0.2327±0.0865* | 0.0614±0.0396* |
| CLY-11 venous group | 10 | 0.2295±0.0173* | 0.2246±0.0856* | 0.0552±0.0325* |
| CLY-19 venous group | 10 | 0.2276±0.0165* | 0.2235±0.0843* | 0.0523±0.0316* |
| CLY-36 venous group | 10 | 0.2282±0.0171* | 0.2241±0.0846* | 0.0536±0.0319* |
| CLY-39 venous group | 10 | 0.2056±0.0156* | 0.2056±0.0762* | 0.0531±0.0306* |
| Positive treatment group | 10 | 0.2389±0.0176* | 0.2789±0.0926* | 0.0723±0.0387* |

| | | | | |
|---|---|---|---|---|
| *Compared with the right ear of the model group, P<0.05 | | | | |

### 3. Experimental conclusion

Compounds CLY-1, CLY-2, CLY-3, CLY-4, CLY-8, CLY-9, CLY-11, CLY-19, CLY-36, and CLY-39 can significantly reduce the symptoms of rabbit ear acne model, reduce pore blockage and acne formation, which shows that they have obvious therapeutic effect on acne.

### Example 12 Compound CLY series inhibits psoriatic inflammatory reaction in mice

### 1. Material:

Positive drug (glucocorticoid drug): mometasone furoate cream (aloxone), product of Shanghai Schering-Plough Pharmaceutical Co., Ltd.
Animal: SPF grade healthy pure mouse (C57BL/6); 8 weeks old.
Preparation method of CLY cream: the matrix components included methyl silicone oil (15%), stearic acid (6%), white vaseline (5%), liquid paraffin (5%), octadecanol (5%), glycerin (20%), alkyl aryl polyglycol ether (1%), fatty alcohol polyoxyethylene ether (1%), Tween-807 (1%), ethyl paraben (0.1%), distilled water (about 31-55%), and it was mixed with an appropriate amount of CLY series compound liquidto form a mixed emulsion.

The cream matrix used in this embodiment refers to the matrix components after the active ingredients were removed from the cream.

### 2. Experimental method:

(1) SPF grade female C57BL/6 mice were purchased at the age of 8 weeks. They were randomly divided into the blank control group, model group, positive control group (external use of aloxone cream), CLY-1 treatment group (external use of 0.5% CLY-1 cream), CLY-2 treatment group (external use of 0.5% CLY-2 cream), CLY-8 treatment group (external use of 0.5% CLY-8 cream), CLY-9 treatment group (external use of 0.5% CLY-9 cream), CLY-19 treatment group (external use of 0.5% CLY-19 cream) CLY-36 treatment group (0.5% CLY-36 cream for external use), 5 in each group. After pentobarbital sodium 80 mg/kg intraperitoneal injection anesthesia, the back was shaved with an area of about 2cm × 3cm, raised in a single cage for 1 day.
(2) The blank control group was locally smeared with vaseline, and the model group, positive control group, and CLY treatment group were regularly smeared with 5% imiquimod cream 62.5mg on the back every day for 6 consecutive days. Photographs were taken every day and PASI scores were performed.
(3) On the first day of modeling, the blank control group and the model group were externally applied with cream matrix twice a day, and the treatment group was externally applied with 0.5% CLY series cream twice a day.

### 3. Experimental results:

(1) As shown in Figure 2, after 6 days of continuous application of 5% imiquimod cream, obvious erythema, scale and infiltration appeared in the back coating area of mice in the model group, while the erythema, scale, and infiltration in the back coating area of mice in each CLY treatment group were significantly lighter than those in the model group, and the erythema, scale, and infiltration in the treatment group were close to those in the positive drug treatment group. These results suggest that CLY series compounds can significantly inhibit the inflammatory reaction of the psoriatic mouse model.

### Example 13 CLY series compounds inhibit the inflammatory reaction of mouse eczema model

### 1. Experimental materials:

Ovalbumin (OVA): PBS is prepared into 20 g/L and stored at - 20 °C.

Kapotriol liniment (trade name: Dulux liniment): the product of Danish Leo Pharmaceutical Co., Ltd.

Positive drug (glucocorticoid drug): mometasone furoate cream (trade name: aloxone), product of Shanghai Schering-Plough Pharmaceutical Co., Ltd.

Preparation method of CLY cream: the matrix components included methyl silicone oil (15%), stearic acid (6%), white vaseline (5%), liquid paraffin (5%), octadecanol (5%), glycerin (20%), alkyl aryl polyglycol ether (1%), fatty alcohol polyoxyethylene ether (1%), Tween-807 (1%), ethyl paraben (0.1%), distilled water (about 31-55%), and it was mixedwith an appropriate amount of CLY series compound liquidto form a mixed emulsion. The cream matrix used in this embodiment refers to the matrix components after the active ingredients were removed from the cream.

Animal: SPF grade healthy pure mouse (C57BL/6); 8 weeks old.

### 2. Experimental method:

SPF grade female C57BL/6 mice were purchasedat the age of 8 weeks (0.02kg), 6 in each group, and randomly divided into the blank control group, model group, positive drug group, CLY-1 treatment group (0.1% CLY-1 cream for external use), CLY-2 treatment group (0.1% CLY-2 cream for external use), CLY-3 treatment group (0.1% CLY-3 cream for external use), CLY-4 treatment group (0.1% CLY-4 cream for external use), CLY-8 treatment group (0.1% CLY-8 cream for external use), CLY-11 treatment group (0.1% CLY-11 cream for external use) CLY-19 treatment group (0.1% CLY-19 cream for external use), CLY-36 treatment group (0.1% CLY-36 cream for external use) and CLY-39 treatment group (0.1% CLY-39 cream for external use).

Model establishment: the normal control group mice were smeared with 75% ethanol 14.3ul on both ears, while the model group, positive drug group and treatment group were smeared with 1 nmoI/L carpotriol liniment 14.3ul on both ears at a regular time every day, and then smeared with 20 g/L OVA 25ul after air drying, once a day, for 12 days to make a model.

From 4 days after the start of modeling, applied the cream matrix on the ear skin of mice in the blank control group and the model group, applied aloxone on the ear skin of mice in the positive drug group, and applied the treatment cream on the ear skin of mice in each treatment group respectively, twice a day in the morning and evening, for 10 consecutive days, took photos every day, and recorded scores.

The thickness of mouse auricle was measured and recorded with ear thickness measuring instrument before and on the 14th day respectively. After the measurement on the 14th day, the mice were sacrificed. Blood was taken and serum was separated.

The enzyme-labeled plate was coated with rabbit anti-mouse interleukin (IL) - 4 antibody, stayed at 4 °C overnight, and the reaction was stopped according to the instructions of the ELISA kit to detect the level of serum IL-4. ELISA kits were all purchased from Raybiotech, USA.

### 3. Experimental results:

(1) Comparison of ear thickness of mice: before modeling, there was no statistically significant difference in ear thickness between groups (P>0.05). After modeling, the ear thickness of mice in each group is shown in Table 12. The model group was significantly higher than each treatment group, The difference between the positive drug group and blank control group was not statistically significant (P>0.05).

**Table 12 Ear thickness of mice in each group after modeling**

| Group ing | mod el group | posit ive group | blan k group | CLY -1 | CLY -2 | CLY -3 | CLY -4 | CLY -8 | CLY -11 | CLY -19 | CLY -36 | CLY -39 |
|---|---|---|---|---|---|---|---|---|---|---|---|---|
| Ear thickn ess (mm ) | 0.37 2 ±0.0 48 | 0.23 6 ±0.0 09 | 0.21 8 ±0.0 04 | 0.25 1 ±0.0 16 | 0.24 6 ±0.0 12 | 0.29 7 ±0.0 39 | 0.32 3 ±0.0 41 | 0.24 5 ±0.0 21 | 0.27 8 ±0.0 23 | 0.25 3 ±0.0 15 | 0.24 8 ±0.0 16 | 0.24 6± 0.01 4 |

(2) Serum IL-4 concentration: before modeling, there was no significant difference in serum IL-4 concentration between groups (P>0.05). After modeling, the level of serum IL-4 in peripheral blood of mice in each group is shown in Table 13. The model group was significantly different from other groups (p<0.01), the difference between the positive group and other groups was not statistically different (p>0.05).

**Table 13 IL-4 level in peripheral blood of mice in each group after modeling**

| Groupi ng | mod el group | positi ve group | blan k group | CL Y-1 | CL Y-2 | CL Y-3 | CL Y-4 | CL Y-8 | CL Y-11 | CL Y-19 | CL Y-36 | CL Y-39 |
|---|---|---|---|---|---|---|---|---|---|---|---|---|
| IL-4 level (pg/ ml) | 9.26 ±0.7 5 | 5.46 ±0.11 | 5.14 ±0.0 6 | 6.76 ±0.1 9 | 5.58 ±0.1 8 | 7.32 ±0.2 1 | 7.51 ±0.2 6 | 5.53 ±0.1 5 | 6.45 ±0.1 8 | 5.72 ±0.1 5 | 5.86 ±0.1 6 | 5.75 ±0.1 5 |

### 4. Experimental conclusion:

Compounds CLY-1, CLY-2, CLY-3, CLY-4, CLY-8, CLY-11, CLY-19, CLY-36, and CLY-39 can all inhibit the inflammatory reaction of eczema mouse model by reducing the level of serum IL-4.

### Example 14 Effect of compounds CLY-1, CLY-2, or CLY-8 on the mouse model of graft-versus-host disease

### 1. Experimental method

1.1 Experimental animals: 6-8-week-old SPF grade male C57BL/6 mice (H-2b) and female BALB/c mice (H-2d), with body weights of 16-21 g, all experimental mice were raised in SPF grade isolation cages, and the feed and padding were sterilized. After 1 week of feeding, the experiment was carried out.

### 1.2 Animal grouping and modeling

Preparation of bone marrow cells: the donor mice were sacrificed by cervical dislocation, soaked in 75% ethanol for 5 minutes, and the two femurs and tibia of the mice were taken out under sterile conditions in the ultra-clean table, and placed in RPMI1640 medium containing 2% fetal bovine serum. Rinsed the bone marrow cavity with RPMI1640 culture medium, took out the bone marrow cells, blew them into the bone marrow suspension with sterile washing tube, filtered them with a 200 mesh filter, centrifuged for 10 min at 1500 r/min, collected the cell precipitates, and added physiological saline for resuspension. In addition, took a small amount of cell suspension and counted the number of nucleated cells after removing the red blood cells from the red blood cell lysate. And adjusted the concentration of nucleated cells in bone marrow suspension to 5 × 1010 L-1_{∘}

Preparation of spleen mononuclear cells: cut the spleen envelope aseptically, put it on a 200-mesh filter screen, soaked it in RPMI1640 medium containing 2% fetal bovine serum by volume, gently grinded the spleen with the handle of a sterile syringe, washed the filter screen several times with the culture medium, collected the filtered spleen cell suspension, centrifuged it at 1500 r/min for 10 min, and collected the spleen cell precipitation. After PBS resuspension, it was blown into a single cell suspension, gently placed on the upper layer of mouse spleen lymphocyte separation solution, centrifuged at 1800 r/min for 20 min, and white membrane cells were collected. PBS was washed twice. After cell count, the cell density was adjusted to 5 × 1010 L-1, and put in a 4 °C refrigerator for use.

C57BL/6 mice were used as bone marrow transplantation donors and BABL/c mice as recipients. BABL/c mice were randomly divided into the control group, model group, and CLY treatment group (each group was injected with 5 mg/kg/d compound CLY-1, CLY-2, or CLY-8 intravenously) according to the number of body weight, with 6 mice in each group. One week before transplantation, BALB/c mice were fed sterile acidified water containing 200 mg/L gentamicin, and mice were given 8.5 Gy whole-body irradiation (cesium source) within 24 hours before transplantation. Model group and CLY-1 group, CLY-2 group, and CLY-8 group were injected with C57BL/6 mouse bone marrow nucleated cells (including cell 1 × 107) and spleen mononuclear cells (including cell 5 × 106) 0.5 mL of mixed solution was used to establish acute GVHD model in mice, and 0.5 mL of normal saline was infused into the tail vein of BABL/c mice in the control group. CLY-1 group, CLY-2 group, and CLY-8 group were injected with CLY compound solution of 5mg/kg/d intravenously, and the other groups were injected with saline of 5mg/kg/d.

### 1.3 Observation indicators and test methods

1.3.1 GVHD symptom score of mice: observed the general conditions of mice every day after transplantation, including body mass, fur, posture, activity, diarrhea, and mental state, and determined whether the mice had acute GVHD. According to the clinical GVHD symptom scoring standard, the clinical GVHD symptoms of mice in each group were scored 11 days after transplantation.

**Table 14 GVHD symptom scoring criteria**

| Standard Level | Level 0 | Level 1 | Level 2 |
|---|---|---|---|
| Body mass reduction | < 10% | >10%and < 25% | > 25% |
| Posture | Normal | Bow visible at rest | Arch affects movement obviously |
| Activity | Normal | mild to moderate reduction | no stimulation, no activity |
| Fur texture | Normal | Fur slightly to moderately curly | Fur obviously curly and disordered |
| Skin integrity | Normal | Scales fall off on the arched paw or tail during rest | Obvious exposed skin |

1.3.2 Analysis of mouse survival time: collected the survival data of mice in each group three weeks after transplantation, drew the survival curve by the Kaplan-Meier method, and compared the differences between the survival rates of mice in each group by Log-rank analysis method.

1.3.3 Histopathological examination: two weeks after transplantation, three mice with GVHD were taken from each group, and the back skin tissue, liver, and small intestine tissue were taken. 40 g/L paraformaldehyde was fixed, paraffin was fixed and embedded, hematoxylin-eosin staining was performed, and the pathological changes of each tissue were observed under the light microscope.

### 2. Experimental results

### 2.1 Effect of CLY series compounds on clinical symptoms and survival time of acute GVHD mice.

2.1.1 Clinical symptoms: The body weight and activity of mice in the radiation control group were reduced, but there were no GVHD symptoms. On the 6th day after transplantation, the mice in the acute GVHD group began to have symptoms of arched back, body mass, reduced activity, trembling, and diarrhea, while the CLY series treatment group also had symptoms of GVHD, but the symptoms were significantly reduced. The clinical symptom score on the 11th day after transplantation showed that the score of acute GVHD group was 7.48 ± 0.77, and that of CLY-1 group, CLY-2 group and CLY-8 group were 5.28 ± 0.53, 4.68 ± 0.42 and 4.56 ± 0.39, respectively. The clinical symptom score of each CLY treatment group was lower than that of the acute GVHD group (P<0.01).

2.1.2 Survival of mice: survival curves were drawn by the Kaplan-Meier method, and the differences between survival rates of mice in each group were compared by the Log-rank analysis method. The results showed that the survival rates of mice in the acute GVHD group, CLY-1 group, CLY-2 group, and CLY-8 group were 12.13%, 61.26%, 58.79%, and 63.48%, respectively. Compared with acute GVHD group, CLY-1 group, CLY-2 group, and CLY-8 group can significantly prolong the survival time of mice (P<0.01).

2.1.3 Pathological changes of skin, liver, and small intestine: 2 weeks after transplantation, the skin, liver, and small intestine of the mice with GVHD symptoms were taken and stained with hematoxylin-eosin to observe the histopathological changes. The results showed that the skin tissue of the mice in each group became thinner and there was no obvious inflammatory cell infiltration; The pathological section of the liver showed that multiple necrotic foci took place and a large number of inflammatory cells infiltrated in the liver of mice in the acute GVHD group, while the liver of mice in CLY-1, CLY-2, and CLY-8 groups had only small necrotic lesions, with a small amount of inflammatory cells infiltrating, or inflammatory factors infiltrating in the hepatic portal area, which was significantly less than that in acute GVHD group.

### 3. Experimental conclusion

Compounds CLY-1, CLY-2, and CLY-8 can all significantly improve the survival time of acute GVHD mice, alleviate clinical symptoms, and show therapeutic effect on acute GVHD.

### Example 15 Effect of CLY series compounds on rat pulmonary fibrosis model

### 1. Experimental method

### 1.1 Materials:

(1) Reagents: bleomycin (4 mg/dose, Tianjin Taihe Pharmaceutical Co., Ltd.), mouse anti-mouse MMP monoclonal antibody (NEO Mark-ers Company), mouse anti-mouse TIMP-1 polyclonal antibody (Wuhan Shide Company), enzyme-linked immunosorbent assay (ELISA) kit (R&D Company of the United States), Quantscript RT Kit reverse transcription kit (Dalian TaKaRa Company).
(2) Experimental animals: SPF grade Wistar rats, half male and half female, aged 51~55d, weighing (180 ± 21) g, from the Animal Center of Nanjing Medical University.

### 1.2 Animal grouping and modeling

The rats were, according to the number of body weight, divided into the blank control group, model group, and CLY series treatment group by random array method, with 12 rats in each group, half male and half female. The rats in each group were anesthetized by intraperitoneal injection of 2% pentobarbital sodium (120 mg/kg), fixed on the operating table, and injected by tracheotomy in the neck. The blank control group was injected with normal saline (1.25 ml/kg), and the model group and CLY treatment group were injected with 5 U/mL bleomycin solution (5 mg/kg), once a day, for 14 consecutive days. From one week after the establishment of the model, the corresponding CLY compound solution (3mg/kg. d) was injected into the tail vein of each treatment group, and the same amount of normal saline was injected into the tail vein of the blank control group and the model group, once a day, for 14 consecutive days.

### 1.3 Observation indicators and test methods

The peripheral venous blood of animals in each group was taken from the tail vein after modeling and 14 days after treatment, and the levels of superoxide dismutase (SOD) and catalase (CAT) in peripheral blood were measured. After taking blood from each group, the rats were sacrificedtwice (after modeling and 14 days of treatment). The right lung tissue of the animals was stored in a refrigerator at - 4 °C for detection of VEGF. The left lung tissue was routinely embedded in paraffin and sectioned. The expression of MMP subtype and TIMP-1 in the lung tissue of the rats was detected by immunohistochemistry. The right lung tissue was ground and homogenized, and the supernatant was taken after 3000 r/min high-speed centrifugation. The lung tissue VEGF protein was detected by ELISA, and the expression of VEGF-mRNA was detected by reverse transcription polymerase chain reaction.

### 2. Experimental results

### 2.1 Effect of CLY series compounds on MMP in rat lung tissue

A small amount of TIMP-1 and MMP subtypes were expressed in the lung tissue of rats in the blank control group. The expression of MMP-2 and MMP-9 in the model group increased and TIMP-1 decreased after modeling, which was statistically significant compared with the blank control group (P<0.05), indicating that the modeling was successful. The expression of MMP-2 and MMP-9 in the CLY series compound group decreased, and the expression of TIMP-1 was up-regulated, which was significantly different from the model group (P<0.05). See Table 15

**Table 15 Comparison of expression of TIMP-1 and MMP in lung tissue of rats in each group (n=6, x ± s)**

| Group | TIMP-1 | MMP-2 | MMP-9 |
|---|---|---|---|
| Model control group | 5.71±0.63 | 3.86±0.29 | 5.17±0.39 |
| Blank control group | 8.95±0.56* | 2.41±0.18* | 3.29±0.22* |
| CLY-1 group | 7.82±0.45* | 2.43±0.21* | 3.32±0.23* |
| CLY-2 group | 8.06±0.49* | 2.51±0.24* | 3.36±0.25* |
| CLY-8 group | 8.82±0.53* | 2.44±0.21* | 3.31±0.21* |
| CLY-11 group | 8.13±0.38* | 2.52±0.23* | 3.53±0.26* |
| CLY-36 group | 7.63±0.52* | 2.59±0.23* | 3.46±0.25* |

| | | | |
|---|---|---|---|
| Note: * Compared with the model control group, P<0.05. | | | |

### 2.2 Effect of CLY series compound on VEGF in lung tissue

The expression of VEGF protein and VEGF-mRNA in the lung tissue of rats in each group is shown in Table 16. The expression of VEGF protein and VEGF-mRNA in the model group was significantly lower, the difference was statistically significant compared with the blank control group (P<0.05), indicating that the pulmonary fibrosis model was successfully established. The expression of VEGF protein and VEGF-mRNA in CLY series compound group was significantly different from that in the model group (P<0.05).

**Table 16 Comparison of expression levels of VEGF protein and VEGF-mRNA in lung tissues of each group (n=6, x ± s)**

| Group | VEGFprotein (pg/ml) | VEGF-mRNA |
|---|---|---|
| Model control group | 37.23±4.16 | 0.52±0.19 |
| Blank control group | 54.26±3.95* | 0.83±0.13* |
| CLY-1 group | 52.16±3.61* | 0.76±0.11* |
| CLY-2 group | 51.18±3.69* | 0.71±0.12* |
| CLY-8 group | 53.16±3.62* | 0.79±0.13* |
| CLY-11 group | 49.86±3.58* | 0.76±0.13* |
| CLY-36 group | 53.87±3.73* | 0.83±0.13* |

| | | |
|---|---|---|
| Note: * Compared with the model control group, P<0.05. | | |

### 2.3 Effects of CLY series compounds on the activities of SOD and CAT enzymes in peripheral blood

The levels of SOD and CAT enzymes in the peripheral blood of rats in each group are shown in Table 17. The activity of SOD and CAT in the peripheral blood of rats in the model group decreased, the difference was statistically significant compared with the blank control group (P<0.05); The activities of SOD and CAT enzymes in the peripheral blood of rats in the CLY series compound group were increased, and the difference was statistically significant compared with the model group (P<0.05).

**Table 17 Comparison of SOD and CAT enzyme levels in peripheral blood of each group (n=6, x ± s)**

| Group | SOD (U/ml) | CAT (kU/g) |
|---|---|---|
| Model control group | 143.36±15.23 | 8.87±1.21 |
| Blank control group | 223.65±13.28* | 15.16±1.29* |
| CLY-1 group | 214.58±13.53* | 14.13±1.22* |
| CLY-2 group | 216.62±13.55* | 14.28±1.39* |
| CLY-8 group | 220.86±13.78* | 14.75±1.22* |
| CLY-11 group | 216.48±13.16* | 13.96±1.36* |
| CLY-36 group | 221.76±13.57* | 13.26±1.26* |

| | | |
|---|---|---|
| Note: * Compared with the model control group, P<0.05. | | |

### 3. Experimental conclusion

Compounds CLY-1, CLY-2, CLY-8, CLY-11, and CLY-36 can all significantly reduce the levels of MMP-2 and MMP-9 in the lung tissue of pulmonary fibrosis mice model, increase the levels of TIMP-1 and VEGF, and increase the levels of SOD and CAT enzymes in the peripheral blood, which shows that they have an inhibitory effect on pulmonary fibrosis.

### Example 16. CLY series compounds can improve the joint symptoms and inflammatory indicators of rheumatoid arthritis (RA) mice

### 1. Experimental materials and methods

### (1) Experimental materials

Freund's Adjuvant Complete (FCA, purchased from Sigma, USA); IL-10 ELISA kit (Lianke Biotechnology Co., Ltd.); IL-17 ELISA kit (Raybiotech, USA).

### (2) Grouping and treatment of experimental animals

Eighteen 5-week-old female Wistar rats were randomly divided into the control group, RA model group, and CLY series compound treatment group, with 6 rats in each group. The control group: disinfected the right toe of rats with 75% alcohol, and injected 0.15mL physiological saline subcutaneously into the right toe of rats. The RA model group: On the first day of the experiment, each rat was routinely disinfected, and 0.15 mL of FCA was injected subcutaneously into the right foot of the rat. The CLY series compound treatment group: On the first day after the experiment, each rat was routinely disinfected, and 0.15mL of FCA was injected subcutaneously into the right plantar of the rat. After 7 days, CLY series compound was administered by gavage with a dose of 10mg/kg once a day. The RA model group and control group were administered by gavage with normal saline (10mg/kg. d) every day for 21 consecutive days. On the 21st day after the intervention of CLY series compounds, 3 mL of blood was collected from the heart of rats, and the serum was isolated. The levels of IL-10 and IL-17 in the serum of rats were detected by ELISA.

### (3) Observation indicators

After the 21st day of intervention, the arthritis score of each group of rats was measured, and the swelling degree of the right toe joint of each group of rats was measured. According to the inflammatory reaction of RA rats, the severity of the ankle joint was scored as 0 to 4, which was normal; 1 point, slightly red and slightly swollen ankle joint; 2 points: erythema and slight swelling from ankle joint to metatarsal joint or metacarpal joint; 3 points: erythema and moderate swelling from ankle joint to metacarpal joint or metatarsophalangeal joint; 4 points, the ankle joint to toe joint were severely red and swollen. Added the limb scores of each rat as the arthritis score, and the maximum score was 16 points.

### 2. Experimental results

### (1) General performance of rats

Compared with the control group, the rats in the RA model group showed decreased appetite, mental depression, limited activity, and swelling of left and right toes. After 21 days of CLY series compounds intervention, the condition of rats was better, compared with other groups.

### (2) Body mass, joint swelling, and arthritis score of rats

After 21 days of drug intervention, the body mass of the control group was higher than that of the other groups, and the difference was statistically significant (P<0.05); The swelling degree and arthritis score of the right toe joint in the RA model group were higher than those in the control group, and the difference was statistically significant (P<0.05). The swelling degree and arthritis score of the CLY series compound treatment group were lower than those in the RA model group, and the difference was statistically significant (see Table 18).

**Table 18 Comparison of arthritis indexes of rats in each group**

| Group | N | Body mass (g) | Swelling degree (mm) | Joint score |
|---|---|---|---|---|
| Control group | 6 | 367.1±18.62* | 8.5±0.32* | - |
| RA model group | 6 | 295.4±9.63^{#} | 10.4±1.45^{#} | 6.63±0.39^{#} |
| CLY-1 group | 6 | 354.5±13.48* | 9.7±0.68* | 4.35±0.37* |
| CLY-2 group | 6 | 358.2±13.92* | 9.4±0.53* | 4.21±0.29* |
| CLY-8 group | 6 | 363.5±13.63* | 9.5±0.72* | 4.32±0.41* |
| CLY-36 group | 6 | 352.6±12.96* | 9.3±0.51* | 4.46±0.42* |

| | | | | |
|---|---|---|---|---|
| Note: * P < 0.05 compared with the RA model group, # P < 0.05 compared with the treatment group | | | | |

### (3) Comparison of IL-17 and IL-10 levels in serum of rats in each group

The content of IL-10 in serum of rats in the control group and CLY series compound treatment group was higher than that in the RA model group (P<0.05); The level of IL-17 in the serum of rats in the control group and the CLY series compound treatment group was lower than that in the RA model group, and the difference was statistically significant (both P<0.05) (see Table 19).

**Table 19 Comparison of serum IL-10 and IL-17 levels of rats in each group**

| Group | N | IL-17 (pg/mL) | IL-10 (pg/mL) |
|---|---|---|---|
| Control group | 6 | 36.83±3.28* | 72.57±8.42* |
| RA model group | 6 | 60.16±6.05^{#} | 45.76±5.63^{#} |
| CLY-1 group | 6 | 46.15±4.23* | 65.36±6.78* |
| CLY-2 group | 6 | 46.23±3.96* | 67.03±7.02* |
| CLY-8 group | 6 | 43.51±4.16* | 66.75±6.78* |
| CLY-36 group | 6 | 44.18±3.97* | 58.96±5.89* |

| | | | |
|---|---|---|---|
| Note: * compared with the RA model groupP < 0.05, # compared with the treatment groupP < 0.05 | | | |

### 3. Experimental conclusion

CLY series of compounds can improve the arthritis symptoms of rheumatoid arthritis mice by reducing the level of IL-17 in peripheral blood and increasing inflammatory indicators such as IL-10.

## Claims

1. A compound represented by formula I, a tautomer thereof, a solvate thereof, or a pharmaceutically acceptable salt thereof; 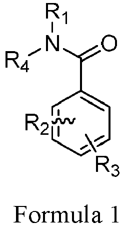
wherein R₁ is substituted or unsubstituted, a 5- to 6-membered heterocycle containing at least one of N, O, and S, or a combination ring of benzene and the abovementioned 5- to 6-membered heterocycle, or a combination ring of at least two of the abovementioned 5- to 6-membered heterocycles, wherein the substituent is H, halogen, hydroxyl, alkoxy or (C1-C4) alkyl;
wherein R₂ is H, halogen, hydroxyl, methoxy, ethoxy, amino, methyl, or ethyl;
wherein R₃ is H, halogen, hydroxyl, methoxy, ethoxy, amino, (C1-C3) alkyl, 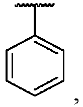 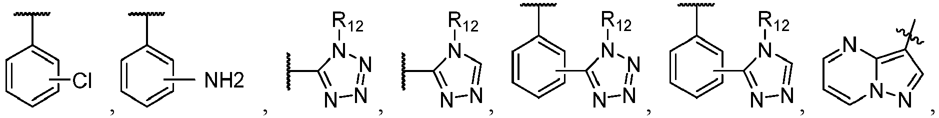 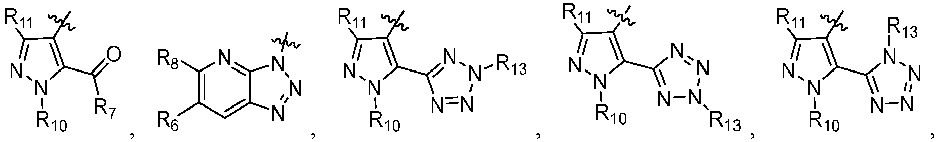 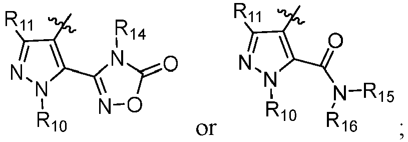
wherein R₄ is substituted or unsubstituted, a 5- to 6-membered cycloalkyl or a 4- to 7-membered heterocyclyl with 1-3 heteroatoms selected from N or O, and the substituent group is selected from H, - NH₂, - OH, (C1-C4) alkyl, (C1-C4) alkoxy, amino, and (C1-C4) alkylamino;
wherein R₆ and R₈ are independently H, halogen or (C1-C4) alkyl, and provided that R₆ and R₈ are not halogen at the same time;
wherein R₇ is hydroxy, (C1-C4) alkoxy, (C1-C4) alkoxycarbonyloxy(C1-C4) alkyl or (C1-C4) alkylcarbonyloxy (Cl-C4) alkyl;
wherein R₁₀ and R₁₁ are independently H, (C1-C4) alkyl or (C3-C6) cycloalkyl;
wherein R₁₂ is selected from H, halogen, - OH, - NH₂ or (C1-C3) alkyl;
wherein R₁₃ is H, (C1-C4) alkyl, (C1-C4) alkylcarbonyloxy (C1-C4) alkyl or (C1-C4) alkoxycarbonyloxy (Cl-C4) alkyl;
wherein R₁₄ is H, (C1-C4) alkyl, (C1-C4) alkylcarbonyloxy (C1-C4) alkyl or (C1-C4) alkoxycarbonyloxy (Cl-C4) alkyl;
wherein R₁₅ is hydroxy, tetrazolyl, (C1-C2) alkylsulfonyl or trifluoromethylsulfonyl;
wherein R₁₆ is H, (C1-C4) alkyl, (C1-C4) alkylcarbonyloxy (C1-C4) alkyl or (C1-C4) alkoxycarbonyloxy (Cl-C4) alkyl.

2. The compound represented by formula I according to claim 1, a tautomer thereof, a solvate thereof, or a pharmaceutically acceptable salt thereof, wherein if R₃ is selected from H, halogen, hydroxyl, methoxy, ethoxy, amino, (C1-C3) alkyl, 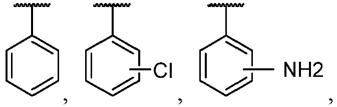 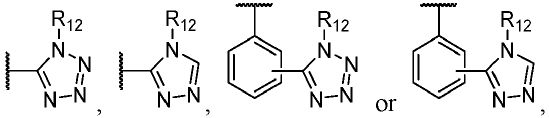 R₄ is 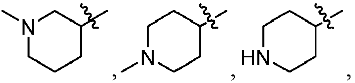 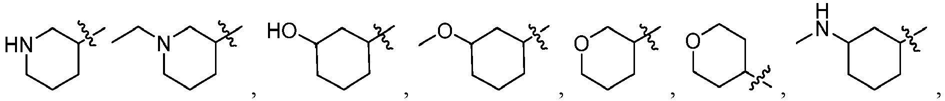 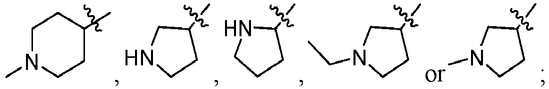 wherein if R₃ is selected from 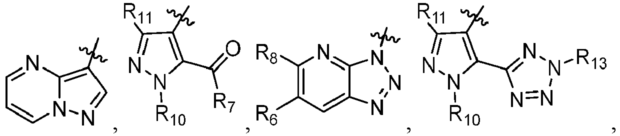 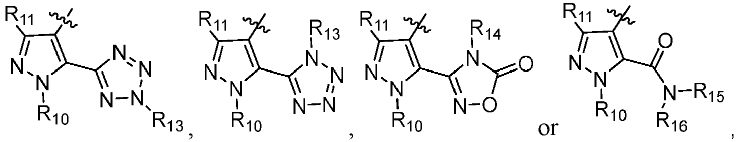 R₄ is

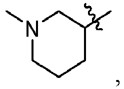

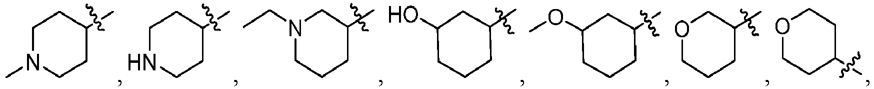

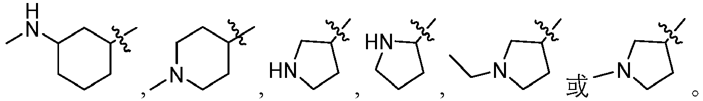

3. The compound according to claim 1, a tautomer thereof, a solvate thereof, or a pharmaceutically acceptable salt thereof, wherein R₂ is H, hydroxy or methyl.

4. The compound according to claim 1, a tautomer thereof, a solvate thereof, or a pharmaceutically acceptable salt thereof, wherein R₁ is a substituted or unsubstituted pyridyl, isoquinolinyl or pyrrolopyridyl and the substituent is H, chlorine or methyl.

5. A compound represented by formula I-a, a tautomer thereof, a solvate thereof or a pharmaceutically acceptable salt thereof, 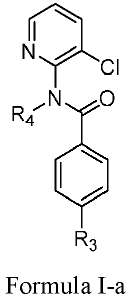
wherein if R₃ is selected from H, halogen, hydroxyl, methoxy, amino, methyl or the following substituted or unsubstituted groups: 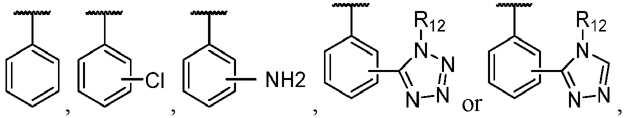 R₄ is 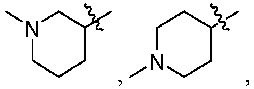 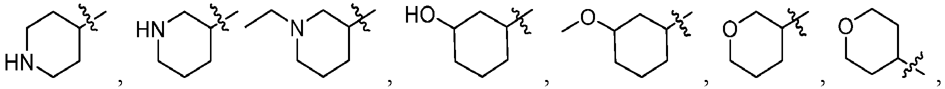 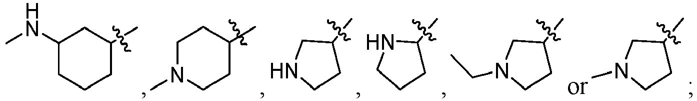
wherein if R₃ is selected from 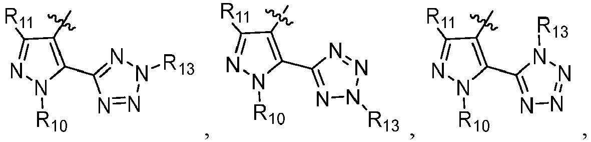 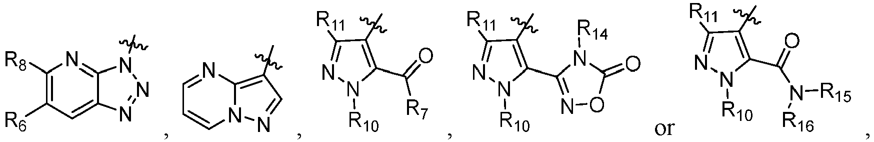 R₄ is 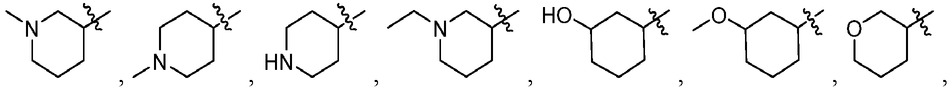 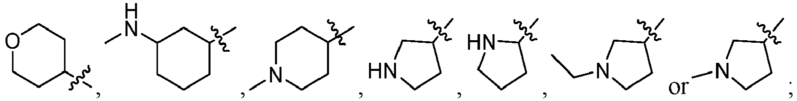
wherein R₆, R₇, R₈, R₁₀, R₁₁, R₁₂, R₁₃, R₁₄, R₁₅ and R₁₆ are defined to be consistent with the claim 1.

6. A compound represented by formula I-b, a tautomer thereof, a solvate thereof or a pharmaceutically acceptable salt thereof, 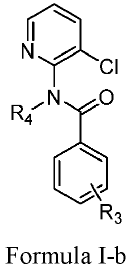
wherein if R₃ is selected from H, halogen, hydroxyl, methoxy, amino, methyl or the following substituted or unsubstituted groups: 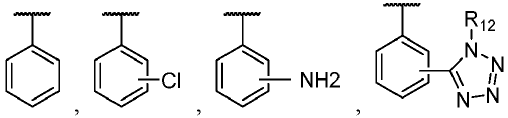 or 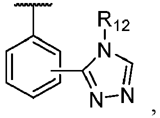 R₄ is 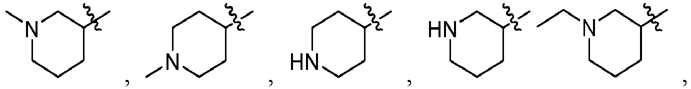 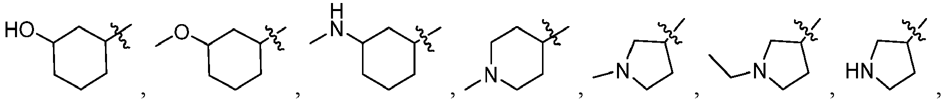 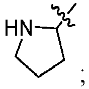
wherein if R₃ is selected from 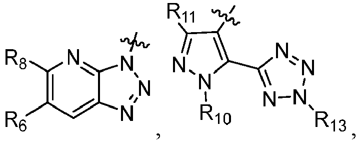 R₄ is 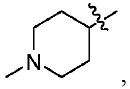 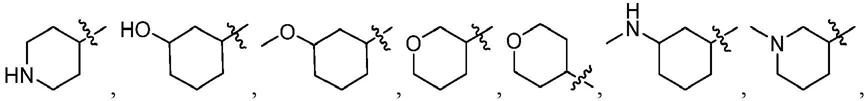 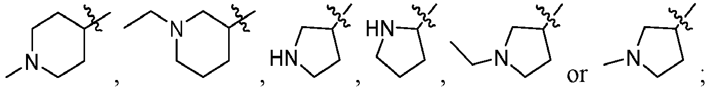
wherein R₆, R₈, R₁₀, R₁₁, R₁₂ and R₁₃ are defined to be consistent with the claim 1.

7. The compound according to any one of claims 1-6, a tautomer thereof, a solvate thereof or a pharmaceutically acceptable salt thereof, wherein R₆ is H or methyl, and R₈ is H.

8. The compound according to any one of claims 1-6, a tautomer thereof, a solvate thereof or a pharmaceutically acceptable salt thereof, wherein R₁₂ is selected from H, halogen, -OH, - NH₂ or methyl.

9. The compound according to any one of claims 1-6, a tautomer thereof, a solvate thereof or a pharmaceutically acceptable salt thereof, wherein the compound is selected from the following compounds:

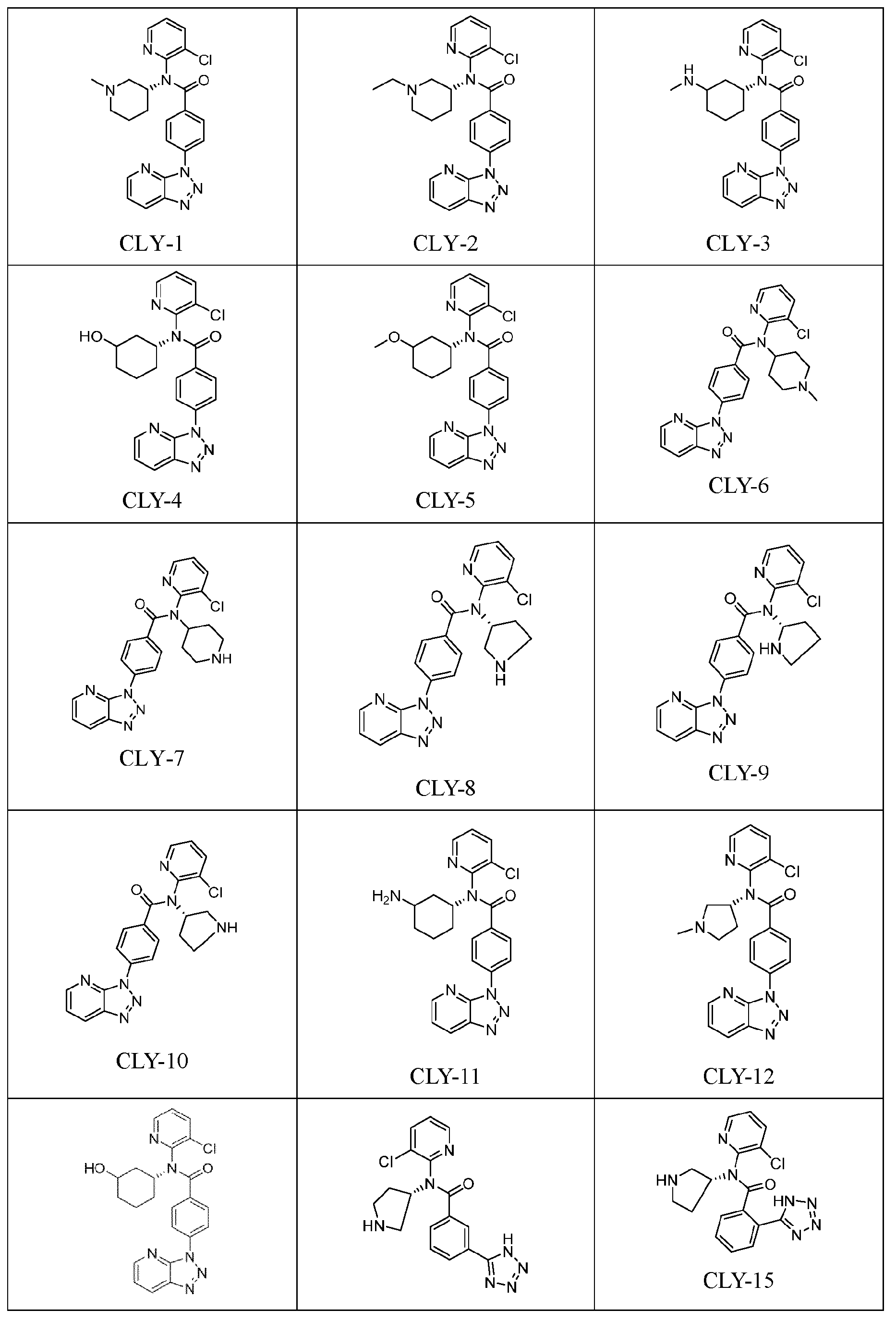

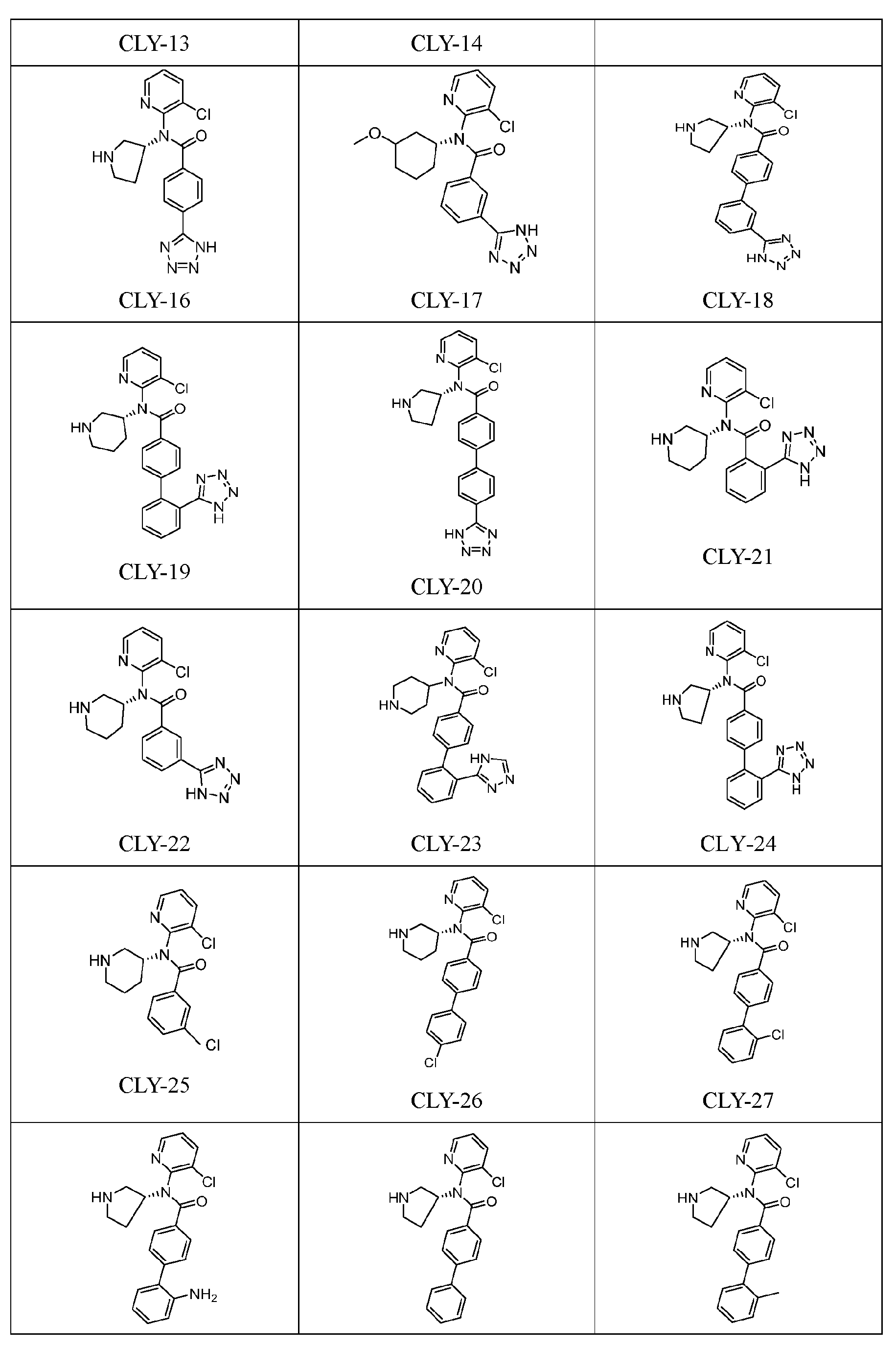

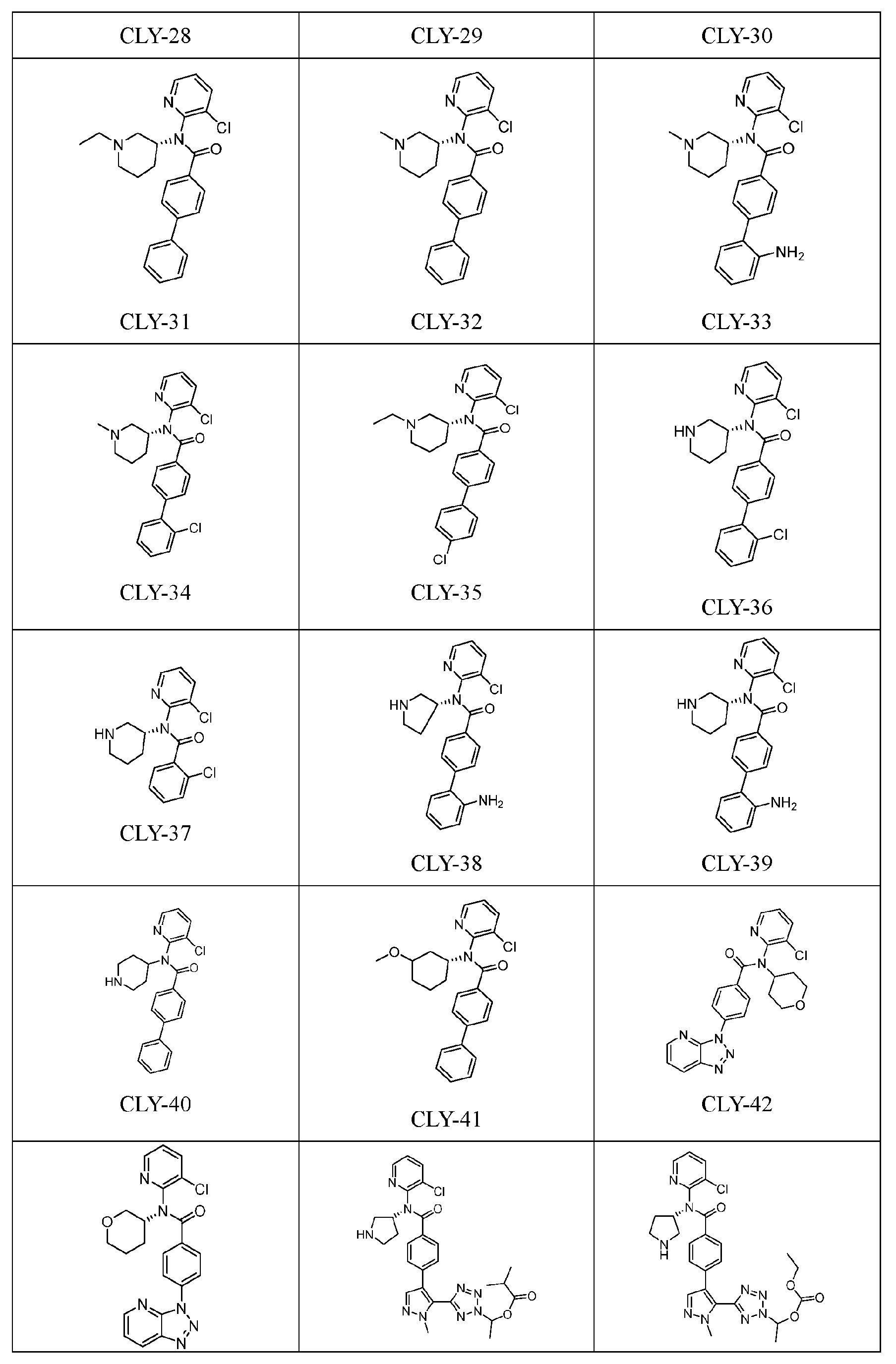

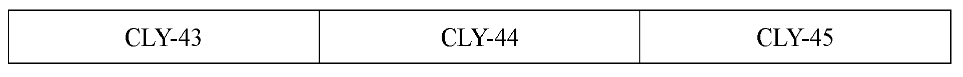

10. A preparation method of a compound represented by formula (I) shown as bellow: 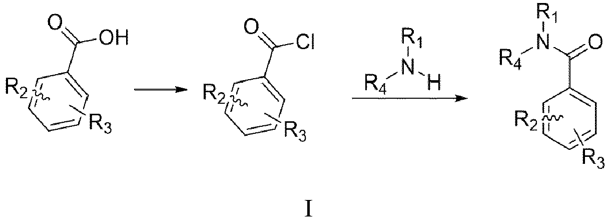
wherein the raw material 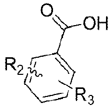 is used to produce 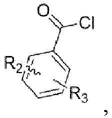 and then 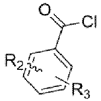 and 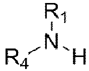 are used to produce the final product I;
wherein R₁, R₂, R₃, and R₄ are defined to be consistent with any one of the claims 1-9.

11. A pharmaceutical composition comprising the compound according to any one of claims 1-9 or the compound prepared by claim 10, a tautomer thereof, a solvate thereof or a pharmaceutically acceptable salt thereof as an active ingredient or main active ingredient supplemented with a pharmaceutically acceptable carrier.

12. Application of the compound according to any one of claims 1-9 or the compound prepared according to claim 10, a tautomer thereof, a solvate thereof or a pharmaceutically acceptable salt thereof or the composition according to claim 11, for the preparation of a medicament for treating and/or preventing diseases.

13. Application of the compound according to any one of claims 1-9 or the compound prepared according to claim 10, a tautomer thereof, a solvate thereof or a pharmaceutically acceptable salt thereof, or the composition according to claim 11 for the preparation of a medicament, for the treatment and/or prevention of chloasma, scar, androgenic alopecia, seborrheic alopecia, alopecia areata, acne, ichthyosis, porokeratosis, psoriasis, eczema, atopic dermatitis, graft versus host disease, pulmonary fibrosis or rheumatoid arthritis.

14. Any pharmaceutically acceptable dosage which is prepared from the compound according to any one of claims 1-9 or the compound prepared according to claim 10, a tautomer thereof, a solvate thereof or a pharmaceutically acceptable salt thereof, or the composition according to claim 11.

15. The dosage form according to claim 14, wherein the dosage form is suitable for oral, parenteral, intraperitoneal, intravenous, intraarterial, skinexternal use, transdermal, sublingual, intramuscular, rectal, transbuccal, intranasal, inhalation, vaginal, intraocular, topical, subcutaneous, intraadipose, intraarticular, intraperitoneal or intrathecal administration.

16. The dosage form according to claim 14, wherein the dosage form can be ointment, gel, emulsion, liniment, lotion, solution, spray, tablet, granule, oral liquid, capsule, dripping pill, enema, film or injection.
